(19) Europäisches Patentamt
European Patent Office
Office européen des brevets

(11) Publication number: **0 423 373 A1**

(12) **EUROPEAN PATENT APPLICATION published in accordance with Art. 158(3) EPC**

(21) Application number: **90907392.6**

(22) Date of filing: **25.04.90**

(86) International application number: **PCT/JP90/00545**

(87) International publication number: **WO 90/12785 (01.11.90 90/25)**

(51) Int. Cl.5: **C07C 281/14**, C07C 337/08, C07D 207/337, C07D 207/34, C07D 307/52, C07D 307/54, C07D 333/22, C07D 333/24, A61K 7/42, C09K 3/00, C08F 12/26

(30) Priority: **27.04.89 JP 110033/89**
**23.05.89 JP 129085/89**
**30.08.89 JP 225941/89**

(43) Date of publication of application: **24.04.91 Bulletin 91/17**

(84) Designated Contracting States: **AT BE CH DE DK ES FR GB IT LI LU NL SE**



(71) Applicant: **EARTH CHEMICAL CO., LTD.**
**3218-12, Sakoshi**
**Ako-shi Hyogo 678-01(JP)**

(72) Inventor: **NAKAYAMA, Tohru**
**109-10, Kariya Ako-shi**
**Hyogo 678-02(JP)**
Inventor: **KAJIFUSA, Noriyuki**
**3143-33, Ozaki Ako-shi**
**Hyogo 678-02(JP)**
Inventor: **HORINAKA, Akio**
**215-6, Manago Ako-shi**
**Hyogo 678-01(JP)**
Inventor: **YAMAGUCHI, Hideo**
**5-3, Oomachi Ako-shi**
**Hyogo 678-02(JP)**
Inventor: **FUJII, Setsuro**
**deceased**
**(JP)**

(74) Representative: **Türk, Dietmar, Dr. rer. nat. et al**
**Türk, Gille + Hrabal Patentanwälte**
**Bruckerstrasse 20**
**W-4000 Düsseldorf 13(DE)**


(54) **STYRENE DERIVATIVE AND METHOD OF PRODUCTION THEREOF, POLYSTYRENE DERIVATIVE PRODUCED THEREFROM AND METHOD OF PRODUCING THE SAME, AND ULTRAVIOLET ABSORBENT AND COSMETICS CONTAINING SAID POLYSTYRENE DERIVATIVE AS ACTIVE INGREDIENT.**


(57) A styrene derivative represented by general formula (1), wherein one of $R_1$ $R_2$ represents vinylbenzyl, and the other represents hydrogen, lower alkyl, $C_2$ to $C_5$ carboxyalkyl, or phenyl; $R_3$ represents hydrogen, halogen, lower alkyl, carboxyl, $C_2$ to $C_5$ carboxyalkyl, $C_3$ to $C_6$ carboxyalkenyl, $C_1$ to $C_4$ alkylamino or nitro; Q represents sulfur or oxygen; and X represents (a), (b), (c) ou (d).

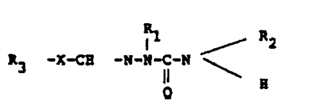 (I)

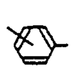 (a) 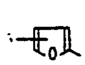 (b) 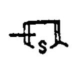 (c) 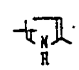 (d)


EP 0 423 373 A1

# STYRENE DERIVATIVE AND METHOD FOR PREPARING THE SAME, POLYSTYRENE DERIVATIVE COMPRISING THE STYRENE DERIVATIVE AND METHOD FOR PREPARING THE SAME, AND SUNSCREENING AGENT AND COSMETIC COMPOSITION OF WHICH ACTIVE INGREDIENT IS THE POLYSTYRENE D RIVATIVE

## TECHNICAL FIELD

The present invention relates to a novel styrene derivative and a method for preparing the same, a polystyrene derivative comprising the styrene derivative and a method for preparing the same, and a sunscreening agent or an ultraviolet absorber (hereinafter referred to as "a sunscreening agent") and a cosmetic composition of which active ingredient is the polystyrene derivative.

## BACKGROUND ART

Recently, a suntan cream and the like containing a sunscreening agent such as p-amino benzoic acid, an ester or a salt thereof as an active ingredient for preventing so-called sunburn (acute solar dermatitis) have been developed. Usually, p-amino benzoic acid has been used together with a light scattering agent such as titanium oxide since p-amino benzoic acid does not absorb ultraviolet rays having a wavelength of nearby 305 nm which mainly effects erythema caused by sunburn. However, p-amino benzoic acid is not preferable since p-amino benzoic acid is absorbed through the skin and circulated in the body so that some people may suffer from diseases. Since the suntan cream and the like containing a light scattering agent are colored in many cases, when the suntan cream is rubbed in the skin of a face or hands, the apperance of them is not preferable.

Accordingly, a polyamino acid derivative composed of a polyamino acid and an aminobenzaldehyde (thio)semicarbazone derivative has been developed as a safe sunscreening agent for human bodies, which is not absorbed through the skin and does not have the defects of the above suntan cream and the like (refer to Japanese Unexamined Patent Publication No. 65865/1981).

However, since aminobenzaldehyde(thio)semicarbazone is low in water solubility, the obtained polyamino acid derivative becomes insoluble in water when the content of aminobenzaldehyde(thio)-semicarbazone derivative is increased, that is, ultraviolet absorptive property is heightened. Therefore, a sunscreening agent having sufficient ultraviolet absorptive property could not be obtained in consideration of preparation of a sunscreening agent since there is a necessity to decrease the content of aminobenzaldehyde(thio)semicarbazone derivative in order to dissolve the polyamino acid derivative in water.

Further, the polyamino acid derivative, which is prepared by combining a polyamino acid with an aminobenzaldehyde(thio)semicarbazone derivative, has the defects that the polyamino acid which is a raw material is expensive, that purity thereof is low because the reaction rate is low and side reactions take place, and that the structure thereof cannot be apparently determined since the aminobenzaldehyde(thio)-semicarbazone derivative is prepared by reacting a high molecular compound with low molecular compounds.

Accordingly, the present inventors have researched in view of the above conventional techniques. As a result, the inventors have found a styrene derivative which can be cheaply and easily prepared and a novel polystyrene derivative comprising the styrene derivative which has excellent water solubility. As a result of further researches of the present inventors, they have found a polystyrene derivative which can be suitably used as a sunscreening agent, and have accomplished the present invention.

## DISCLOSURE OF INVENTION

The present invention relates to
(1) a styrene derivative represented by the general formula (I):

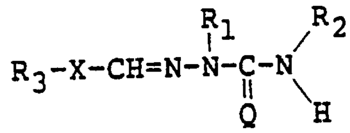

(I)

wherein one of $R_1$ and $R_2$ is vinylbenzyl group, another is hydrogen atom, a lower alkyl group, a carboxyalkyl group having 2 to 5 carbon atoms or phenyl group, $R_3$ is hydrogen atom, a halogen atom, a lower alkyl group, carboxyl group, a carboxyalkyl group having 2 to 5 carbon atoms, a carboxyalkenyl group having 3 to 6 carbon atoms, an alkylamino group having 1 to 4 carbon atoms or nitro group, Q is sulfur atom or oxygen atom, X is

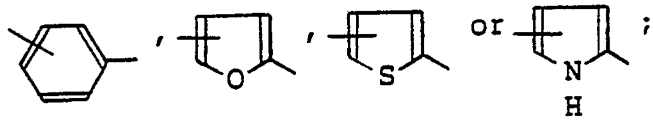

(2) a method for preparing the styrene derivative, characterized by carrying out a condensation reaction of a (thio)semicarbazide represented by the general formula (II):

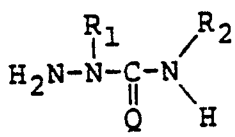

(II)

wherein $R_1$, $R_2$ and Q are the same as mentioned above, with a compound represented by the general formula (III):

$$R_3\text{-X-CHO} \qquad \text{(III)}$$

wherein $R_3$ and X are the same as mentioned above;

(3) a polystyrene derivative represented by the general formula (IV):

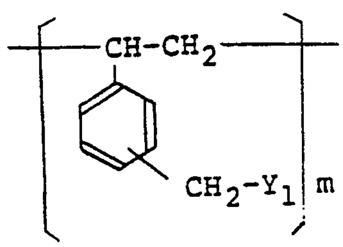

(VI)

wherein $Y_1$ is a group represented by the formula:

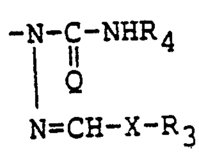

wherein $R_3$, Q and X are the same as mentioned above, $R_4$ is hydrogen atom, a lower alkyl group, a carboxyalkyl group having 2 to 5 carbon atoms, phenyl group or a group represented by the formula:

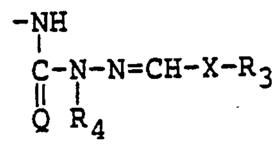

wherein $R_3$, $R_4$, Q and X are the same as mentioned above, m is an integer of 6 to 1000;
(4) a polystyrene derivative represented by the general formula (V):

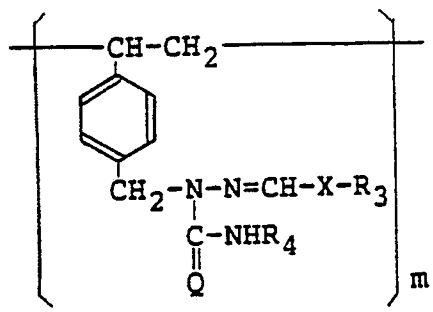

(V)

wherein $R_3$, $R_4$, Q, X and m are the same as mentioned above;
(5) a polystyrene derivative prepared by polymerizing the above-mentioned styrene derivative and a compound having unsaturated aliphatic groups containing at least one carbon-carbon double bond which contributes to polymerization;
(6) a polystyrene derivative represented by the general formula (VI):

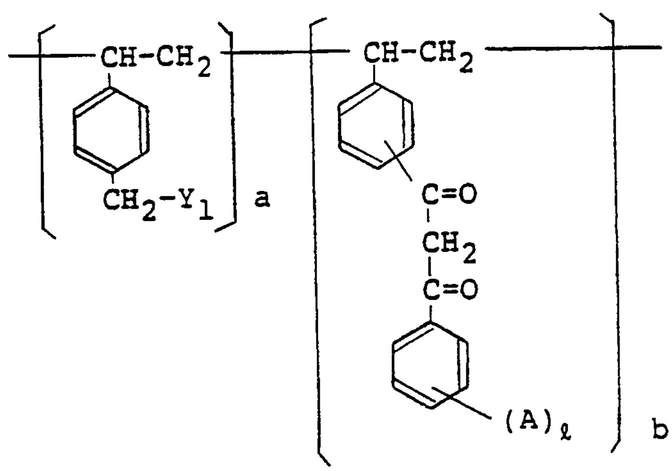

(VI)

wherein $Y_1$ is the same as mentioned above, A is a lower alkyl group, carboxyl group, a lower alkoxy group or a lower alkoxy carbonyl group, $\ell$ is an integer of 0 to 3, a and b are positive integers satisfying $m = a + b$, m is the same as mentioned above;
(7) a method for preparing the polystyrene derivative, characterized by polymerizing in a solution at least one compound represented by the general formula (I):

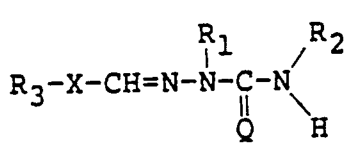

(I)

wherein $R_1$, $R_2$, $R_3$, Q and X are the same as mentioned above, or polymerizing in a solution the compound and a compound having unsaturated aliphatic groups containing at least one carbon-carbon

double bond which is terminated by carboxyl group or a salt thereof, anhydride, ester, amide or imide; (8) a method for preparing the polystyrene derivative, characterized by polymerizing one or at least two kinds of a styrene derivative represented by the general formula (VII):

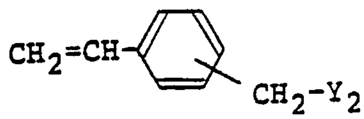

(VII)

wherein $Y_2$ is a group represented by the formula:

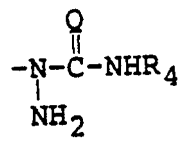

(wherein $R_4$ and Q are the same as mentioned above), or a group represented by the formula:

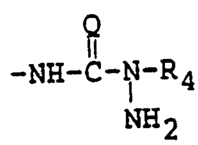

(wherein $R_4$ and Q are the same as mentioned above), or copolymerizing the above styrene derivative represented by the general formula (VII) and a styrene derivative represented by the general formula (VIII):

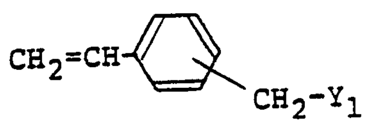

(VIII)

wherein $Y_1$ is the same as mentioned above, and carrying out a condensation reaction of an obtained copolymer with a compound represented by the general formula (III):

$R_3$-X-CHO

wherein $R_3$ and X are the same as mentioned above;
(9) a sunscreening agent of which active ingredient is the polystyrene derivative; and
(10) a cosmetic composition containing the polystyrene derivative.

BRIEF DESCRIPTION OF DRAWINGS

Fig. 1, Fig. 3, Fig. 5, Fig. 7. Fig. 9, Fig. 11, Fig. 13, Fig. 15, Fig. 17, Fig. 19, Fig. 21, Fig. 23, Fig. 25, Fig. 27, Fig. 29, Fig. 31, Fig. 33, Fig. 35, Fig. 37, Fig. 39, Fig. 13, Fig. 41, Fig. 43 and Fig. 45 are graphs showing ultraviolet adsorption spectrum of the polystyrene derivative obtained in Examples 15 to 35 and Examples 58 and 59 of the present invention, respectively; Fig. 2, Fig. 4, Fig. 6, Fig. 8, Fig. 10, Fig. 12, Fig. 14, Fig. 16, Fig. 18, Fig. 20, Fig. 22, Fig. 24, Fig. 26, Fig. 28, Fig. 30, Fig. 32, Fig. 34, Fig. 36, Fig. 38, Fig. 40, Fig. 42, Fig. 44 and Fig. 46 are graphs showing infrared transmittance spectrum of the polystyrene derivative obtained in Examples 15 to 35 and Examples 58 and 59 of the present invention, respectively.

## BEST MODE FOR CARRYING OUT THE INVENTION

A styrene derivative of the present invention is useful itself as a sunscreening agent since the styrene derivative of the present invention has an absorption wavelength range and large extinction coefficient desirable for a sunscreening agent. However, the above-mentioned styrene derivative might be absorbed through the skin as well as a commercially available sunscreening agent when the styrene derivative is used as articles for the human bodies since the styrene derivative is not a high molecular weight compound. Accordingly, the present inventors have developed a polystyrene derivative of the present invention in view of effectiveness of a property to be easily polymerized which is another characteristic of the styrene derivative.

The polystyrene derivative of the present invention has a desired absorption wavelength range as well as the styrene derivative has and a large extinction coefficient and is excellent in, for instance, safety as a cosmetic component, and can be used as a sunscreening agent having a high absorptive property of ultraviolet rays.

The styrene derivative of the present invention is a compound represented by the general formula (I):

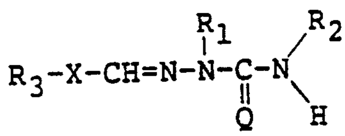

(I)

wherein one of $R_1$ and $R_2$ is vinylbenzyl group, another is hydrogen atom, a lower alkyl group, a carboxyalkyl group having 2 to 5 carbon atoms or phenyl group, $R_3$ is hydrogen atom, a halogen atom, a lower alkyl group having, for instance, 1 to 4 carbon atoms, carboxyl group, a carboxyalkyl group having 2 to 5 carbon atoms, a carboxyalkenyl group having 3 to 6 carbon atoms, an alkylamino group having 1 to 4 carbon atoms or nitro group, Q is sulfur atom or oxygen atom, X is

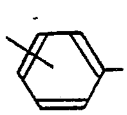 , 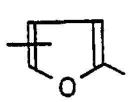 , 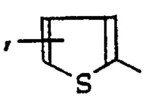 or 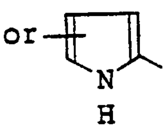 .

In the general formula (I), a partial constitutional formula (IX):

$$R_3\text{-}X\text{-}CH= \qquad (IX)$$

wherein $R_3$ and X are the same as mentiond above, is an aldehyde bonded to (thio)semicarbazide group. As a compound having the partial consitutional formula (IX), for instance, substituted aromatic aldehydes such as benzaldehyde, nitrobenzaldehyde, chlorobenzaldehyde, nitrobenzaldehyde, dimethylaminobenzaldehyde, methoxybenzaldehyde, hydroxybenzaldehyde, carboxybenzaldehyde, cinnamaldehyde and formylcinnamic acid; heterocyclic aldehydes such as thienylaldehyde, furylaldehyde, pyrollealdehyde and pyridinealdehyde; and the like can be exemplified since the compound has suitable absorption wavelength and absorbance of ultraviolet rays. When the above-mentioned partial constitutional formula (IX) is a substituted aromatic aldehyde, the substituent bonded to the substituted aromatic aldehyde can be at any position of o, m and p-positions of the aldehyde group bonded to the benzene nucleus. It is preferable that the substituent is at the p-position since ultraviolet absorptive property is particularly heightened when the substituent is at p-position. When the above-mentioned partial constitutional formula (IX) is a heterocyclic aldehyde, the aldehyde group can be at any position of 2 to 5-positions of the hetero atom.

In the general formula (I), a partial constitutional formula (X):

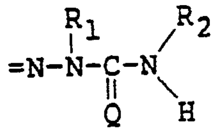

(X)

wherein $R_1$, $R_2$ and Q are the same as mentioned above, is a (thio)semicarbazide bonded to the substituted aromatic aldehyde or heterocyclic aldehyde. The vinyl group of the vinylbenzyl group can be at any position of o, m and p-positions of the methylene group bonded to the benzene ring. Examples of the above-mentioned (thio)semicarbazide are, for instance,

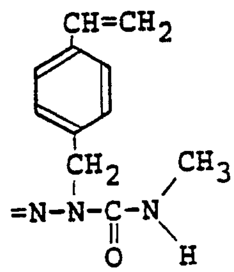

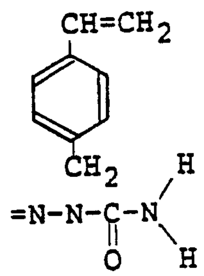

,

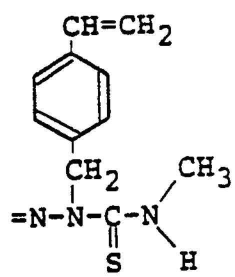

,

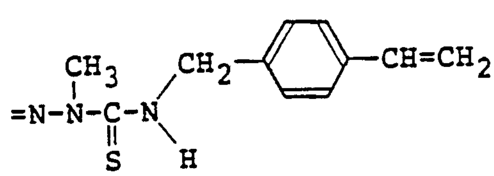

and the like.

A method for preparing the styrene derivative of the present invention is as follows.

The styrene derivative is easily prepared by a condensation reaction of a (thio)semicarbazide represented by the general formula (II):

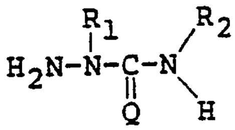

(II)

wherein $R_1$, $R_2$ and Q are the same as mentioned above with an aldehyde derivative represented by the general formula (III):

$$R_3\text{-}X\text{-}CHO \qquad (III)$$

wherein $R_3$ and X are the same as mentioned above in the presence of acids as occasion demands.

The reaction is carried out in a solvent such as methanol, ethanol, isopropanol, tetrahydrofuran or dioxane, which can sufficiently dissolve the starting materials at room temperature or with heating.

A polystyrene derivative of the present invention is obtained by polymerizing a styrene derivative represented by the general formula (I):

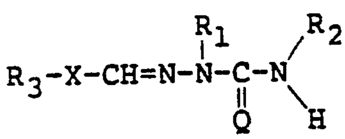

(I)

wherein $R_1$, $R_2$, $R_3$, Q and X are the same as mentioned above. The polystyrene derivative is represented by the general formula (IV):

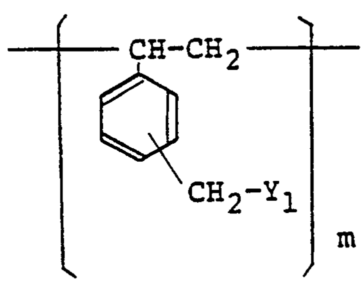

(IV)

wherein $Y_1$ is a group represented by the formula:

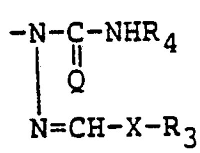

(wherein $R_3$, Q and X are the same as mentioned above, $R_4$ is hydrogen atom, a lower alkyl group, a carboxyalkyl group having 2 to 5 carbon atoms or phenyl group) or a group represented by the formula:

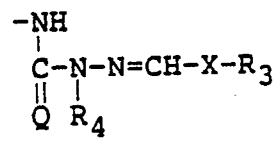

(wherein $R_3$, $R_4$ Q and X are the same as mentioned above, m is an integer of 6 to 1000).

When $R_1$ is vinylbenzyl group in the above-mentionedgeneral formula (I), the polystyrene derivative is represented by the general formula (XI):

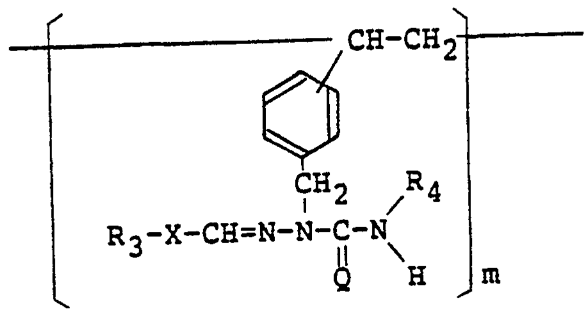

(XI)

wherein $R_3$, $R_4$, Q, X and m are the same as mentioned above. When $R_2$ is vinylbenzyl group in the general formula (I), the polystyrene derivative is represented by the general formula (XII):

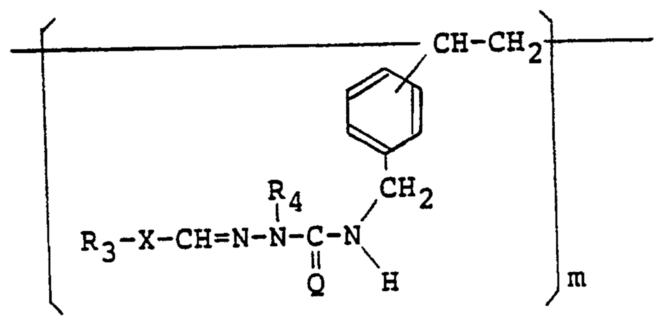

(XII)

wherein $R_3$, $R_4$, Q, X and m are the same as mentioned above.

It is desirable that carboxyl group is contained in $R_3$, that is, for example, $R_3$ is -COOH, -CH=CH-COOH or the like in the above-mentioned polystyrene derivative since the polystyrene derivative reacts with a base mentioned later to form a salt and impart water solubility. It is preferable that Q is sulfur atom since the polystyrene derivative exhibits ultraviolet absorptive property greater than the polystyrene derivative in which Q is oxygen atom. However, the polystyrene derivative in which Q is oxygen atom is very useful in some cases since the wavelength at a maximum absorption is slightly shifted toward the shorter wavelength in comparison with the polystyrene derivative in which Q is sulfur atom.

It is desirable that the above-mentioned X is a benzene ring since the ultraviolet absorptive property can be further heightened, but furan, thiophene, pyrrole ring and the like can also be used as the above-mentioned X since these exhibits ultraviolet absorptive properties. The above-mentioned m denotes a polymerization degree and is not particularly limited. However, it is desirable that m is usually 6 to 1000, preferably 20 to 1000.

The polystyrene derivative of the present invention can contain {Z} portion as represented by the general formula (XIII):

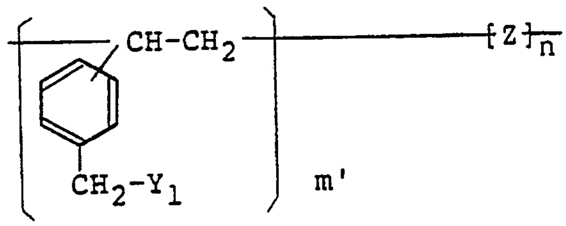

(XIII)

wherein $Y_1$ is the same as mentioned above, m' and n are positive integers, Z is mentioned later. The above-mentioned {Z} portion is introduced in order to impart water solubility to the polystyrene derivative. When the polystyrene derivative represented by the general formula (XI) or (XII) does not have water solubility or has low water solubility, this structure is employed. The water solubility is a desirable property which exhibits excellent effects such as facilitation of formulation.

Examples of the above-mentioned {Z} are, for instance,

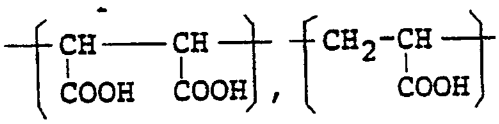

and the like.

The above-mentioned m' denotes m-n (m is the same as mentioned above). It is desirable that n satisfies that n/m' > 0.5 in order to exhibit a remarkable effect for imparting water solubility to a polystyrene derivative. It is most desirable that n usually satisfies that 2 > n/m' > 0.5 since ultraviolet absorptive property is lowered when n is extremely large.

As a method for preparing a polystyrene derivative of the present invention, there are exemplified methods in which an azo compound or a peroxide is used as a polymerization initiator and a polymerization is carried out in a solution, such as

(1) a method comprising

(i) polymerizing at least one kind of the styrene derivative of the present invention represented by the general formula (I):

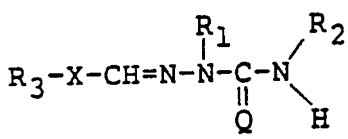

(I)

wherein $R_1$, $R_2$, $R_3$, Q and X are the same as mentioned above, or

(ii) polymerizing at least one kind of the above styrene derivative and at least one kind of a compound having unsaturated aliphatic groups containing at least one carbon-carbon double bond which is terminated by carboxyl group or a salt thereof, an anhydride, an ester, an amide or imide, such as maleic anhydride, maleic acid, fumaric acid, acrylic acid or a salt, ester amide or imide of maleic acid, fumaric acid or acrylic acid (hereinafter referred to as "monomer A"), and

(2) a method comprising

(i) polymerizing one kind or at least two kinds of a styrene derivative represented by the general formula (VII):

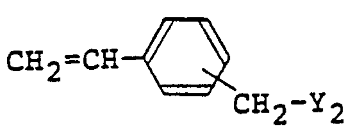

(VII)

wherein $Y_2$ is a group represented by the formula:

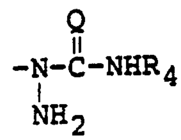

(wherein $R_4$ and Q are the same as mentioned above) or a group represented by the formula:

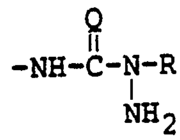

(wherein $R_4$ and Q are the same as mentioned above), or

(ii) polymerizing the styrene derivative represented by the above-mentioned general formula (VII) and a styrene derivative represented by the general formula (VIII):

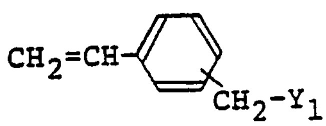

(VIII)

wherein $Y_1$ is the same as mentioned above, and carrying out a condensation reaction of an obtained polymer with the compound represented by the above-mentioned general formula (III).

Examples of the azo compound used as a polymerization initiator are, for instance, α,α'-azobisisobutyronitrile, dimethyl α,α'-azobis(isobutyrate), α,α'-azobis(2,4-dimethylvaleronitrile), azobiscyclohexanecarbonitrile, and the like. Examples of the peroxide are, for instance, benzoyl peroxide, ammonium persulfate, t-butyl hydroperoxide, di-t-butyl peroxide, and the like.

The polymerization reaction of the present invention is carried out in a solution. As a solvent used at the polymerization, any type solvent which is generally used at radical polymerization can be used, but a solvent having an ability of dissolving starting monomers and a resulting polymer is preferable. Examples of the solvent are, for instance, N,N-dimethylformamide, N,N-dimethylacetamide, N-methylpyrrolidone, tetrahydrofuran, 2-butanone, and the like.

Also, the temperature at polymerization can be room temperature to 100°C as well as the general condition at a radical polymerization, and it is preferable that the atmosphere at polymerization is replaced by inert gas such as nitrogen gas or argon gas.

When the obtained polystyrene derivative is used as an aqueous solution, it is preferable that carboxyl group contained in the polystyrene derivative is changed into a salt of an alkali metal such as lithium, sodium or potassium, ammonia, or an ammonium salt such as ethanolamine, propanolamine or triethylamine.

A salt of carboxylic acid of the above-mentioned polystyrene derivative is easily formed from the polystyrene derivative by suspending the polystyrene derivative in water and adding a base such as an alkali metal hydroxide, ammonia or an amine, and the resulting salt dissolves in water. Also, a salt of carboxylic acid of the above-mentioned polystyrene derivative containing an ester, acid anhydride or amide is formed by suspending the polystyrene derivative in water in the same manner as mentioned above and hydrolyzing with a strong base such as an alkali metal hydroxide, and water solubility is imparted to an obtained polystyrene derivative.

One example of the method for preparing the polystyrene derivative of the present invention is explained hereinafter.

As first and second methods, there are methods comprising polymerizing only the styrene derivative of the present invention. One embodiment of the reaction is explained hereinafter.

(First method)

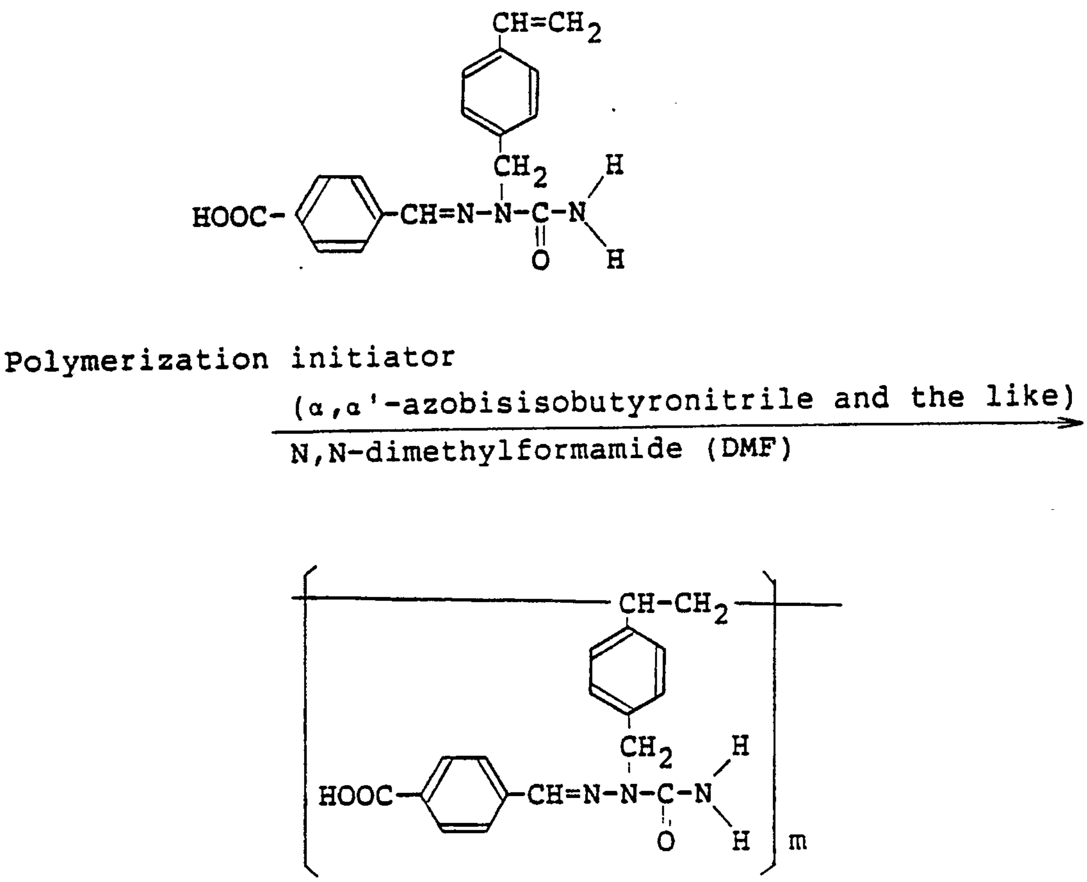


(wherein m is the same as mentioned above)

(Second method)

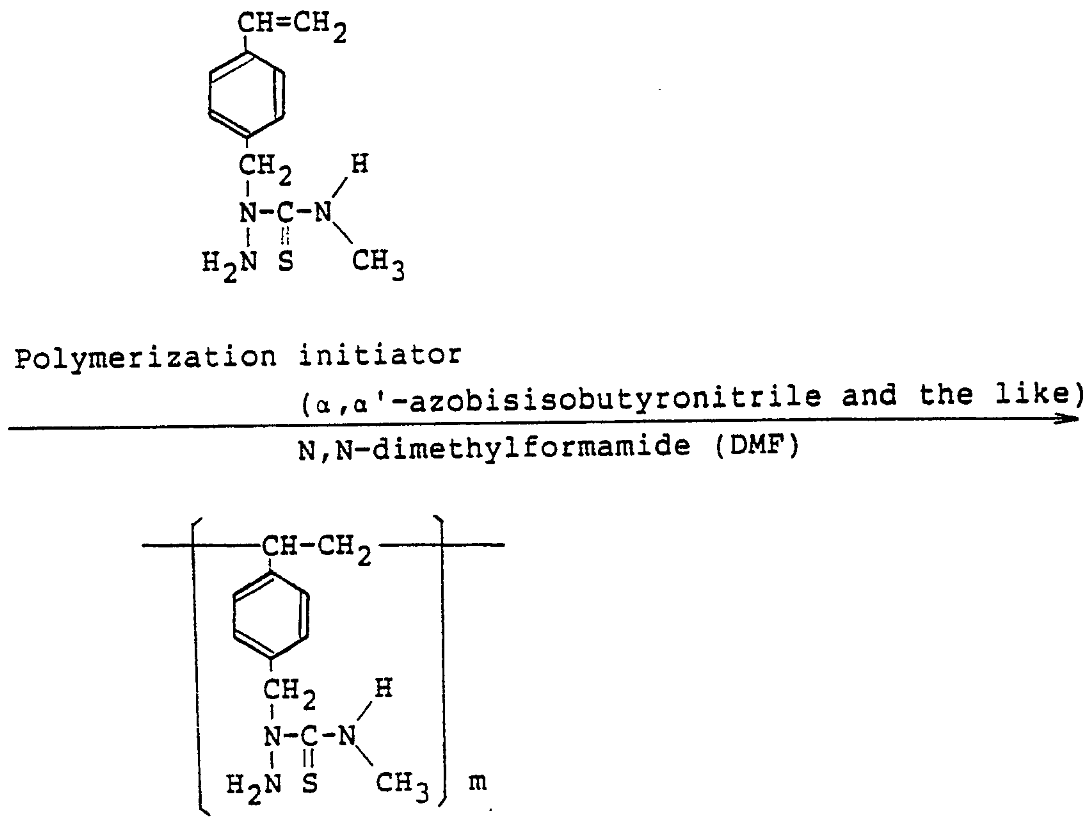


(wherein m is the same as mentioned above)

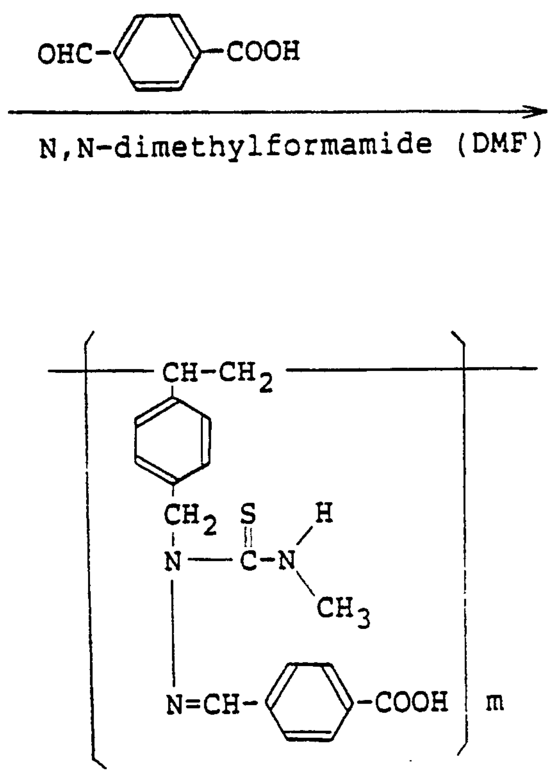


Other than the above-mentioned first and second methods, there is a method comprising polymerizing at least two kinds of the styrene derivative of the present invention, as mentioned above. The method is

explained as third method hereinafter.

(Third method)

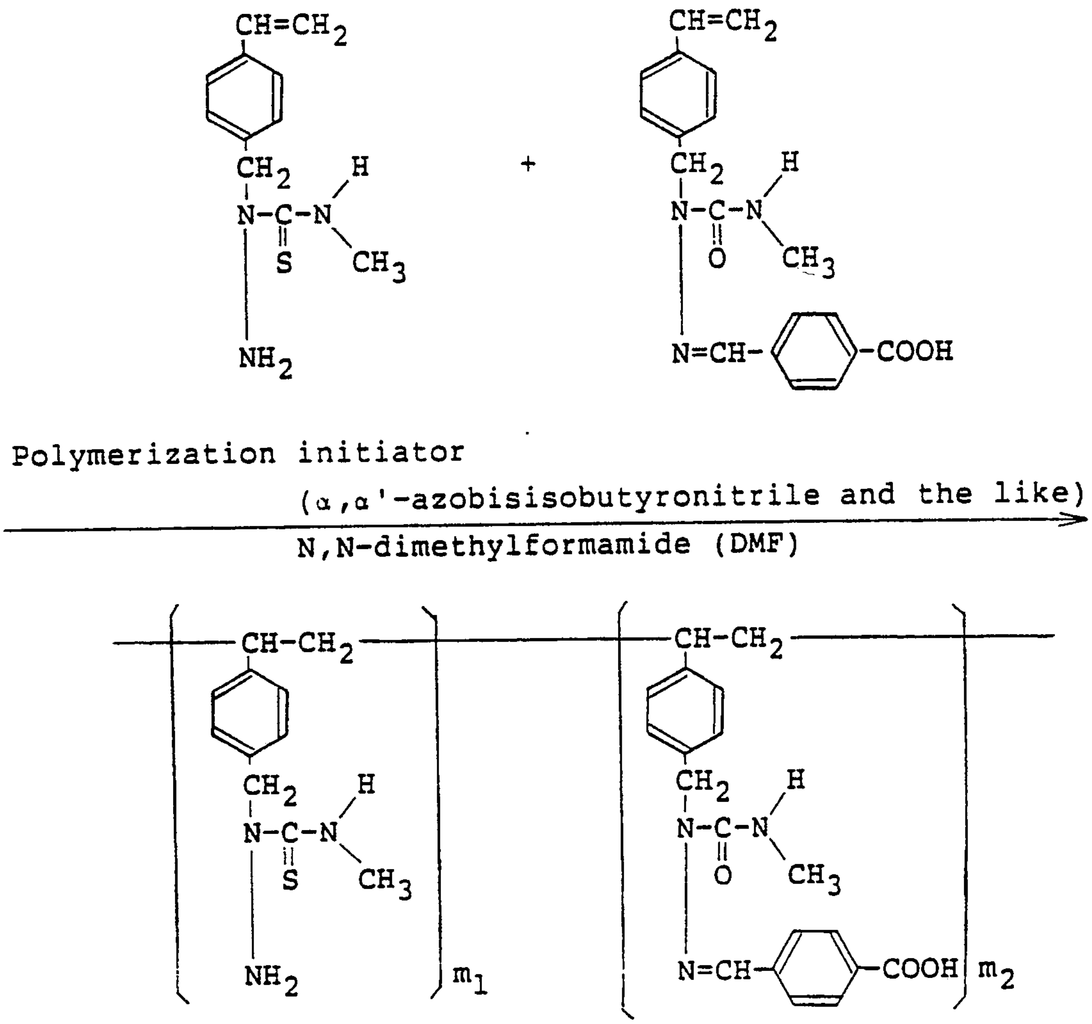


(wherein $m_1$ and $m_2$ are positive integers which satisfy $m = m_1 + m_2$, m is the same as mentioned above)

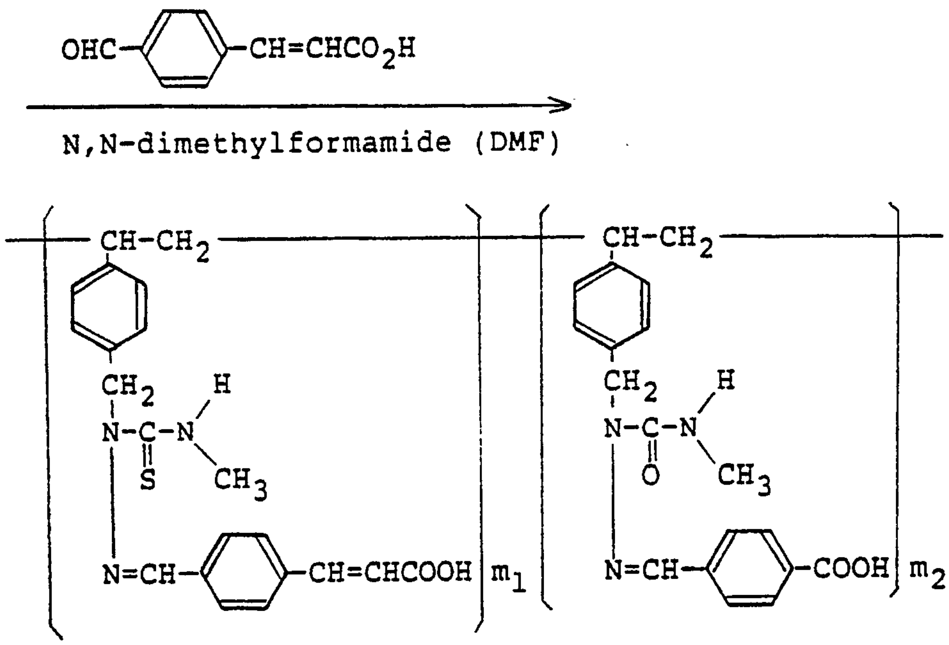


Also, as fourth method, there is a method comprising copolymerizing the styrene derivative and other monomer A. One embodiment of the reaction is explained hereinafter.

(Fourth method)

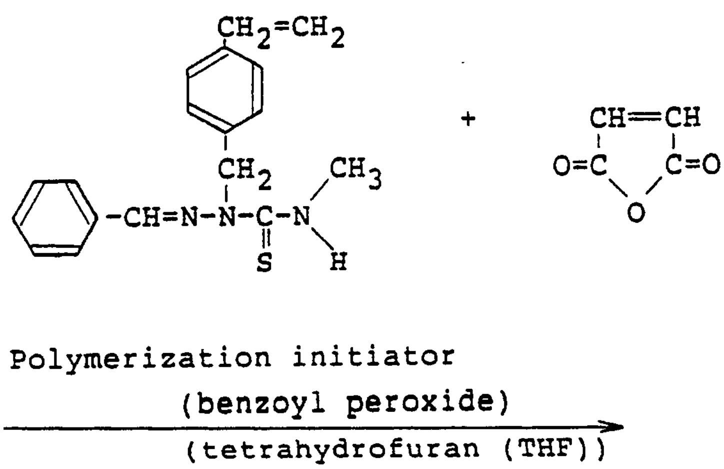

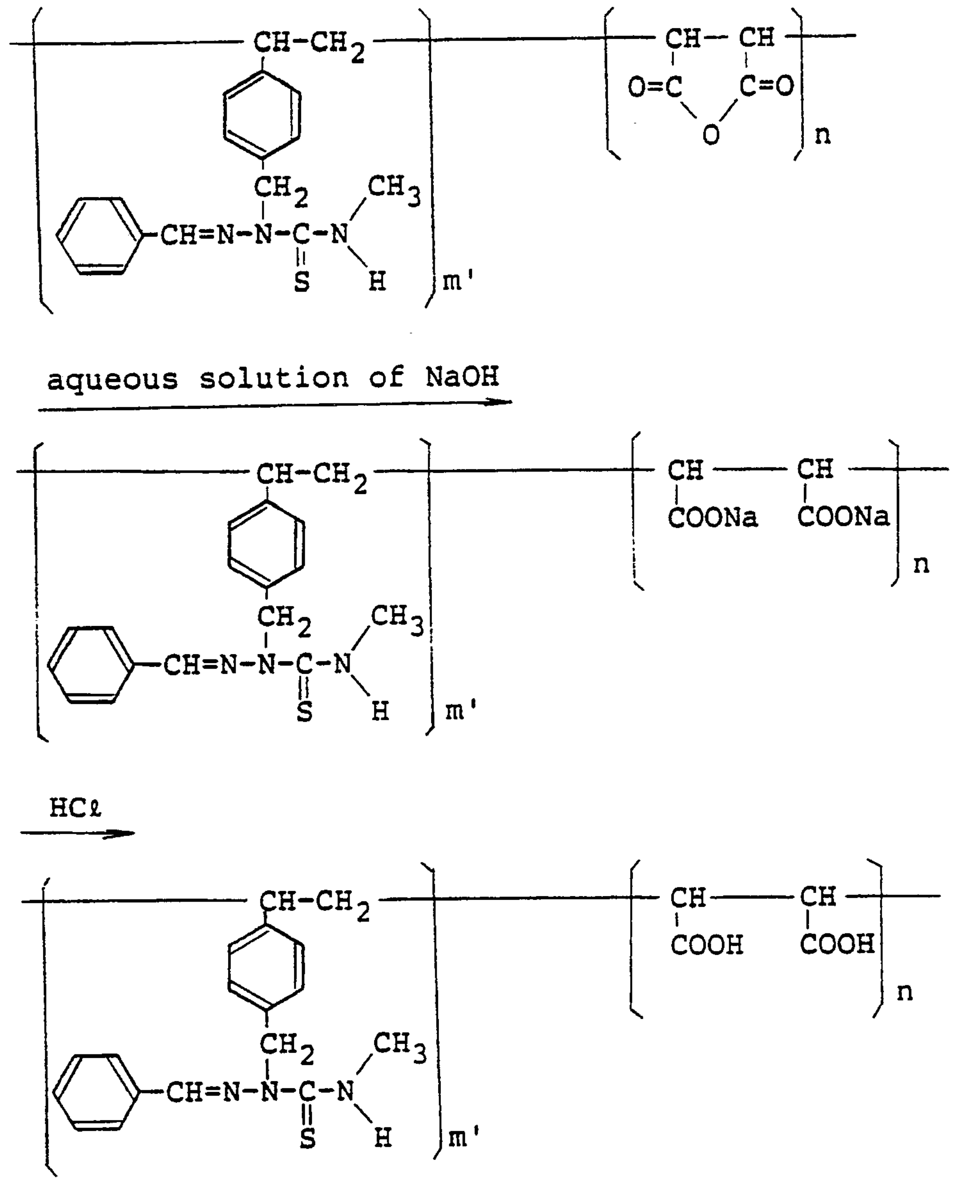


(wherein m' and n are the same as mentioned above)

In the above-mentioned second method and third method, it is preferable that the reaction of the intermediate of the polystyrene derivative of the present invention and an aldehyde is carried out with heating in a solvent having a high compatibility with the both. In this case, since water is generated as a reaction product, it is preferable that resulting water is removed, for instance, by using a drying agent or reducing the pressure of atmosphere. As the characteristics of the third method, as is clear from the above-mentioned reaction formula, when the polymerization reaction is applied to a compound containing -CH = CH-groupsuch as formylcinnamic acid, there can be cited that such group is prevented from being affected by the polymerization.

The thus obtained polystyrene derivative of the present invention is a homopolymer composed of the recurring unit represented by the general formula (XIV):

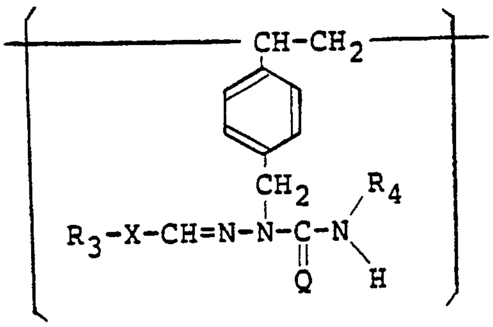

(XIV)

or the general formula (XV):

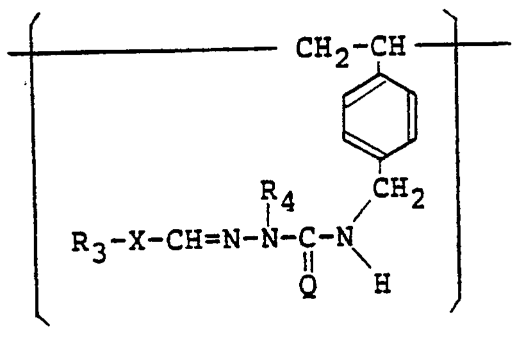

(XV)

wherein $R_3$, $R_4$, Q and X are the same as mentioned above, or a random copolymer comprising the above recurring unit and a unit represented by the above-mentioned formula {Z}. This polystyrene derivative can be preferably used as a sunscreening agent since the polystyrene derivative exhibits strong absorbance particularly within a range of wavelength of 280 to 400 nm and is very stable for heat, light and the like. The polystyrene derivative of the present invention is difficult to be absorbed through the skin to human bodies in comparison with conventional ultraviolet absorptive compounds having a low molecular weight since the polystyrene derivative is a high molecular compound as mentioned above. Accordingly, the polystyrene derivative exhibits functions and effects that safety for human bodies is highly imparted as well as the effects are continuied for a long period of time.

The polystyrene derivative of the present invention can be the following compound other than the case that the styrene derivative represented by the above-mentioned general formula (I) and a compound having unsaturated aliphatic groups containing at least one carbon-carbon double bond which contributes to polymerization is the above-mentioned Z.

That is, the compound is represented by the general formula (XVI):

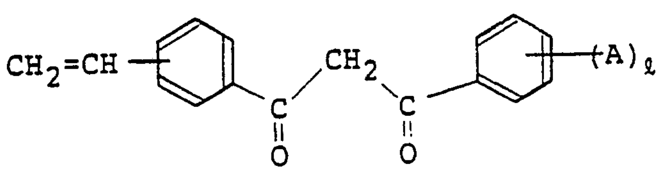

(XVI)

wherein A is a lower alkyl group, carboxyl group, a lower alkoxy group or a lower alkoxycarbonyl group, $\ell$ is an integer of 0 to 3. The compound has a benzoylmethane structure in its skeleton adsorbing UV-A effectively.

In the general formula (XVI), A is, for instance, a lower alkyl group or lower alkoxy group having 1 to 5 carbon atoms or so, a lower alkoxylcarbonyl group having 2 to 6 carbon atoms or so, carboxyl group or the like. These groups are preferable since they impart a suitable UV-A absorptive wavelength and a large absorbance to a styrene derivative. Among them, methoxy group, methoxycarbonyl group and carboxyl group are particularly preferable. In addition, $\ell$ is preferably 1 or 2.

In the styrene derivative represented by the above-mentioned general formula (XVI), a positional relationship of vinyl group and carbonyl group bonded to the benzene nucleus can be any of o-, m- and p-positions, but p-position is preferable since a styrene derivative having a high ultraviolet absorptive property

can be obtained. A positional relationship of A and carbonyl group bonded to the benzen nucleus can be any of o-, m-, p-positions, but when one of $A_1$ is bonded to the benzene nuoleus, p-position is preferable, and when two of $A_1$ are bonded to the benzene nucleus, o- and p-positions are preferable, respectively since ultraviolet absorbance of the styrene derivative comes to be greater.

In consideration of the above-mentioned description, among the styrene derivative represented by the above-mentioned general formula (XVI), in particular, a styrene derivative represented by the general formula (XVII):

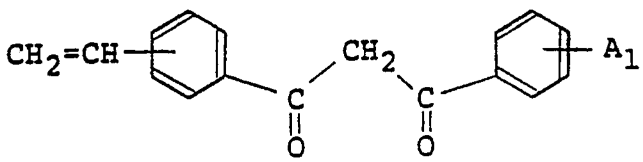

(XVII)

(wherein $A_1$ is methoxy group, methoxycarbonyl group or carboxyl group) is suitably used.

As a copolymer comprising the styrene derivative represented by the above-mentioned general formula (VIII) and the styrene derivative represented by the general formula (XVI), for instance , a copolymer represented by the general formula (VI):

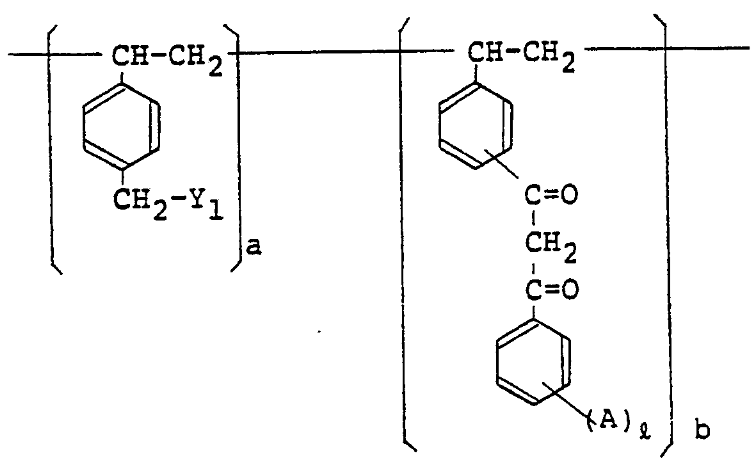

(VI)

wherein $Y_1$, A and $\ell$ are the same as mentioned above, a and b are positive integers satisfying $m = a + b$, m is the same as mentioned above, and the like can be exemplified. On the other hand, the polystyrene derivative represented by the general formula (VI) can be prepared in the same manner as mentioned above.

In the concrete, the polystyrene derivative represented by the general formula (VI) can be prepared by polymerizing at least one kind of the compound represented by the general formula (I):

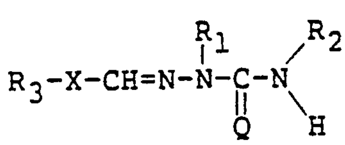

(I)

wherein $R_1$, $R_2$, $R_3$, X and Q are the same as mentioned above, and at least one kind of the compound represented by the general formula (XVI):

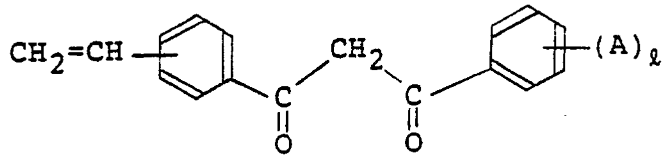

(XVI)

wherein A and ℓ are the same as mentioned above.

The polymerization is carried out in a solution at room temperature to 100° C in an atmosphere of inert gas with an azo compound or a peroxide as a polymerization initiator in the same manner as mentioned above.

The thus obtained polystyrene derivative of the present invention is concretely a random copolymer comprising the recurring unit represented by the general formula (XIV):

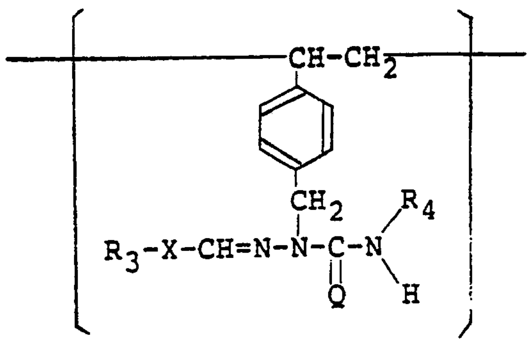

(XIV)

or the general formula (XV):

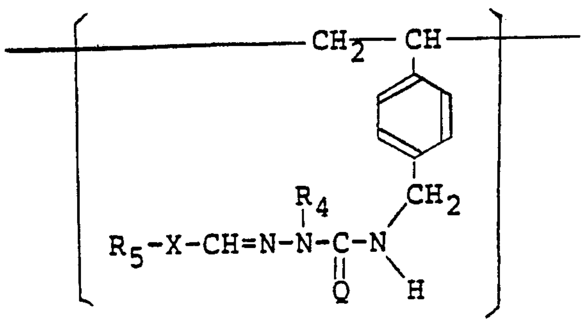

(XV)

wherein $R_3$, $R_4$ and Q are the same as mentioned above, and a recurring unit represented by the general formula (XVIII):

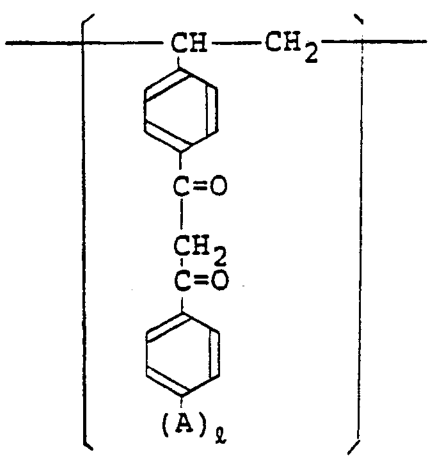

(XVIII)

wherein A and ℓ are the same as mentioned above. The polystyrene derivative can be suitably used as a sunscreening agent since the polystyrene derivative exhibits strong absorbance particularly within a range of wavelength of 280 to 400 nm and is a very stable compound for heat, light and the like. Also, the polystyrene derivative of the present invention is difficult to be absorbed through the skin to human bodies in comparison with conventional ultraviolet absorptive compounds having a low molecular weight since the polystyrene derivative is a high molecular compound as mentioned above. Accordingly, the polystyrene derivative exhibits functions and effects that safety for the human bodies is highly imparted as well as the effect is continued for a long period of time.

Any of the polystyrene derivative of the present invention can be suitably used as a sunscreening agent as mentioned above, and the polystyrene derivative can be used by dissolving in a solvent or including in other materials such as a resin material. In particular, the polystyrene derivative showing water solubility of the present invention can be suitably used in a cosmetic composition containing a sunscreening agent for human bodies in the form such as liquid or ointment in accordance with its use.

When the polystyrene derivative of the present invention is used in a liquid cosmetic composition containing a sunscreening agent, after the polystyrene derivative is changed into a salt and dissolved in water, the aqueous solution can be used as liquids such as toilet water, tonic, lotion and milky lotion with a base material such as glycerol, propylene glycol or vegetable oil. When the polystyrene derivative is used in a creamy cosmetic composition containing a sunscreening agent, after the polystyrene derivative is changed into a salt and dissolved in water, the aqueous solution is used by adding into a conventionally used base material such as an ointment prepared by emulsifying, for example, white vaseline, stearyl alcohol, propylene glycol, sodium lauryl sulfate. In addition, other sunscreening agents can be added to the base material as occasion demands.

The content of the polystyrene derivative in a cosmetic composition cannot be absolutely determined since the content is different depending upon kinds of substituent and degree of substitution of the polystyrene derivative, kinds of a base material of cosmetic composition, kinds of other sunscreening agent added as occasion demands, and the like. For instance, when the polystyrene derivative is dissolved to give a cosmetic composition, it is preferable that the content of the polystyrene derivative is adjusted to 0.1 to 50 % by weight, preferably 2 to 25 % by weight. When the content is less than 0.1 % by weight, the effect of absorption of ultraviolet rays exhibited by adding the polystyrene derivative cannot be expected, and when the content exceeds 50 % by weight, there is a tendency that disperion property of the polystyrene derivative comes to be lowered. Also, for instance, when the polystyrene derivative is suspended or dispersed in a state of solid or powder to give a cosmetic composition, it is preferable that the content of the polystyrene derivative in the cosmetic composition is adjusted to 0.1 to 50 % by weight, preferably 2 to 35 % by weight. When the content is less than 0.1 % by weight, the effect of absorption of ultraviolet rays exhibited by adding the polystyrene derivative is small, and when the content is more than 50 % by weight, improvements of effects cannot be expected although the obtained cosmetic composition can be used, and it becoms economically disadvantageous.

To the cosmetic composition containing the above-mentioned sunscreening agent, perfume, colorant, vehicle and other sunscreening agent, various raw materials for cosmetics, and the like can be suitably added.

Next, the styrene derivative and polystyrene derivative of the present invention are more specifically explained by means of the following Examples. However, the present invention is not restricted by the Examples.

## Example 1

[Preparation of p'-carboxybenzaldehyde 4-methyl-2-p-vinylbenzylthiosemicarbazone]

In 50 mℓ of ether was dissolved 7.00 g of methyl isothiocyanate, and 100 mℓ of an ether solution containing 20.00 g of p-vinylbenzylhydrazine and 200 mg of hydroquinone was addd thereto while cooling with ice. Then, the reaction was carried out at room temperature for 3 hours. After the reaction, ether was distilled away, and recrystallization was carried out with ethanol to give 17.21 g of 4-methyl-2-p-vinylbenzylthiosemicarbazide in the form of colorless needle-like crystalline (yield 81.7 %). Then, 8.60 g of the obtained compound was dissolved in 50 mℓ of tetrahydrofuran, 86 mg of hydroquinone and 0.1 mℓ of hydrochloric acid were added thereto, then 40 mℓ of a tetrahydrofuran solution of 5.97 g of terephthalaldehydic acid was added thereto, and the reaction was carried out at room temperature for 5 hours. After the

reaction, tetrahydrofuran was distilled away and the residue was recrystallized with acetone-water to give 10.50 g of colorless crystalline (yield 76.3 %).

Physical properties of the obtained crystalline were measured according to the following methods.

(Ultraviolet absorption spectrum)

The wavelength at the maximum absorption (λ max) and the molar extinction coefficient (ε) were found by measuring ultraviolet absorption spectrum of an ethanol solution of the sample having a concentration of 10 ppm in a quartz cell having an optical path of 10 mm by means of a spectrophotometer (UV-200S, made by SHIMADZU CORPORATION). The results are shown below.

Wavelength at the maximum absorption (λ max): 331 nm

Molar extinction coefficient (ε) : 33,000

(Melting point)

The measurement was carried out by means of a melting point measuring apparatus (MP type, made by Yanagimoto Co., LTD.). The result is shown below.

Melting point (mp): 245.0° to 248.0 °C

(Nuclear magnetic resonance spectrum)

The measurement was carried out by means of a high resolution nuclear magnetic resonance apparatus (R-40 type, made by Hitachi, Ltd.) with tetramethylsilane as an internal substance, as DMSO-$d_6$ (deuterated dimethyl sulfoxide) solution. The results are shown below.

$^1$H NMR (DMSO-$d_6$) δppm: 3.21 (3H, d, J = 3.75 Hz), 5.20 (1H, d, J = 10.00 Hz), 5.75 (1H, d, J = 17.50 Hz), 5.97 (2H, s), 6.70 (1H, dd, J = 10.00 and 17.50 Hz), 7.20 and 7.45 (2H, d, J = 8.75 Hz, respectively), 7.83 (1H, s), 7.97 (4H, s), 9.15 (1H, broad)

(Infrared absorption spectrum)

The measurement was carried out by means of a diffraction grating infrared spectrophotometer (IR-700 type, made by JAPAN SPECTROSCOPIC Co., LTD.) according to KBr method. The results are shown below.

IR ν max (KBr) $cm^{-1}$: 3300, 1684, 1498, 1312, 1286, 1044, 920, 905

Example 2

[Preparation of p'-carboxybenzaldehyde 2-methyl-4-P-vinylbenzylthiosemicarbazone]

In 100 mℓ of chloroform was dissolved 50.00 g of p-vinylbenzylamine and 500 mg of hydroquinone, and 18.7 mℓ of carbon disulfide and 40 mℓ of triethylamine were slowly added dropwise thereto. Then, the reaction was carried out at room temperature for 2 hours. After that, 40 mℓ of triethylamine and 28.9 mℓ of ethyl chloroformate were added again thereto in order, and the reaction was carried out for 1 hour. After the reation, 100 mℓ of water and 100 mℓ of 10 % hydrochloric acid were added thereto to separate the organic phase. Then, the organic phase was washed with water, and dried with Glauber's salt. The organic phase from which the Glauber's salt was filtered away was concentrated to give 42.00 g of a crude product. This product was dissolved in 120 mℓ of ethanol, 20 mℓ of ethanol solution of 5.00 g of methylhydrazine was added thereto while cooling with ice, and the reaction was carried out at room temperature for 2 hours. After the reaction, the crude product was recrystallized with ethanol to recover 16.9 g of 2-methyl-4-p-vinylbenzylthiosemicarbazide in the form of colorless crystalline (yield 70.0 %). In 100 mℓ of tetrahydrofuran, 6.90 g of this compound was reacted with 4.50 g of terephthalaldehydic acid in the presence of 69 mg of hydroquinone and 0.1 mℓ of hydrochloric acid in the same manner as in Example 1

to give 9.19 g of colorless crystalline (yield 83.8 %). Physical properties of the obtained crystalline were measured in the same manner as in Example 1. The results are shown below.

(Ultraviolet absoprtion spectrum)

Wavelength at the maximum absorption (λ max) : 332 nm
Molar extinction coefficient (ε) : 40,000

(Melting point)

Melting point (mp): 220.5° to 222.0 °C

(Nuclear magnetic resonance spectrum)

$^1$H NMR (DMSO-$d_6$) δppm: 3.90 (3H, s), 4.95 (2H, d, J = 6.25 Hz), 5.20 (1H, d, J = 10.00 Hz), 5.77 (1H, d, J = 17.50 Hz), 6.77 (1H, dd, J = 10.00 and 17.50 Hz), 7.37 and 7.39 (2H, s, respectively), 8.03 and 8.05 (2H, s, respectively), 8.01 (1H, s), 9.53 (1H, broad)

(Infrared absorption spectrum)

IR ν max (KBr) $cm^{-1}$: 3330, 1680, 1520, 1418, 1358, 1284, 1100, 975, 910

Example 3

[Preparation of p'-carboxyvinylenebenzaldehyde 4-methyl-2-p-vinylbenzylthiosemicarbazone]

In the same procedure as in Example 1, 5.05 g of 4-methyl 2-p-vinylbenzylthiosemicarbazide obtained in Example 1 and 4.00 g of p-formylcinnamic acid were reacted in 250 mℓ of dioxane in the presence of 0.5 mℓ of hydrochloric acid and 35 mg of hydroquinone, and recrystallization was carried out with dioxane-water to give 7.70 g of colorless crystalline (yield 90.4 %). Physical properties of the obtained crystalline were measured in the same manner as in Example 1. The results are shown below.

(Ultraviolet absorption spectrum)

Wavelength at the maximum absorption (λ max): 348 nm
Molar extinction coefficient (ε): 60,000

(Melting point)

Melting point (mp): 216.0° to 222.0 °C

(Nuclear magnetic resonance spectrum)

$^1$H NMR (Acetone-$d_6$) δ ppm: 3.27 (3H, d, J = 5.00 Hz), 5.20 (1H, d, J = 10.00 Hz), 5.75 (1H, d, J = 17.50 Hz), 6.03 (2H, s), 6.50 (1H, d, J = 15.00 Hz), 6.73 (1H, dd, J = 10.00 and 17.50 Hz), 7.30 and 7.45 (2H, d, J = 8.75 Hz, respectively), 7.63 and 7.75 (2H, d, J = 8.85 Hz, respectively), 7.81 (1H, s), 8.95 (1H, broad)

(Infrared absorption spectrum)

IR ν max (KBr) $cm^{-1}$: 3360, 1680, 1624, 1516, 1358, 1104, 978, 918, 820

Example 4

[Preparation of p'-carboxybenzaldehyde 2-p-vinylbenzylsemicarbazone]

In a mixed solvent of 300 mℓ of water and 30 mℓ of ethanol was dissolved 21.50 g of p-vinylbenzylhydrazine, and 8.50 g of sodium cyanate (purity at least 85 %, made by WAKO PURE CHEMICAL INDUSTRIES, LTD.) was added thereto in a state of powder little by little while stirring and cooling with water for 1 hour. During the addition, pH was adjusted to about 7 by adding dilute hydrochloric acid. After the addition, stirring was continued for 3 hours, and then a product was collected by filtration and dried. After that, the product was recrystallized by using 120 mℓ of ethanol to give 15.10 g of 2-p-vinylbenzylsemicarbazide in the form of colorless needle-like crystalline (yield 59.0 %). Then, 10.00 g of the obtained one was dissolved in 200 mℓ of ethanol, 7.50 g of terephthalaldehydic acid was added thereto and the reaction was carried out at 50 °C for 5 hours. After that, it was cooled with ice and water, the reaction product was collected by filtration and recrystallized with 400 mℓ of ethanol to give 13.20 g of colorless crystalline (yield 81.7 %). Physical properties of the obtained crystalline were measured in the same manner as in Example 1. The results are shown below.

(Ultraviolet absorption spectrum)

Wavelength at the maximum absorption (λ max): 306 nm
Molar extinction coefficient (ε): 29,000

(Melting point)

Melting point (mp): 231.0° to 237.0°C

(Nuclear magnetic resonance spectrum)

$^1$N NMR (DSMO-$d_6$) δ ppm: 5.20 (1H, d, J = 10.00 Hz), 5.23 (2H, s), 5.75 (1H, d, J = 17.50 Hz), 6.70 (1H, dd, J = 10.00 and 17.50 Hz), 7.03 (2H, broad), 7.25 and 7.43 (2H, d, J = 8.75 Hz, respectively), 7.67 (1H, s), 7.79 and 7.81 (2H, s, respectively)

(Infrared absorption spectrum)

IR ν max (KBr) $cm^{-1}$: 3476, 3356, 1674, 1596, 1552, 1432, 1412, 1252, 1224, 926, 906, 766

Example 5

[Preparation of p'-carboxybenzaldehyde 4-methyl-2-p-vinylbenzylsemicarbazone]

In the same manner as in Example 1, after 7.45 g of methyl isocyanate and 21.50 g of p-vinylbenzylhydrazine were reacted in 130 mℓ of ether at room temperature for 3 hours, a crude product was recrystallized with 60 mℓ of ethanol to give 15.31 g of 4-methyl-2-p-vinylbenzylsemicarbazide in the form of colorless needle-like crystalline (yield 57.3 %). After 10.25 g of the obtained compound was reacted with 7.30 g of terephthalaldehydic acid in 200 mℓ of ethanol at 50 °C for 5 hours, it was cooled with ice and water, and the reaction product was collected by filtration. Then, the reaction product was recrystallized with 350 mℓ of ethanol to give 13.65 g of colorless crystalline (yield 83.2 %).

Physical properties of the obtained crystalline were measured in the same manner as in Example 1. The results are shown below.

(Ultraviolet absorption spectrum)

Wavelength at the maximum absorption (λ max) : 310 nm
Molar extinction coefficient (ε): 29,000

(Melting point)

Melting point (mp): 227.5° to 230.0 °C

(Nuclear magnetic resonance spectrum)

$^1$H NMR (DMSO-$d_6$) δ ppm: 2.89 (3H, d, J = 5.00 Hz), 5.20 (1H, d, J = 10.00 Hz), 5.25 (2H, s), 5.75 (1H, d, J = 17.50 Hz), 6.70 (1H, dd, J = 10.00 and 17.50 Hz), 7.25 and 7.43 (2H, d, J = 8.75, respectively), 7.67 (1H, s), 7.90 and 7.93 (2H, s, respectively), 8.17 (1H, broad)

(Infrared absorption spectrum)

IR ν max (KBr) $cm^{-1}$: 3364, 1672, 1586, 1508, 1420, 1286, 1126, 1106, 920, 906, 770

Example 6

[Preparation of benzaldehyde 4-methyl-2-p-vinylbenzylthiosemicarbazone]

In 50 mℓ of ethanol was dissolved 5.00 g of 4-methyl-2-p-vinylbenzylthiosemicarbazone, and 0.5 mℓ of hydrochloric acid and 50 mg of hydroquinone were added thereto. Then, 30 mℓ of ethanol solution of 2.62 g of benzaldehyde was slowly added dropwise thereto. After the addition, the reaction was carried out at room temperature for 3 hours, and then recrystallization was carried out with ethanol-water to give 6.58 g of colorless crystalline (yield 89.0 %). Physical properties of the obtained crystalline were measured in the same manner as in Example 1. The results are shown below.

(Ultraviolet absorption spectrum)

Wavelength at the maximum absorption (λ max): 317 nm
Molar extinction coefficient (ε): 33,000

(Melting point)

Melting point (mp): 174.5° to 175.5 °C

(Nuclear magnetic resonance spectrum)

$^1$H NMR (CDC$\ell_3$) δ ppm: 3.31 (3H, d, J =5.00 Hz), 5.17 (1H, d, J = 10.00 Hz), 5.67 (1H, d, J = 17.50 Hz), 5.89 (2H, s), 6.67 (1H, dd, J = 10.00 and 17.50 Hz), 7.13 to 7.53 (8H, m), 7.57 (1H, s), 8.37 (1H, broad)

(Infrared absorption spectrum)

IR ν max (KBr) $cm^{-1}$: 3352, 1520, 1358, 1110, 978, 914

Example 7

[Preparation of p'-methylbenzaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 2.09 g of p-methylbenzaldehyde were reacted in 50 mℓ of ethanol in the presence of 0.5 mℓ of hydrochloric acid and 35 mg of hydroquinone, and recrystallization was carried out with ethanol-water to give 4.65 g of colorless crystalline (yield 90.0 %). Physical properties of the obtained crystalline were measured in the same manner as in Example 1. The results are shown below.

(Ultraviolet absorption spectrum)

Wavelength at the maximum absorption (λ max): 317 nm
Molar extinction coefficient (ε): 33,000

(Melting point)

Melting point (mp): 103.0° to 106.5 °C

(Nuclear magnetic resonance spectrum)

$^1$H NMR (CDCℓ$_3$) δ ppm: 2.30 (3H, s), 3.30 (3H, d, J = 5.00 Hz), 5.19 (1H, d, J = 10.00 Hz), 5.89 (2H, s), 5.67 (1H, d, J = 17.50 Hz), 6.67 (1H, dd, J = 10.00 and 17.50 Hz), 7.15 and 7.27 (2H, d, J = 8.75 Hz, respectively), 7.25 and 7.45 (2H, d, J = 8.75 Hz, respectively), 7.59 (1H, s), 8.37 (1H, broad)

(Infrared absorption spectrum)

IR ν max (KBr) cm$^{-1}$: 3304, 1520, 1358, 1188, 1104, 974, 910

Example 8

[Preparation of p'-dimethylaminobenzaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 2.33 g of p-dimethylaminobenzaldehyde were reacted in 100 mℓ of isopropanol in the presence of 0.5 mℓ of hydrochloric acid and 35 mg of hydroquinone, and recrystallization was carried out with isopropanol-water to give 4.00 g of colorless crystalline (yield 75.0 %).

Physical properties of the obtained crystalline were measured in the same manner as in Example 1. The results are shown below.

(Ultraviolet absorption spectrum)

Wavelength at the maximum absorption (λ max): 358 nm
Molar extinction coefficient (ε): 38,000

(Melting point)

Melting point (mp): 164.0° to 165.0 °C

(Nuclear magnetic resonance spectrum)

$^1$H NMR (CDCℓ$_3$) δ ppm: 2.95 (6H, s), 3.30 (3H, d, J = 5.00 Hz), 5.19 (1H, d, J = 10.00 Hz), 5.67 (1H, d, J = 17.50 Hz), 5.87 (2H, s), 6.69 (1H, dd, J = 10.00 and 17.50 Hz), 7.13 to 7.50 (8H, m), 7.53 (1H, s), 8.35 (1H, broad)

(Infrared absorption spectrum)

IR ν max (KBr) $cm^{-1}$: 3332, 1594, 1522, 1354, 1180, 1110, 986, 812

Example 9

[Preparation of p'-chlorobenzaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 2.44 g of p-chlrobenzaldehyde were reacted in 50 mℓ of ethanol in the presence of 0.5 mℓ of hydrochloric acid and 35 mg of hydroquinone, and recrystallization was carried out with ethanol-water to give 4.61 g of colorless crystalline (yield 84.8 %).

Physical properties of the obtained crystalline were measured in the same manner as in Example 1. The results are shown below.

(Ultraviolet absorption spectrum)

Wavelength at the maximum absorption (λ max): 318 nm
Molar extinction coefficient (ε): 41,000

(Melting point)

Melting point (mp): 169.0° to 170.5° C

(Nuclear magnetic resonance spectrum)

$^1$H NMR ($CDCℓ_3$) δ ppm: 3.31 (3H, d, J = 5.00 Hz), 5.21 (1H, d, J = 10.00 Hz), 5.70 (1H, d, J = 17.50 Hz), 5.89 (2H, s), 6.69 (1H, dd, J = 10.00 and 17.50 Hz), 7.19 and 7.37 (2H, d, J = 8.75 Hz, respectively), 7.35 and 7.43 (2H, s, respectively), 7.53 (1H, s), 8.33 (1H, broad)

(Infrared absorption spectrum)

IR ν max (KBr) $cm^{-1}$: 3292, 1526, 1360, 1184, 1090, 974, 910, 818

Example 10

[Preparation of p'-nitrobenzaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 2.63 of p-nitrobenzaldehyde were reacted in 100 mℓ of ethanol in the presence of 0.5 mℓ of hydrochloric acid and 35 mg of hydroquinone. Then, recrystallization was carried out with methanol-water to give 3.91 g of colorless crystalline (yield 70.0 %).

Physical properties of the obtained crystalline were measured in the same manner as in Example 1. The results are shown below.

(Ultraviolet absorption spectrum)

Wavelength at the maximum absorption (λ max): 359 nm
Molar extinction coefficient (ε): 36,000

(Melting point)

Melting point (mp): 189.5° to 191.0° C

(Nuclear magnetic resonance spectrum)

$^1$H NMR ($CDCl_3$) $\delta$ ppm: 3.35 (3H, d, J = 5.00 Hz), 5.21 (1H, d, J = 10.00 Hz), 5.70 (1H, d, J = 17.50 Hz), 5.93 (2H, s), 6.69 (1H, dd, J = 10.00 and 17.50 Hz), 7.17 and 7.37 (2H, d, J = 8.75 Hz, respectively), 7.60 (1H, s), 7.67 and 8.09 (2H, d, J = 8.75 Hz, respectively), 8.35 (1H, broad)

(Infrared absorption spectrum)

IR $\nu$ max (KBr) $cm^{-1}$: 3364, 1506, 1360, 1336, 1316, 1069, 920, 900, 842

Example 11

[Preparation of 2'-furylaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 1.67 g of 2-furylaldehyde were reacted in 50 mℓ of ethanol in the presence of 0.5 mℓ of hydrochloric acid and 35 mg of hydroquinone. Then, recrystallization was carried out with ethanol-water to give 4.16 g of colorless crystalline (yield 87.7 %).

Physical properties of the obtained crystalline were measured in the same manner as in Example 1. The results are shown below.

(Ultraviolet absorption spectrum)

Wavelength at the maximum absorption ($\lambda$ max): 321 nm
Molar extinction coefficient ($\epsilon$): 34,000

(Melting point)

Melting point (mp): 128.5° to 130.0° C

(Nuclear magnetic resonance spectrum)

$^1$H NMR ($CDCl_3$) $\delta$ ppm: 3.30 (3H, d, J = 5.00 Hz), 5.20 (1H, d, J = 10.00 Hz), 5.70 (1H, d, J = 17.50 Hz), 5.87 (2H, s), 6.43 (1H, m), 6.55 (2H, m), 6.69 (1H, dd, J = 10.00 and 17.50 Hz) 7.17 and 7.37 (2H, d, J = 8.75 Hz, respectively), 7.41 (1H, s), 8.40 (1H, m)

(Infrared absorption spectrum)

IR $\nu$ max (KBr) $cm^{-1}$: 3344, 1520, 1358, 1307, 1186, 1110, 980, 900, 750

Example 12

[Preparation of 2'-thienylaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 1.95 g of 2-thienylaldehyde were reacted in 50 mℓ of ethanol in the presence of 0.5 mℓ of hydrochloric acid and 35 mg of hydroquinone. Then, recrystallization was carried out with ethanol-water to give 4.12 g of colorless crystalline (yield 89.1 %).

Physical properties of the obtained crystalline were measured in the same manner as in Example 1. The results are shown below.

(Ultraviolet absorption spectrum)

Wavelength at the maximum absorption (λ max): 333 nm
Molar extinction coefficient (ε): 39,000

(Melting point)

Melting point (mp): 128.0° to 130.0° C

(Nuclear magnetic resonance spectrum)

$^1$N NMR ($CDCl_3$) δ ppm: 3.31 (3H, d, J = 5.00 Hz), 5.15 (1H, d, J = 10.00 Hz). 5.70 (1H, d, J = 17.50 Hz), 5.90 (2H, s), 6.69 (1H, dd, J = 10.00 and 17.50 Hz), 7.17 and 7.37 (2H, d, J = 8.75 Hz, respectively), 7.30 (1H, m), 7.35 and 7.41 (1H, d, J= 6.25 Hz, respectively), 7.53 (1H, s), 8.33 (1H, broad)

(Infrared absorption spectrum)

IR ν max (KBr) $cm^{-1}$: 3432, 1622, 1520, 1420, 1358, 978, 904, 778

Example 13

[Preparation of isonicotinic aldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 1.86 g of isonicotinicaldehyde were reacted in 50 mℓ of isopropanol in the presence of 0.5 mℓ of hydrochloric acid and 35 mg of hydroquinone. Then, recrystallization was carried out with isopropanol-water to give 3.76 g of colorless crystalline (yield 76.5 %).

Physical properties of the obtained crystalline were measured in the same manner as in Example 1. The results are shown below.

(Ultraviolet absorption spectrum)

Wavelength at the maximum absorption (λ max): 325 nm
Molar extinction coefficient (ε): 36,000

(Melting point)

Melting point (mp): 162.5° to 164.5° C

(Nuclear magnetic resonance spectrum)

$^1$H NMR ($CDCl_3$) δ ppm: 3.33 (3H, d, J = 5.00 Hz), 5.21 (1H, d, J = 10.00 Hz), 5.70 (1H, d, J = 17.50 Hz), 5.90 (2H, s), 6.69 (1H, dd, J = 10.00 and 17.50 Hz), 7.17 and 7.37 (2H, d, J = 8.75 Hz, respectively), 7.36 and 8.59 (2H, d, J= 6.25 Hz, respectively), 7.49 (1H, s), 8.37 (1H, broad)

(Infrared absorption spectrum)

IR ν max (KBr) $cm^{-1}$: 3350, 1590, 1514, 1110, 1084, 982, 924, 906, 816

Example 14

[Preparation of cinnamaldehyde 4-methyl-2-p-vinylbenzylthiosemicarbazone]

In the same procedure as in Example 6, 3.50 g of 4-methyl-2-p-vinylbenzylthiosemicarbazide obtained in Example 1 and 2.30 g of cinnamaldehyde were reacted in 50 mℓ of ethanol in the presence of 0.5 mℓ of hydrochloric acid and 35 mg of hydroquinone. Then, recrystallization was carried out with ethanol-water to give 5.19 g of colorless crystalline (yield 97.9 %).

Physical properties of the obtained crystalline were measured in the same manner as in Example 1. The results are shown below.

(Ultraviolet absorption spectrum)

Wavelength at the maximum absorption (λ max): 335 nm
Molar extinction coefficient (ε): 46,000

(Melting point)

Melting point (mp): 155.0° to 156.5° C

(Nuclear magnetic resonance spectrum)

$^1$H NMR ($CDCℓ_3$) δ ppm: 3.29 (3H, d, J = 5.00 Hz), 5.21 (1H, d, J = 10.00 Hz), 5.70 (1H, d, J = 17.50 Hz), 5.85 (2H, s), 6.70 (1H, dd, J = 10.00 Hz and 17.50 Hz), 6.73 and 6.75 (1H, s, respectively), 7.17 (2H, d, J = 8.75 Hz), 7.35 (7H, broad, s), 7.43 (1H, s), 8.35 (1H, broad)

(Infrared absorption spectrum)

IR ν max (KBr) $cm^{-1}$: 3244, 1524, 1358, 1190, 980, 964, 914, 746, 686

Example 15

[Preparation of poly(p'-carboxybenzaldehyde 4-methyl-2-P-vinylbenzylthiosemicarbazone)]

In 14 mℓ of N,N-dimethylformamide was dissolved 3.50 g of p'-carboxybenzaldehyde 4-methyl-2-p-vinylbenzylthiosemicarbazone obtained in Example 1. While passing dry nitrogen gas, the solution was heated to 60° C. Then, 1 mℓ of an N,N-dimethylformamide solution of 70 mg of α,α'-azobisisobutyronitrile was added thereto, and the reaction was carried out at 60° to 62° C for 24 hours. After the reaction, the reaction solution was cooled with ice, poured into 200 mℓ of ethyl acetate, and the resulting polymer was precipitated and recovered. The polymer was dissolved again in 5 mℓ of N,N-dimethylformamide, and the solution was poured into 200 mℓ of ethyl acetate to reprecipitate and recover the polymer. Then, the polymer was dried under vacuum to give 2.89 g of light yellow powder (yield 82.5 %).

Physical properties of the obtained product were measured in the same manner as in Example 1. Also, weight average molecular weight and elementary analysis were measured in accordance with the following methods. The result is shown below.

(Weight average molecular weight)

Suitable amount of the sample was dissolved in a 0.01N NaOH aqueous solution, and then analysis was carried out by means of a high performance liquid chromatography (made by Toso CO., LTD., pump: CCPE, column: TSK gel G $4000SW_{XL}$ (7.8 x 300 mm) + G $2000SW_{XL}$ (7.8 X 300 mm)), and water soluble polystyrene (made by SHOWA DENKO K.K., weight average molecular weight: 690,000 to 1,600) was used as a standard substance. The result is shown below.

Weight average molecular weight ($\overline{Mw}$): 185,000

(Elementary analysis)

Carbon, hydrogen and nitrogen were analyzed at the same time by means of an elementary analyzer (240° C, made by Perkin-elmer Corp.), and sulfur was analyzed by oxygen-flask method. The results are shown below.

C: 64.41 %, H: 5.39 %, N: 11.92 %, S: 9.10 %

(Thermal decomposition temperature)

The decomposition temperature of a product was measured when the product was heated from room temperature to 300° C by means of a melting point measuring apparatus (MP type, made by Yanagimoto CO., LTD.). The results are shown below.

Decomposition temperature: 240° C

(Ultraviolet absorption spectrum)

The measurement was carried out in a DMF solution. The result of the measurement is shown in Fig. 1.

(Infrared absorption spectrum)

The result of the measurement is shown in Fig. 2.

Next, with respect to the polystyrene derivative obtained in Example 15, acute toxicity and local stimulation were examined according to the following methods. The results are as follows:

(Acute toxicity)

Oral administration was conducted against mice, and $LD_{50}$ was found. Concerning $LD_{50}$, no mouse died even by the administration of 2000 mg/kg, and the mice recovered as good as normal mice on the next day of the administration. And no sex difference was observed.

(Local stimulation)

(a) Into the conjunctival sac of a rabbit, 0.1 mℓ of a 10 % aqueous solution of the triethanolamine salt of the obtained polystyrene derivative was dropped, and the stimulation was measured according to the criteria for the judgements of Draize and Kay and Calandra. As the result, it was judged that there was no stimulation. (b) A scratch was given to the notal skin of a guinea pig with a sterilized injection needle of 18 guage, and thereon an adhesive tape used for a patch test impregnated with 1 mℓ of a 10 % aqueous solution of the triethanolamine salt of the above-mentioned polystyrene derivative was applied. Then, the estimation of the stimulation was conducted according to the criteria of Draize. As the result, it was judged by the Draize method that the stimulation was mild even when a 10 % aqueous solution was used, and in fact, there was no practical stimulation.

(Percutaneous absorption)

The skin of a quinea pig was peeled, washed with physiological saline and then set in a percutaneous absopriton apparatus which was made in accordance with the description in Franz, T. J, J. Invest, Dermotal 64 , 190, 1975, by means of a diffusion cell. The sample room was filled with 0.5 mℓ of a 1 % aqueous solution of sodium salt of the obtained polystyrene derivative, and the acceptor was filled with physiological saline. After maintaining at 33° C for 20 hours while stirring with a magnetic stirrier, the physiological saline in the acceptor was taken out, and the ultraviolet absorbency was measured. As the result, when the polystyrene derivative was used, the absorption curve of the ultraviolet absorption spectrum was similar to that obtained when the physiological saline was solely used as a negative control. When a compound

having a lower molecular weight (1 % aqueous solution of sodium salt of p-aminobenzoic acid) was used as a positive control, the compound was observed by the ultraviolet absorption spectrum corresponding to 95 ppm in connection.

From the results mentioned above, it is confirmed that the polystyrene derivative of the present invention does not permeate into skin.

From the above-mentioned, it is confirmed that the polystyrene derivative of the present invention does not involve stimulation or percutaneous absorption, and is useful not only for ordinary sunscreen but also for protecting exposed skin of patients of hypersensitiveness against sunlight.

## Example 16

[Preparation of a copolymer of (p'-carboxybenzaldehyde 4-methyl-2-p-vinylbenzylthiosemicarbazone) and maleic acid]

In 18 mℓ of dry N,N-dimethylformamide were dissolved 3.50 g of p'-carboxybenzaldehyde 4-methyl-2-p-vinylbenzylthiosemicarbazone obtained in Example 1 and 0.97 g of maleic anhydride. After the solution was heated to 60 °C while passing through dry nitrogen gas, 1 mℓ of an N,N-dimethylformamide solution of 89 mg of $\alpha,\alpha'$-azobisisobutyronitrilewas added thereto. Then, the reaction was carried out at 60° to 62°C for 24 hours. After the reaction, the reaction solution was cooled with ice. Then, the reaction solution was poured into 200 mℓ of methanol, and the resulting polymer was precipitated and recovered. The polymer was dissolved again in 7 mℓ of N,N-dimethylformamide, poured into 200 mℓ of methanol and reprecipitated. Then, the polymer was collected by filtration, and dried under vacuum to give 3.60 g of light yellow powder (yield 82.0 %). To 50 mℓ of 0.05 % NaOH aqueous solution was added 3.50 g of the obtained copolymer, and the reaction was carried out for 4 hours. Then, the reaction solution was diluted with 100 mℓ of water and poured into a cellophane tube (made by WAKO PURE CHEMICAL INDUSTRY) to conduct dialysis three times with 5 ℓ of deionized water. After pH of the dialyzed solution in the tube was adjusted to 2 by adding 1N hydrochloric acid, the produced precipitate was collected by filtration and dried under vacuum to give 2.78 g of light yellow powder (yield 79.5 %).

Physical properties of the obtained product were measured in the same manner as in Example 1 and Example 15. The results are shown below.

(Weight average molecular weight)

Weight average molecular weight ($\overline{Mw}$): 4,000

(Value of elementary analysis)

C: 59.00 %, H: 5.01 %, N: 8.99 %, S: 6.51 %

(Thermal decomposition temperature)

Decomposition temperature: 255°C

(Ultraviolet absorption spectrum)

The measurement was carried out as a DMF solution. The result of the measurement is shown in Fig. 3.

(Infrared absorption spectrum)

The result of the measurement is shown in Fig. 4.

Example 17

[Preparation of poly(p'-carboxybenzaldehyde 2-methyl-4-p-vinylbenzylthiosemicarbazone)]

In the same manner as in Example 15, 3.00 g of p' -carboxybenzaldehyde 2-methyl-4-p-vinylbenzylthiosemicarbazone obtained in Example 2 was subjected to the reaction at 60° to 62° C in 12 mℓ of dry N,N-dimethylformamide in a nitrogen stream with 60 mg of α,α'-azobisisobutyronitrile for 24 hours. Then, the same treatment as in Example 15 was conducted to give 2.34 g of light yellow powder (yield 78.0 %).

Physical properties of the obtained product were measured in the same manner as in Example 1 and Example 15. The results are shown below.

(Weight average molecular weight)

Weight average molecular weight ($\overline{Mw}$): 32,000

(Value of elementary analysis)

C: 64.39 %, H: 5.35 %, N: 12.00 %, S: 9.11 %

(Thermal decomposition temperature)

Decomposition temperature: over 300° C
(it did not decompose until the temperature reached 300° C.)

(Ultraviolet absorption spectrum)

The measurement was carried out as a DMF solution. The result of the measurement is shown in Fig. 5.

(Infrared absorption spectrum)

The result of the measurement is shown in Fig. 6.

Example 18

[Preparation of a copolymer of (p'-carboxybenzaldehyde 2-methyl-4-p-vinylbenzylthiosemicarbazone) and maleic acid]

In the same manner as in Example 16, in 15 mℓ of N,N-dimethylacetamide were dissolved 3.00 g of p'-carboxybenzaldehyde 2-methyl-4-p-vinylbenzylthiosemicarbazone obtained in Example 2 and 0.83 g of maleic anhydride, 76 mg of 2,2'-azobis(2,4-dimethylvaleronitrile) was added thereto, and the polymerization was carried out at 60° to 62° C in a nitrogen stream for 24 hours. Then, precipitation and recovery of the polymerization composition were repeated in the same manner as in Example 16 to give 3.41 g of light yellow powder (yield 88.7 %). Then, 3.00 g of the obtained polymerization composition was subjected to hydrolysis with alkali, dialysis and treatment with hydrochloric acid to give 2.73 g of light yellow powder (yield 91.0 %).

Physical properties of the obtained product were measured in the same manner as in Example 1 and Example 15. The results are shown below.

(Weight average molecular weight)

Weight average molecular weight ($\overline{Mw}$): 5,000

(Value of elementary analysis)

C: 59.09 %, H: 5.20 %, N: 8.69 %, S: 6.57 %

(Thermal decomposition temperature)

Decomposition temperature: 260° C

(Ultraviolet absorption spectrum)

The measurement was carried out as a DMF solution. The result of the measurement is shown in Fig. 7.

(Infrared absorption spectrum)

The result of the measurement is shown in Fig. 8.

Example 19

[Preparation of poly(p'-carboxyvinylenebenzaldehyde 4-methyl-2-p-vinylbenzylthiosemicarbazone)]

In the same manner as in Example 15, after 3.00 g of p'-formylcinnamic acid 4-methyl-2-p-vinylbenzylthiosemicarbazone obtained in Example 3 was reacted in 12 mℓ of dry N,N-dimethylacetamide at 60° to 62° C in a nitrogen stream with 60 mg of benzoyl peroxide for 50 hours, the same treatment as in Example 15 was conducted to give 2.58 g of light yellow powder (yield 86.0 %).

Physical properties of the obtained product were measured in the same manner as in Example 1 and Example 15. The results are shown below.

(Weight average molecular weight)

Weight average molecular weight ($\overline{Mw}$): 18,000

(Value of elementary analysis)

C: 66.00 %, H: 5.89 %, N: 11.41 %, S: 8.39 %

(Thermal decomposition temperature)

Decomposition temperature: 225° C

(Ultraviolet absorption spectrum)

The measurement was carried out as a DMF solution. The result of the measurement is shown in Fig. 9.

(Infrared absorption spectrum)

The result of the measurement is shown in Fig. 10.

Example 20

[Preparation of a copolymer of (p'-carboxyvinylenebenzaldehyde 4-methyl-2-p-vinylbenzylthiosemicarbazone) and maleic acid]

In 19 mℓ of dry N,N-dimethylformamide were dissolved 2.00 g of 4-methyl-2-p-vinylbenzylthiosemicarbazide obtained in Example 1 and 3.64 g of maleic acid. After the solution was heated to 60 °C in a nitrogen stream, 1 mℓ of a dry N,N-dimethylformamide solution of 110 mg of α,α'-azobisisobutyronitrile was added thereto and the reaction was carried out at 60° to 62 °C for 24 hours. After the reaction, the treatment was conducted in the same manner as in Example 15 to give 3.04 g of light yellow powder (yield 53.9 %). After 1.00 g of this powder and 2.19 g of p-formylcinnamic acid were dissolved in 200 mℓ of 0.1 N NaOH aqueous solution, they were reacted at 60 °C for 24 hours. After the reaction, the reaction solution was poured into a cellophane tube to conduct dialysis three times with 5 ℓ of deionized water. After pH of the dialyzed solution in the tube was adjusted to 2 by adding 1N hydrochloric acid, the produced precipitate was collected by filtration and dried under vacuum to give 1.21 g of light yellow powder (yield 70.0 %).

Physical properties of the obtained product were measured in the same manner as in Example 1 and Example 15. The results are shown below.

(Weight average molecular weight)

Weight average molecular weight ($\overline{Mw}$): 50,000

(Value of elementary analysis)

C: 55.53 %, H: 4.52 %, N: 7.99 %, S: 5.78 %

(Thermal decomposition temperature)

Decomposition temperature: 265 °C

(Ultraviolet absorption spectrum)

The measurement was carried out as a DMF solution. The result of the measurement is shown in Fig. 11.

(Infrared absorption spectrum)

The result of the measurement is shown in Fig. 12.

Example 21

[Preparation of poly(p'-carboxybenzaldehyde 2-p-vinylbenzylsemicarbazone)]

In 17.5 mℓ of dry N,N-dimethylformamide, 3.50 g p'-carboxybenzaldehyde 2-p-vinylbenzylsemicarbazone obtained in Example 4 was reacted at 60° to 62 °C in a nitrogen stream with 70 mg of α,α'-azobisisobutyronitrile for 24 hours in the same manner as in Example 15. Then, the treatment was conducted in the same manner as in Example 15 to give 3.40 g of colorless powder (yield 97.1 %).

Physical properties of the obtained product were measured in the same manner as in Example 1 and Example 15. The results are shown below.

(Weight average molecular weight)

Weight average molecular weight ($\overline{Mw}$): 100,000

(Value of elementary analysis)

C: 67.03 %, H: 5.16 %, N: 12.77 %

(Thermal decomposition temperature)

Decomposition temperature: over 300 °C
(it did not decompose until the temperature reached 300 °C.)

(Ultraviolet absorption spectrum)

The measurement was carried out as a DMF solution. The result of the measurement is shown in Fig. 13.

(Infrared absorption spectrum)

The result of the measurement is shown in Fig. 14.

Example 22

[Preparation of poly(p'-carboxylbenzaldehyde 4-methyl-2-p-vinylbenzylsemicarbazone)]

In the same manner as in Example 15, 5.00 g of p'-carboxybenzaldehyde 4-methyl-2-p-vinylbenzylsemicarbazone obtained in Example 5 was reacted at 40° to 42 °C in 20 mℓ of dry N,N-dimethylformamide in a nitrogen stream with 150 mg of α,α'-azobisisobutyronitrile for 50 hours. Then, the same treatment as in Example 15 was conducted to give 3.20 g of colorless powder (yield 64.0 %).

Physical properties of the obtained product were measured in the same manner as in Example 1 and Example 15. The results are shown below.

(Weight average molecular weight)

Weight average molecular weight ($\overline{Mw}$): 130,000

(Value of elementary analysis)

C: 67.78 %, H: 5.59 %, N: 12.30 %

(Thermal decomposition temperature)

Decomposition temperature: 260 °C

(Ultraviolet absorption spectrum)

The measurement was carried out as a DMF solution. The result of the measurement is shown in Fig. 15.

(Infrared absorption spectrum)

The result of the measurement is shown in Fig. 16.

Example 23

[Preparation of a copolymer of (benzaldehyde 4-methyl-2-p-vinylbenzylthiosemicarbazone) and maleic acid]

In the same manner as in Example 16, in 25 mℓ of tetrahydrofuran were dissolved 5.00 g of benzaldehyde 4-methyl-2-p-vinylbenzylthiosemicarbazone and 1.60 g of maleic anhydride, and were polymerized in a nitrogen stream with 132 mg of benzoyl peroxide at 60° to 62°C for 50 hours. Then, precipitation and recovery of the polymerization composition were repeated to give 4.92 g of light yellow powder (yield 74.5 %). Then, 4.50 g of the obtained polymerization composition was subjected to hydrolysis with alkali, dialysis and treatment with hydrochloric acid to give 3.93 g of light yellow powder (yield 87.3 %).

Physical properties of the obtained product were measured in the same manner as in Example 1 and Example 15. The results are shown below.

(Weight average molecular weight)

Weight average molecular weight ($\overline{Mw}$): 4,000

(Value of elementary analysis)

C: 62.33 %, H: 5.63 %, N: 9.31 %, S: 7.40 %

(Thermal decomposition temperature)

Decomposition temperature: 250°C

(Ultraviolet absorption spectrum)

The measurement was carried out as a DMF solution. The result of the measurement is shown in Fig. 17.

(Infrared absorption spectrum)

The result of the measurement is shown in Fig. 18.

Example 24

[Preparation of a copolymer of (p'-methylbenzaldehyde 4-methyl-2-p-vinylbenzylthiosemicarbazone) and acrylic acid]

In the same manner as in Example 16, in 20 mℓ of N,N-dimethylacetamide were dissolved 4.00 g of p'-methylbenzaldehyde 4-methyl-2-p-vinylbenzylthiosemicarbazone and 1.22 g of methyl acrylate, and were polymerized in a nitrogen stream with 104 mg of 2,2'-azobis(2,4-dimethylvaleronitrile) at 60° to 62°C for 30 hours. Then, precipitation and recovery of the polymerization composition were repeated to give 3.79 g of light yellow powder (yield 72.6 %). Then, 3.50 g of the obtained polymerization composition was subjected to hydrolysis with alkali, dialysis and treatment with hydrochloric acid to give 3.11 g of light yellow powder (yield 88.8 %).

Physical properties of the obtained product were measured in the same manner as in Example 1 and Example 15. The results are shown below.

(Weight average molecular weight)

Weight average molecular weight ($\overline{Mw}$): 6,000

(Value of elementary analysis)

C: 67.17 %, H: 6.59 %, N: 10.43 %, S: 7.85 %

(Thermal decomposition temperature)

Decomposition temperature: 210°C

(Ultraviolet absorption spectrum)

The measurement was carried out as a DMF solution. The result of the measurement is shown in Fig. 19.

(Infrared absorption spectrum)

The result of the measurement is shown in Fig. 20.

Example 25

[Preparation of a copolymer of (p'-dimethylaminobenzaldehyde 4-methyl-2-p-vinylbenzylthiosemicarbazone) and maleic acid]

In the same manner as in Example 16, in 18 mℓ of dry N,N-dimethylformamide were dissolved 3.50 g of p'-dimethylaminobenzaldehyde 4-methyl-2-p-vinylbenzylthiosemicarbazone obtained in Example 8 and 1.02 g of maleic anhydride, and were polymerized in a nitrogen stream with 92 mg of α,α'-azobisisobutyronitrile at 80° to 82°C for 15 hours. Then, precipitation and recovery of the polymerization composition were repeated to give 3.71 g of light yellow powder (yield 82.1 %). Then, 3.50 g of the obtained polymerization composition was subjected to hydrolysis with alkali, dialysis and treatment with hydrochloric acid to give 3.01 g of light yellow powder (yield 86.0 %).

Physical properties of the obtained product were measured in the same manner as in Example 1 and Example 15. The results are shown below.

(Weight average molecular weight)

Weight average molecular weight ($\overline{Mw}$): 3,000

(Value of elementary analysis)

C: 61.72 %, H: 6.24 %, N: 11.71 %, S: 6.59 %

(Thermal decomposition temperature)

Decomposition temperature: 170 ° C

(Ultraviolet absorption spectrum)

The measurement was carried out as a DMF solution. The result of the measurement is shown in Fig. 21.

(Infrared absorption spectrum)

The result of the measurement is shown in Fig. 22.

Example 26

[Preparation of a copolymer of (p'-chlorobenzaldehyde 4-methyl-2-p-vinylbenzylthiosemicarbazone) and maleic acid]

In the same manner as in Example 15, in 18 mℓ of dry N,N-dimethylformamide were dissolved 3.50 g of p'-chlorobenzaldehyde 4-methyl-2-p-vinylbenzylthiosemicarbazone obtained in Example 9 and 1.00 g of maleic acid, and were polymerized in a nitrogen stream with 90 mg of $\alpha,\alpha'$-azobisisobutyronitrle at 60 ° to 62 ° C for 24 hours. Then, precipitation and recovery of the polymerization composition were repeated to give 3.47 g of light yellow powder (yield 77.1 %).

Physical properties of the obtained product were measured in the same manner as in Example 1 and Example 15. The results are shown below.

(Weight average molecular weight)

Weight average molecular weight ($\overline{Mw}$): 6,800

(Value of elementary analysis)

C: 57.68 %, H: 4.99 %, N: 8.93 %, S: 6.74 %

(Thermal decomposition temperature)

Decomposition temperature: 225 ° C

(Ultraviolet absorption spectrum)

The measurement was carried out as a DMF solution. The result of the measurement is shown in Fig. 23.

(Infrared absorption spectrum)

The result of the measurement is shown in Fig. 24.

Example 27

[Preparation of a copolymer of (p'-nitrobenzaldehyde 4-methyl-2-p-vinylbenzylthiosemicarbazone) and maleic acid]

In the same manner as in Example 16, in 18 mℓ of dry N,N-dimethylformamide were dissolved 3.50 g of p'-nitrobenzaldehyde 4-methyl-2-p-vinylbenzylthiosemicarbazone obtained in Example 10 and 0.96 g of maleic anhydride, and were polymerized in a nitrogen stream with 89 mg of 2,2'-azobis(2,4-dimethylvaleronitrile) at 60° to 62°C for 30 hours. Then, precipitation and recovery of the polymerization composition were repeated to give 3.67 g of light yellow powder (yield 82.3 %). Then, 3.50 g of the obtained polymerization composition was subjected to hydrolysis with alkali, dialysis and treatment with hydrochloric acid to give 2.81 g of light yellow powder (yield 80.2 %).

Physical properties of the obtained product were measured in the same manner as in Example 1 and Example 15. The results are shown below.

(Weight average molecular weight)

Weight average molecular weight ($\overline{Mw}$): 3,500

(Value of elementary analysis)

C: 56.40 %, H: 4.91 %, N: 11.77 %, S: 6.61 %

(Thermal decomposition temperature)

Decomposition temperature: 230°C

(Ultraviolet absorption spectrum)

The measurement was carried out as a DMF solution. The result of the measurement is shown in Fig. 25.

(Infrared absorption spectrum)

The result of the measurement is shown in Fig. 26.

Example 28

[Preparation of a copolymer of (2'-furylaldehyde 4-methyl-2-p-vinylbenzylthiosemicarbazone) and maleic acid]

In the same manner as in Example 16, in 19 mℓ of N-methylpyrrolidone were dissolved 3.50 g of 2'-furylaldehyde 4-methyl-2-p-vinylbenzylthiosemicarbazone obtained in Example 11 and 1.15 g of maleic anhydride, and were polymerized in a nitrogen stream with 93 mg of ammonium persulfate at 60° to 62°C for 60 hours. Then, precipitation and recovery of the polymerization composition were repeated to give 3.73 g of light yellow powder (yield 80.2 %). Then, 3.50 g of the obtained polymerization composition was subjected to hydrolysis with alkali, dialysis and treatment with hydrochloric acid to give 3.11 g of light

yellow powder (yield 88.8 %).

Physical properties of the obtained product were measured in the same manner as in Example 1 and Example 15. The results are shown below.

(Weight average molecular weight)

Weight average molecular weight ($\overline{Mw}$ ): 1,200

(Value of elementary analysis)

C: 58.09 %, H: 5.30 %, N: 9.91 %, S: 7.39 %

(Thermal decomposition temperature)

Decomposition temperature: 215 °C

(Ultraviolet absorption spectrum)

The measurement was carried out as a DMF solution. The result of the measurement is shown in Fig. 27.

(Infrared absorption spectrum)

The result of the measurement is shown in Fig. 28.

Example 29

[Preparation of a copolymer of (2'-thienylaldehyde 4-methyl-2-p-vinylbenzylthiosemicarbazone) and maleic acid]

In the same manner as in Example 16, in 18 mℓ of 2-butanone were dissolved 3.50 g of 2'-thienylaldehyde 4-methyl-2-p-vinylbenzylthiosemicarbazone obtained in Example 12 and 1.09 g of maleic anhydride, and were polymerized at 60° to 62°C in a nitrogen stream with 92 mg of α,α'-azobis-isobutyronitrile for 30 hours. Then, precipitation and recovery of the polymerization composition were repeated to give 3.31 g of light yellow powder (yield 72.1 %). Then, 3.00 g of the obtained polymerization composition was subjected to hydrolysis with alkali, dialysis and treatment with hydrochloric acid to give 2.67 g of light yellow powder (yield 89.0 %).

Physical properties of the obtained product were measured in the same manner as in Example 1 and Example 15. The results are shown below.

(Weight average molecular weight)

Weight average molecular weight ($\overline{Mw}$ ): 3,000

(Value of elementary analysis)

C: 55.96 %, H: 5.11 %, N: 9.54 %, S: 14.70 %

(Thermal decomposition temperature)

Decomposition temperature: 200 °C

(Ultraviolet absorption spectrum)

The measurement was carried out as a DMF solution. The result of the measurement is shown in Fig. 29.

(Infrared absorption spectrum)

The result of the measurement is shown in Fig. 30.

Example 30

[Preparation of a copolymer of (isonicotinicaldehyde 4-methyl-2-p-vinylbenzylthiosemicarbazone) and acrylic acid]

In the same manner as in Example 16, in 18 mℓ of dry N,N-dimethylformamide were dissolved 3.50 g of isonicotinicaldehyde 4-methyl-2-p-vinylbenzylthiosemicarbazone obtained in Example 13 and 1.11 g of acrylic acid, and were polymerized in a nitrogen stream with 92 mg of $\alpha,\alpha'$-azobisisobutyronitrile at 60 ° to 62 °C for 30 hours. Then, precipitation and recovery of the polymerization composition were repeated to give 3.71 g of light yellow powder (yield 80.5 %).

Physical properties of the obtained product were measured in the same manner as in Example 1 and Example 15. The results are shown below.

(Weight average molecular weight)

Weight average molecular weight ($\overline{Mw}$): 2,000

(Value of elementary analysis)

C: 62.87 %, H: 5.89 %, N: 14.60 %, S: 8.06 %

(Thermal decomposition temperature)

Decomposition temperature: over 300 °C
(it did not decompose until the temperature reached 300 °C.)

(Ultraviolet absorption spectrum)

The measurement was carried out as a DMF solution. The result of the measurement is shown in Fig. 31.

(Infrared absorption spectrum)

The result of the measurement is shown in Fig. 32.

Example 31

[Preparation of a copolymer of (cinnamoylaldehyde 4-methyl-2-p-vinylbenzylthiosemicarbazone) and maleic acid]

In the same manner as in Example 16, in 23 mℓ of dry N,N-dimethylformamide were dissolved 4.50 g of cinnamoylaldehyde 4-methyl-2-p-vinylbenzyl thiosemicarbazone obtained in Example 14 and 1.32 g of maleic anhydride, and were polymerized in a nitrogen stream with 116 mg of α,α'-azobisisobutyronitrile at 60° to 62° C for 24 hours. Then, precipitation and recovery of the polymerization composition were repeated to give 4.37 g of light yellow powder (yield 75.1 %). Then, 4.00 g of the obtained polymerization composition was subjected to hydrolysis with alkali, dialysis and treatment with hydrochloric acid to give 3.52 g of light yellow powder (yield 88.0 %).

Physical properties of the obtained product were measured in the same manner as in Example 1 and Example 15. The results are shown below.

(Weight average molecular weight)

Weight average molecular weight ($\overline{Mw}$): 2,000

(Value of elementary analysis)

C: 64.10 %, H: 5.71 %, N: 9.90 %, S: 6.99 %

(Thermal decomposition temperature)

Decomposition temperature: 230° C

(Ultraviolet absorption spectrum)

The measurement was carried out as a DMF solution. The result of the measurement is shown in Fig. 33.

(Infrared absorption spectrum)

The result of the measurement is shown in Fig. 34.

Example 32

[Preparation of poly(p'-carboxybenzaldehyde 4-methyl-2-p-vinylbenzylthiosemicarbazone)]

In 10 mℓ of dry N,N-dimethylformamide was dissolved 2.21 g of 4-methyl-2-p-vinylbenzylthiosemicarbazide as an intermediate obtained in Example 1, and was heated to 60° C while passing through dry nitrogen gas. Then, 1 mℓ of an N,N-dimethylformamide solution of 45 mg of α,α'-azobisisobutylronitrile was added thereto, and the reaction was carried out at 60° to 62° C for 24 hours. After the reaction, the reaction solution was concentrated under reduced pressure, 70 mℓ of methanol was added thereto, and the solution was allowed to stand while stirring thoroughly. The produced precipitate was collected by filtration and dried.

The precipitate was dissolved again in 50 mℓ of N,N-dimethylformamide, and 3.0 g of terephthalaldehydic acid was added thereto. Then, the reaction was carried out at 70° C under reduced pressure for 2 hours. After the reaction, the reaction solution was concentrated under reduced pressure, and dissolved in 1

% aqueous triethanolamine solution. Then, the solution was poured into a cellophane tube, and was subjected to dialysis and purification. Then, pH of the dialysed solution in the tube was adjusted to 2.5 by adding diluted hydrochloric acid. The produced precipitate was collected by filtration and dried under vacuum to give 3.1 g of light yellow powder (yield 87.8 %).

Physical properties of the obtained product were measured in the same manner as in Example 1 and Example 15. The results are shown below.

(Weight average molecular weight)

Weight average molecular weight ($\overline{Mw}$): 30,000

(Value of elementary analysis)

C: 64.89 %, H: 5.41 %, N: 11.71 %, S: 8.80 %

(Thermal decomposition temperature)

Decomposition temperature: over 300 °C
(it did not decompose until the temperature reached 300 °C.)

(Ultraviolet absorption spectrum)

The result of the measurement is shown in Fig. 35.

(Infrared absorption spectrum)

The result of the measurement is shown in Fig. 36.

## Example 33

[Preparation of poly[(p'-carboxyvinylbenzaldehyde 4-methyl-2-p-vinylbenzylthiosemicarbazone)-(p'-carboxybenzaldehyde 4-methyl-2-p-vinylbenzylsemicarbazone)]]

In 25 mℓ of dry N,N-dimethylformamide were dissolved 2.21 g of 4-methyl-2-p-vinylbenzylthiosemicarbazide obtained in Example 1 as an intermediate and 3.37 g of p'-carboxybenzaldehyde 4-methyl-2-p-vinylbenzylsemicarbazone obtained in Example 5, and the solution was heated to 60 °C while passing through dry nitrogen gas. Then, 2 mℓ of an N,N-dimethylformamide solution of 100 mg of α,α'-azobisisobutyronitrile was added thereto, and the reaction was carried out at 60° to 62 °C for 24 hours. After the reaction, 75 mℓ of N,N-dimethylformamide was added to the reaction solution, and 3.50 g of p-formylcinnamic acid was added thereto. Then, the reaction was carried out at 70 °C under reduced pressure for 2 hours. After the reaction, the reaction solution was concentrated under reduced pressure, and the concentrated solution was dissolved in 1 % triethanolamine aqueous solution. Then, the solution was poured into a cellophane tube and subjected to dialysis and purification. Then, pH of the dialysed solution in the tube was adjusted to 2.5 by adding diluted hydrochloric acid, and a produced precipitate was collected by filtration and dried under vacuum. As the result, 5.23 g of light yellow powder was obtained (yield 73.1 %).

Physical properties of the obtained product were measured in the same manner as in Example 1 and Example 15. The results are shown below.

(Weight average molecular weight)

Weight average molecular weight ($\overline{Mw}$ ): 12,000

(Value of elementary analysis)

C: 67.12 %, H: 5.60 %, N: 11.82 %, S: 4.51 %

(Thermal decomposition temperature)

Decomposition temperature: 290 °C

(Ultraviolet absorption spectrum)

The result of the measurement is shown in Fig. 37.

(Infrared absorption spectrum)

The result of the measurement is shown in Fig. 38.

Example 34

[Preparation of poly[(p'-carboxyvinylenebenzaldehyde 4-methyl-2-p-vinylbenzylsemicarbazone)-(p'-carboxyvinylenebenzaldehyde 4-methyl-2-p-vinylbenzylthiosemicarbazone)]

In 20 mℓ of dry N,N-dimethylformamide were dissolved 2.05 g of 4-methyl-2-p-vinylbenzylsemicarbazide obtained in Example 5 as an intermediate and 2.21 g of 4-methyl-2-p-vinylbenzylthiosemicarbazide obtained in Example 1 as an intermediate, and the solution was dried at 60 °C while passing through dry nitrogen gas. Then, 2 mℓ of an N,N-dimethylformamide solution of 80 mg of α,α'-azobisisobutyronitrile was added thereto, and the reaction was carried out at 60 ° to 62 °C for 24 hours. After the reaction, 75 mℓ of N,N-dimethylformamidewas added to the reaction solution, and 5.30 g of p-formylcinnamic acid was added thereto. Then, the reaction was carried out at 70 °C under reduced pressure for 2 hours. After the reaction, the reaction solution was concentrated under reduced pressure, and the concentrated solution was dissolved in 1 % triethanolamine aqueous solution. Then, the solution was poured into a cellophane tube and subjected to dialysis and purification. Then, pH of the dialysed solution in the tube was adjusted to 2.5 by adding diluted hydrochloric acid, and a produced precipitate was collected by filtration and dried under vacuum. As the result, 6.10 g of light yellow powder was obtained (yield 82.3 %).

Physical properties of the obtained product were measured in the same manner as in Example 1 and Example 15. The results are shown below.

(Weight average molecular weight)

Weight average molecular weight ($\overline{Mw}$ ): 11,000

(Value of elementary analysis)

C: 67.93 %, H: 5.71 %, N: 11.11 %, S: 4.39 %

(Thermal decomposition temperature)

Decomposition temperature: 265 °C

(Ultraviolet absorption spectrum)

The result of the measurement is shown in Fig. 39.

(Infrared absorption spectrum)

The result of the measurement is shown in Fig. 40.

Example 35

[Preparation of poly[(p'-carboxybenzaldehyde 4-methyl-2-p-vinylbenzylsemicarbazone)-(p'-carboxybenzaldehyde 4-methyl-2-p-vinylbenzylthiosemicarbazone)]]

In the same manner as in Example 15, 3.00 g p'-carboxybenzaldehyde 4-methyl-2-p-vinylbenzylsemicarbazone obtained in Example 5 and 3.00 g of p'-carboxybenzaldehyde 4-methyl-2-p-vinylbenzylthiosemicarbazoneobtained in Example 1 were dried, and the reaction was carried out in 30 mℓ of N,N-dimethylformamide in a nitrogen stream with 120 mg of α,α'-azobisisobutyronitrile at 60° to 62°C for 24 hours. Then, the treatment was conducted in the same manner as in Example 15 to give 5.58 g of light yellow powder (yield 93.0 %).

Physical properties of the obtained product were measured in the same manner as in Example 1 and Example 15. The results are shown below.

(Weight average molecular weight)

Weight average molecular weight ($\overline{Mw}$): 31,000

(Value of elementary analysis)

C: 66.36 %, H: 5.52 %, N: 12.09 %, S: 4.32 %

(Thermal decomposition temperature)

Decomposition temperature: over 300°C
(it did not decompose until the temperature reached 300°C.)

(Ultraviolet absorption spectrum)

The result of the measurement is shown in Fig. 41.

(Infrared absorption spectrum)

The result of the measurement is shown in Fig. 42.

Example 36

On a water bath, 4.8 g of white vaseline and 4.3 g of stearyl alcohol were mixed with heating (75°C).

Next, 3.0 g of propylene glycol, 0.4 g of sodium lauryl sulfate, 1.5 g of the polystyrene derivative obtained in Example 15, 0.8 g of triethanolamine and 11.0 g of purified water were mixed, and the mixture was heated to 75°C to give a uniform solution. Then, the solution was added to the mixture of white

vaseline and stearyl alcohol, thoroughly mixed with stirring, and cooled to room temperature to give a sunscreen ointment.

The obtained sunscreen ointment had properties like those of a hydrophilic ointment given in Japanese pharmacopoeia. Then, the effect of sunscreen was examined according to the following method. As the result, when the sunscreen ointment obtained in the above was applied, no difference of the color of the irradiated area and the non-irradiated area was observed, while the skin in the control area turned red nearly crimson.

(Testing method)

Hair of the notal of three guinea pigs was cut, and the drug (ointment) was applied thereto in an amount of about 2 mg per 1 $cm^2$. Then, ultraviolet rays were irradiated for 10 minutes by six fluorescent lights (FL-20 SE, made by TOSHIBA CORPORATION) from a height of 20 cm from the notal. On the other hand, as a control area, a base prepared without mixing the sunscreening agent was applied, and treatments were carried out in the same manner as above. The judgement of the effect was obserbed with naked eyes after 24 hours passed.

Example 37

A sunscreen ointment was prepared in the same manner as in Example 36 except that the polystyrene derivative obtained in Example 16 was used instead of the polystyrene derivative obtained in Example 15.

The sunscreen effect of the obtained sunscreen ointment was examined in the same manner as in Example 36. As the result, when the sunscreen ointment obtained in the above was applied, no difference of the color between the irradiated area and the non-irradiated area was observed, while the skin in the control area turned red nearly crimson.

Example 38

A sunscreen lotion was prepared by mixing 1.5 g of the polystyrene derivative obtained in Example 17, 0.4 g of titanium oxide, 3.0 g of glycerol, 1.2 g of triethanolamine, 0.2 g of polyoxyethylene lanolin, 0.2 g of laurylphosphoric acid and 10.5 g of purified water, and further mixing by means of supersonic waves so that they were uniformly dispersed.

The obtained sunscreen lotion was sticky liquid in which white fine particles were suspended.

Next, the sunscreen effect was examined in accordance with the method shown in Example 36. As the result, when the sunscreen lotion obtained in the above was applied, no difference of the color between the irradiated area and the non-irradiated area was observed, while the skin in the control area turned red nearly crimson.

Example 39

A uniform oil layer was prepared by mixing 1.7 g of tri(caprylcapronic acid) glycerol, 1.7 g liquid paraffin, 0.8 g of stearyl alcohol, 0.6 g of oleyl alcohol and 0.3 g of sorbitan monoisostearate and heating the mixture to 75 °C.

Then, besides the above-mentioned oil layer, a sunscreen ointment was prepared by mixing 1.5 g of the polystyrene derivative obtained in Example 18, 0.4 g of titanium oxide, 0.8 g of glycerol, 0.8 g of D-sorbit, 1.2 g of triethanolamine, 0.2 g of polyoxyethylene lanolin, 0.2 g of lauryl phosphoric acid and 6.6 g of purified water so that the components were uniformly dispersed, heating the mixture to 75 °C, adding the above oil layer thereto with stirring and cooling them to room temperature while continuously stirring.

The obtained sunscreen ointment had properties like those of a hydrophilic ointment given in Japanese pharmacopoeia.

Next, the sunscreen effect of the obtained sunscreen ointment was examined in the same manner as in Example 36. As the result, when the sunscreen ointment obtained in the above was applied, no difference of the color between the irradiated area and the non-irradiated area was obserbed, while the skin in the control area turned red nearly crimson.

Examples 40 to 56

A sunscreen ointment was prepared in the same manner as in Example 39 except that the polystyrene derivative obtained in each of Examples 19 to 35 was used instead of the polystyrene derivative obtained in Example 18, respectively.

The sunscreen effect of the obtained sunscreen ointment was examined in the same manner as in Example 36. As the result, when the sunscreen ointment obtained in the above was applied, no difference of the color between the irradiate area and the non-irradiated area was observed, while the skin in the control area turned red nearly crimson.

Example 57

[4-carboxy-4'-vinyldibenzoylmethane]

In 20 mℓ of n-hexane was suspended 1.3 g of sodium hydride (made by WAKO PURE CHEMICAL INDUSTRY, 60 % by weight in oil). After the suspension was stirred for 10 minutes, the suspension was allowed to stand and the supernatant was removed. Then, 50 mℓ of benzene, 3.1 g of dimethyl terephthalate and 1.9 g of 4-vinylacetophenone were added to the suspension, 10 mℓ of dimethylacetamide was slowly added thereto with stirring, and the stirring was continued at room temperature for about 3 hours in that condition.

After stirring, 5 mℓ of water was slowly added dropwise to the above mixture, the mixture was poured into 150 mℓ of ethyl acetate, and the ethyl acetate solution was washed with 200 mℓ of water three times.

After the ethyl acetate layer was dried with anhydrous sodium sulfate, ethyl acetate was distilled away under reduced pressure. The residue was recrystallized with ethanol to give 1.7 g of light yellow crystalline (yield 41 %).

Physical properties of the obtained crystalline are shown below.

(Ultraviolet absorption spectrum)

Wavelength at the maximum absorption ($\lambda$ max): 361 nm
Molar extinction coefficient ($\epsilon$): 33,000

(Melting point)

Melting point (mp): 129.5° to 130.0° C

(Nuclear magnetic resonance spectrum)

NMR ($CDC\ell_3$) $\delta$ ppm:
3.93 (3H, S)
5.40 (1H, d, J = 10.8 Hz)
5.87 (1H, d, J = 18.0 Hz)
6.76 (1H, d • d, J = 10.8 Hz, 18.0 Hz)
6.85 (1H, S)
7.52 (2H, d, J = 8.0 Hz)
7.90 to 8.23 (6H, m)

(Infrared absorption spectrum)

IR $\nu$ max (KBr) $cm^{-1}$:
2990, 2980 , 2950, 2845, 1715, 1600, 1580, 1490, 1435, 1275, 1110

In 100 mℓ of tetrahydrofuran was dissolved 1.5 g of 4-methyoxycarbonyl-4'-vinyldibenzoylmethane obtained in the above, and 100 mℓ of 1N sodium hydroxide aqueous solution was added thereto. The

solution was violently stirred at room temparature for 3 hours.

Next, after tetrahydrofuran was distilled away under reduced pressure, the solution was acidified by slowly adding 120 mℓ of 1N hydrochloric acid while cooling with ice and extracted with 100 mℓ of ethyl acetate three times. Then, ethyl acetate layer was collected and dried with sodium sulfuric anhydride, and ethyl acetate was distilled away under reduced pressure to give 1.3 g of yellow crystalline (yield 91 %). Physical properties of the obtained crystalline were examined. The results are shown below.

(Ultraviolet absorption spectrum)

Wavelength at the maximum absorption (λ max): 361 nm
Molar extinction coefficient (ε): 34,000

(Melting point)

Melting point (mp): over 300 °C

(Nuclear magnetic resonance spectrum)

NMR (DMSO-$d^6$) δ ppm:
5.44 (1H, d, J = 11.0 Hz)
6.02 (1H, d, J = 17.0 Hz)
6.83 (1H, d • d, J = 11.0 Hz, 17.0 Hz)
7.36 (1H, S)
7.66 (2H, d, J = 8.0 Hz)
8.05 to 8.40 (6H, m)

(Infrared absorption spectrum)

IR ν max (KBr) $cm^{-1}$:
3080, 3000, 2660, 2520, 1685, 1600, 1420, 1390, 1225, 1115

Example 58

[Preparation of poly[(p-carboxybenzaldehyde 4-methyl-2-p'-vinylbenzylsemicarbazone)-(p-carboxy-p'-vinyldibenzoylmethane)]]

In 13 mℓ of dimethylformamide were dissolved 1.50 g of 4-carboxy-p'-vinyldibenzoylmethane obtained in Example 57 and 1.70 g of p-carboxybenzaldehyde 4-methyl-2-(p-vinylbenzyl)semicarbazone and 60 mg of α,α'-azobisisobutyronitrile, and the polymerization was carried out in a nitrogen stream at 60 °C for 41 hours. Then, the reaction solution was poured into 50 mℓ of methanol, and the produced precipitate was collected by filtration. The precipitate was dissolved in 5 mℓ of dimethylformamide, and precipitation was carried out again with 50 mℓ of methanol to give 0.91 g of a product of yellow powder (yield 28 %).

Physical properties of the obtained product were measured in the same manner as in Example 15. The results are shown below.

(Weight average molecular weight)

Weight average molecular wight ($\overline{Mw}$): 110,000

(Differential thermal analysis)

Changes from room temperature to 300 °C were measured in an atmosphere of nitrogen gas by means

of a thermal analysis station (TAS-100 type, made by Rigaku Denki Co., Ltd.). The results are shown below.

Differential thermal analysis (DTA): 224.6 °C (Endothermic) 266.7 °C (Exothermic)

(Ultraviolet absorption spectrum)

The result of measurement is shown in Fig. 43.

(Infrared absoprtion spectrum)

The result of measurement is shown in Fig. 44.

(Elementary analysis)

C: 70.25 %, N: 6.72 %, H: 5.33 %

Example 59

[Preparation of poly[(p-carboxybenzaldehyde 2-p'-vinylbenzylsemicarbazone)-(p-carboxy-p'-vinyldibenzoylmethane)]]

The same procedure as in Example 11 was carried out except that 1.50 g of p-carboxy-p-vinyldibenzoylmethane in Example 57 and 1.65 g of p-caraboxybenzaldehyde 4-methyl 2-(p-vinylbenzyl) semicarbazone and 63 mg of 2,2'-azobisisobutyronitrile were dissolved in 13 mℓ of dimethylformamide to give 2.49 g of a product of yellow powder (yield 79.0 %).

Physical properties of the obtaiend product were measured in the same manner as in Example 58. The results are shown below.

(Weight average molecular weight)

Weight average molecular weight ($\overline{Mw}$): 50,000

(Differential thermal analysis)

Differential thermal anylysis (DTA): 260.6 °C (Exothermic)

(Ultraviolet absorption spectrum)

The measurement was carried out as an N,N-dimethylformamide solution.

The result of measurement is shown in Fig. 45.

(Infrared absorption spectrum)

The result of measurement is shown in Fig. 46.

(Elementary analysis)

C: 69.81 %, N: 6.97 %, H: 5.20 %

Examples 60 and 61

A uniform oil layer was prepared by mixing 1.7 g of tri(caprylcapronic acid)glycerol, 1.7 g of liquid paraffin, 0.8 g stearyl alcohol, 0.6 g of oleyl alcohol and 0.3 g of sorbitan monoisostearate and heating the mixture to 75° C.

Then, besides the above-mentioned oil layer, a sunscreen ointment was prepared by mixing 1.5 g of the polystyrene derivative obtaiend in Example 58 or 59, 0.4 g of titanium oxide, 0.8 g of glycerol, 0.8 g of D-sorbit, 1.2 g of triethanolamine, 0.2 g of polyoxyethylene lanolin, 0.2 g of lanolinic acid and 6.6 g of purified water so that the components were uniformly dispersed, heating the mixture to 75° C, adding the above oil layer thereto with stirring and cooling to room temperature while stirring.

The obtained sunscreen ointment had properties like those of a hydrophilic ointment given in Japanese pharmacopoeia.

Next, the sunscreen effect of the obtained sunscreen ointment was examined in the same manner as in Example 36. As the result, when the sunscreen ointment obtained in the above was applied, no difference of the color between the applied area and the non-applied area was observed, while the skin in the control area turned red nearly crimson.

INDUSTRIAL APPLICABILITY

The styrene derivative of the present invention can be suitably used as a sunscreening agent itself since the styrene derivative effectively absorbs ultraviolet rays having a wavelength of 280 to 400 nm. Further, the styrene derivative can be easily homopolymerized or copolymerized, and the homopolymer and a copolymer of the styrene derivative and other monomer can also be suitably used as a sunscreening agent since they effectively absorb ultravilet rays having a wavelength of 280 to 400 nm.

In addition, the polystyrene derivative of the present invention is excellent in safety for human bodies since the polystyrene derivative is difficult to be absorbed into the skin. Accordingly, the polystyrene derivative can be preferably added to a cosmetic composition and the like as a sunscreening agent.

## Claims

**1.** A styrene derivative represented by the general formula (I):

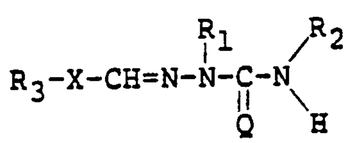

(I)

wherein one of $R_1$ and $R_2$ is vinylbenzyl group, another is hydrogen atom, a lower alkyl group, a carboxyalkyl group having 2 to 5 carbon atom or phenyl group, $R_3$ is hydrogen atom, a halogen atom, a lower alkyl group, carboxyl group, a carboxyalkyl group having 2 to 5 carbon atoms, a carboxyalkenyl group having 3 to 6 carbon atoms, an alkylamino group having 1 to 4 carbon atoms or nitro group, Q is sulfur atom or oxygen atom, X is

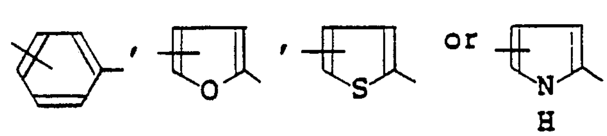

or .

**2.** A method for preparing the styrene derivative of Claim 1, characterized by carrying out a condensation

reaction of a (thio)semicarbazide represented by the general formula (II):

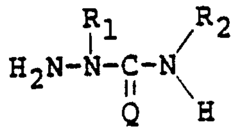

(II)

wherein one of $R_1$ and $R_2$ is vinylbenzyl group, another is hydrogen atom, a lower alkyl group, a carboxyalkyl group having 2 to 5 carbon atoms or phenyl group, Q is sulfur atom or oxygen atom, with a compound represented by the general formula (III):

$$R_3\text{-}X\text{-}CHO \qquad (III)$$

wherein $R_3$ is hydrogen atom, a halogen atom, a lower alkyl group, carboxyl group, a carboxyalkyl group having 2 to 5 carbon atoms, carboxyalkenyl group having 3 to 6 carbon atoms, an alkylamino group having 1 to 4 carbon atoms or nitro group, X is

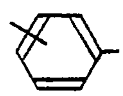 , 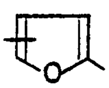 , 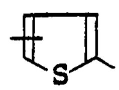 or 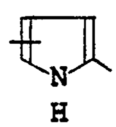 .

3. A polystyrene derivative derived from the styrene derivative of Claim 1 represented by the general formula (IV):

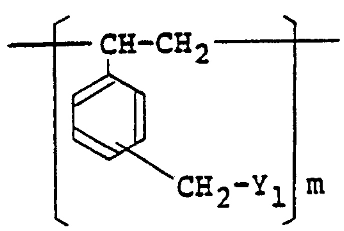

(IV)

wherein $Y_1$ is a group represented by the formula:

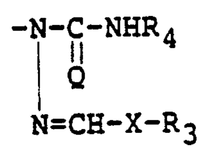

(wherein $R_3$ is hydrogen atom, a halogen atom, a lower alkyl group, carboxyl group, a carboxyalkyl group having 2 to 5 carbon atoms, a carboxylalkenyl group having 3 to 6 carbon atoms, an alkylamino group having 1 to 4 carbon atoms or nitro group, $R_4$ is hydrogen atom, a lower alkyl group, a carboxyalkyl group having 2 to 5 carbon atoms or phenyl group, Q is sulfur atom or oxygen atom, X is

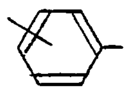 , 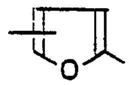 , 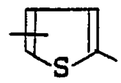 or 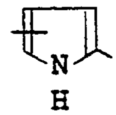 )

or a group represented by the formula:

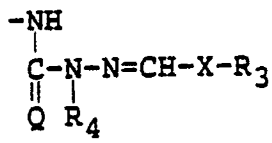

(wherein $R_3$, $R_4$, Q and X are the same as mentioned above), m is an integer of 6 to 1000.

4. The polystyrene derivative of Claim 3, which is represented by the general formula (V):

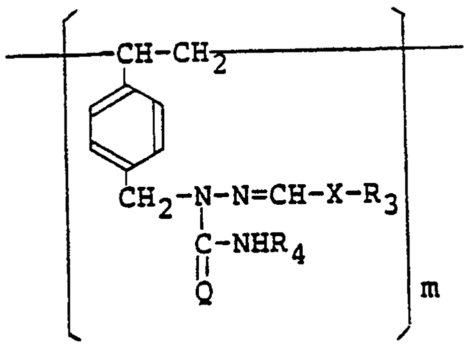

(V)

wherein $R_3$, $R_4$, Q, X and m are the same as mentioned above.

5. A polystyrene derivative prepared by copolymerizing the styrene derivative of Claim 1 and a compound having unsaturated aliphatic groups containing at least one carbon-carbon double bond which contributes to polymerization.

6. The polystyrene derivative of Claim 5, which is represented by the general formula (VI):

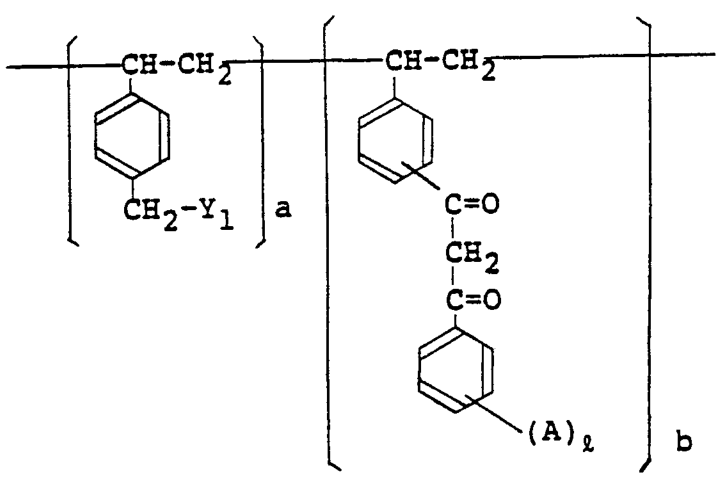

(VI)

wherein $Y_1$ is a group represented by the formula:

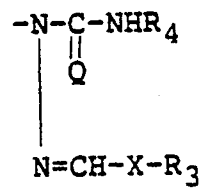

(wherein $R_3$ is hydrogen atom, a halogen atom, a lower alkyl group, carboxyl group, a carboxyalkyl group having 2 to 5 carbon atoms, a carboxyalkenyl group having 3 to 6 carbon atoms, an alkylamino group having 1 to 4 carbon atoms or nitro group, $R_4$ is hydrogen atom, a lower alkyl group, a carboxyalkyl group having 2 to 5 carbon atoms or phenyl group, Q is sulfur atom or oxygen atom, X is

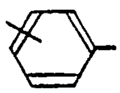 , 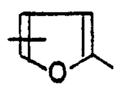 , 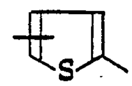 or 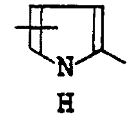 )

or a group represented by the formula:

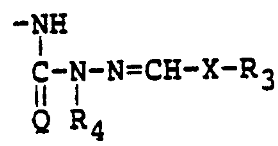

(wherein $R_3$, $R_4$, Q and X are the same as mentioned above), A is a lower alkyl group, carboxyl group, a lower alkoxy group or a lower alkoxycarbonyl group, $\ell$ is an integer of 0 to 3, a and b are positive integers satisfying m = a + b, m is an integer of 6 to 1000.

7. A method for preparing the above-mentioned polystyrene derivative, characterized by polymerizing in a solution at least one compound represented by the general formula (I):

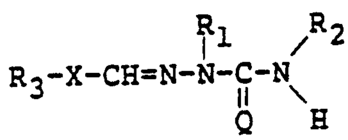 (I)

wherein one of $R_1$ and $R_2$ is vinylbenzyl group, another is hydrogen atom, a lower alkyl group having 2 to 5 carbon atoms, a carboxylalkyl group or phenyl group, $R_3$ is hydrogen atom, a halogen atom, a lower alkyl group, carboxyl group, a carboxyalkyl group having 2 to 5 carbon atoms, carboxyalkenyl group having 3 to 6 carbon atoms, an alkylamino group having 1 to 4 carbon atoms or nitro group, X is

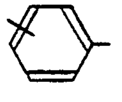 , 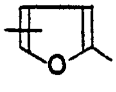 , 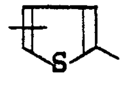 or 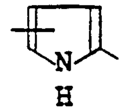 ,

Q is sulfur atom or oxygen atom, or polymerizing in a solution the above compound and a compound having unsaturated aliphatic groups containing at least one carbon-carbon double bond, which is terminated by a carboxyl group or a salt thereof, anhydride, ester, amide or imide.

8. A method for preparing the polystyrene derivative of Claim 3 or 5, characterized by polymerizing one or at least two kinds of a styrene derivative represented by the general formula (VII):

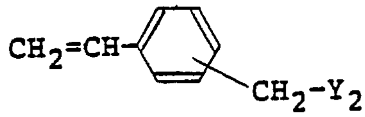

(VII)

wherein $Y_2$ is a group represented by the formula:

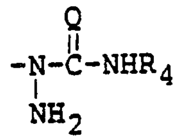

(wherein $R_4$ is hydrogen atom, a lower alkyl group, a carboxyalkyl group having 2 to 5 carbon atoms or phenyl group, Q is sulfur atom or oxygen atom) or a group represented by the formula:

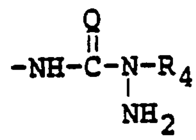

(wherein $R_4$ and Q are the same as mentioned above), or copolymerizing the styrene derivative represented by the general formula (VII) and a styrene derivative represented by the general formula (VIII):

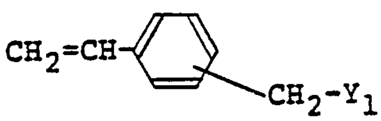

(VIII)

wherein $Y_1$ is a group represented by the formula

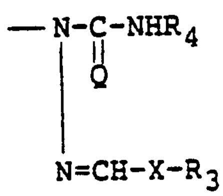

(wherein $R_3$ is hydrogen atom, a halogen atom, a lower alkyl group, carboxyl group, a carboxyalkyl group having 2 to 5 carbon atoms, a carboxyakenyl group having 3 to 6 carbon atoms, an alkylamino group having 1 to 4 carbon atoms or nitro group, $R_4$ and Q are the same as mentioned above, X is

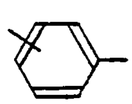 , 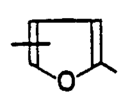 , 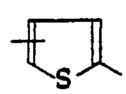 , or 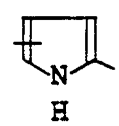 )

or a group represented by the formula:

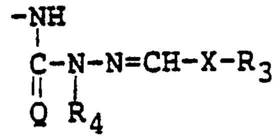

(wherein $R_3$, $R_4$, Q and X are the same as mentioned above), and carrying out a condensation reaction of the obtained copolymer with a compound represented by the general formula (III):

$$R_3-X-CHO \qquad (III)$$

wherein $R_3$ and X are the same as mentioned above.

**9.** A sunscreening agent of which an active ingredient is the polystyrene derivative of Claim 3 or Claim 5.

**10.** A cosmetic composition containing the polystyrene derivative of Claim 3 or Claim 5.

FIG. 1

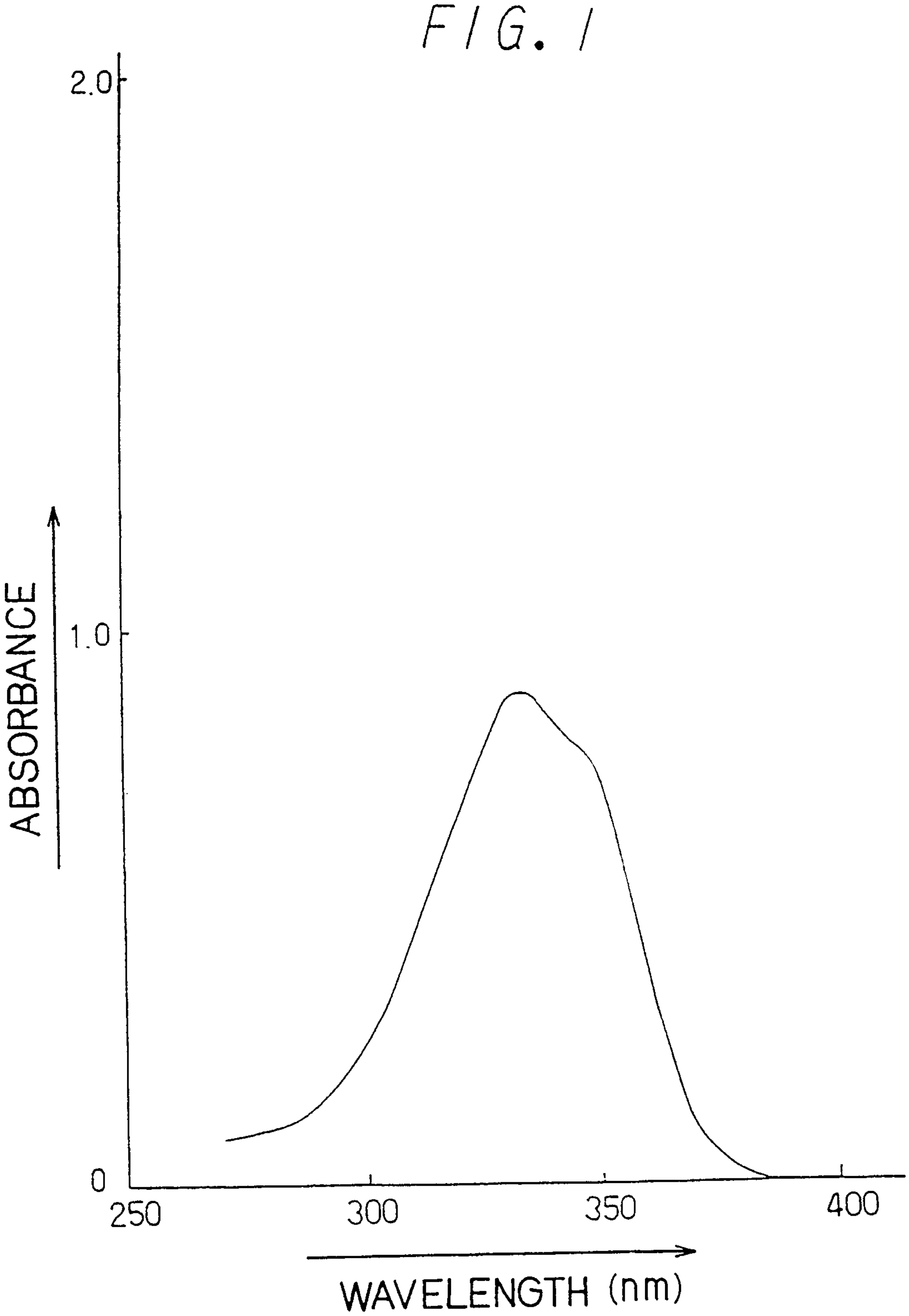

FIG. 2

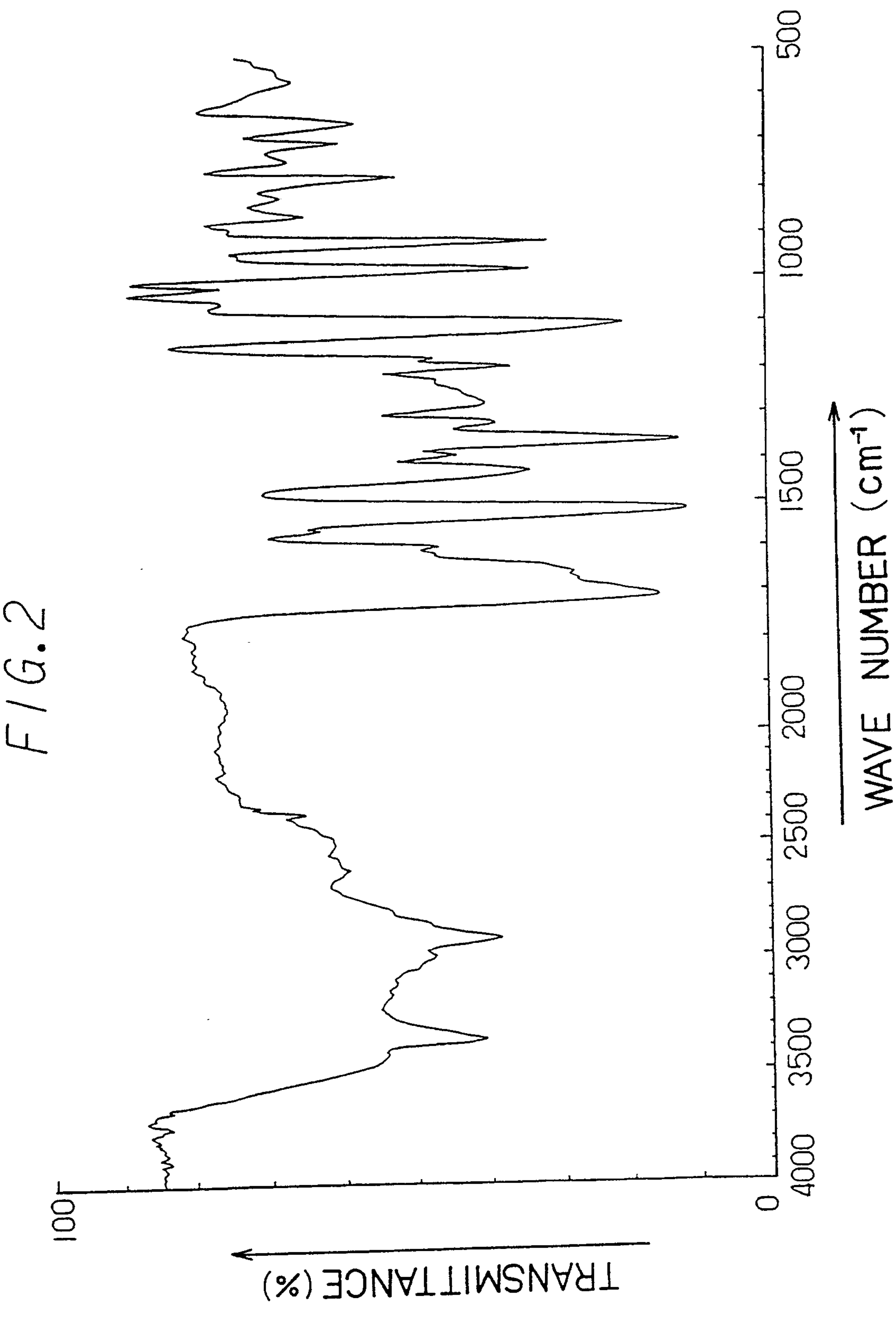
100
0
TRANSMITTANCE (%)
4000
3500
3000
2500
2000
1500
1000
500
WAVE NUMBER ($cm^{-1}$)

FIG.3

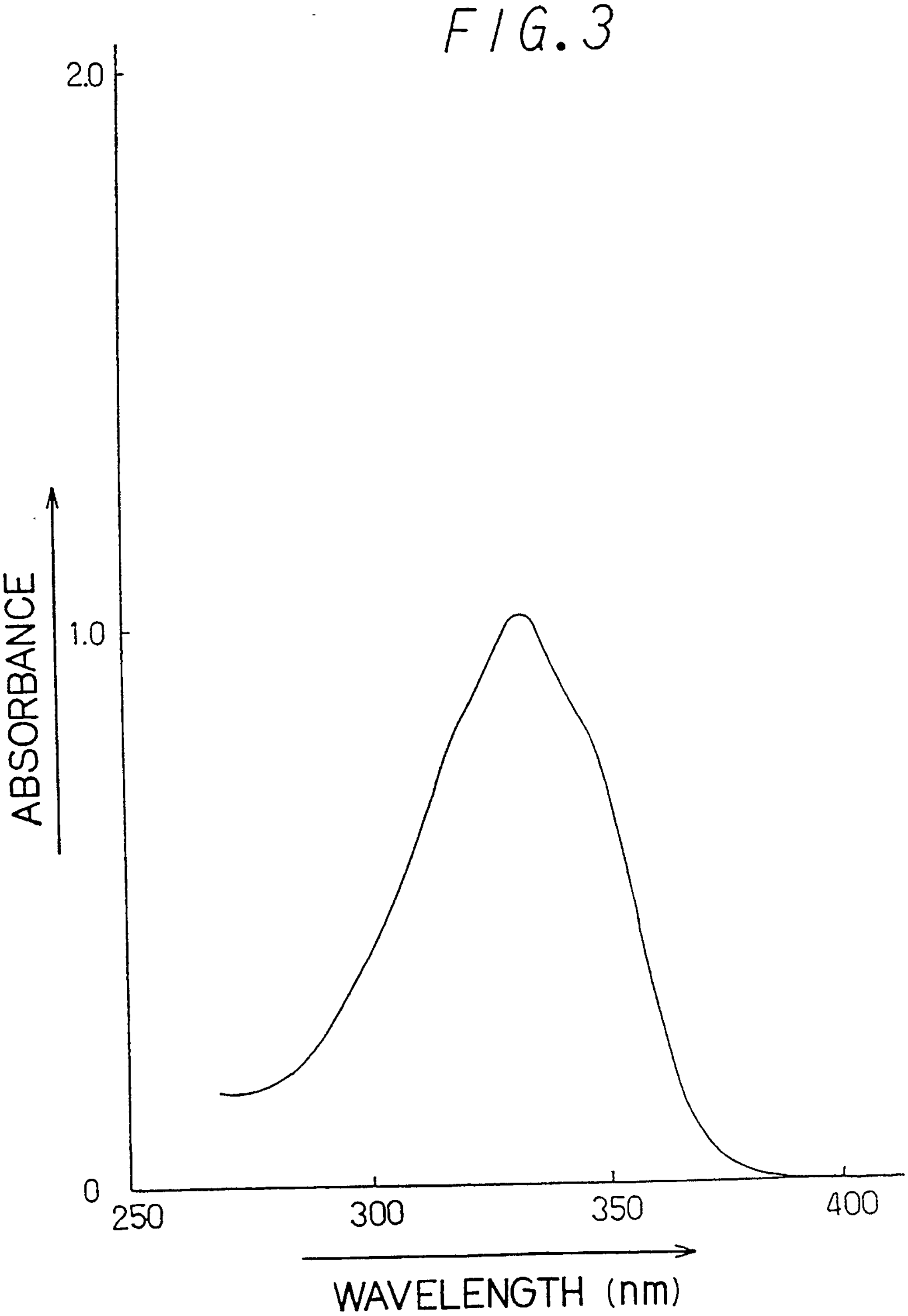

FIG.4

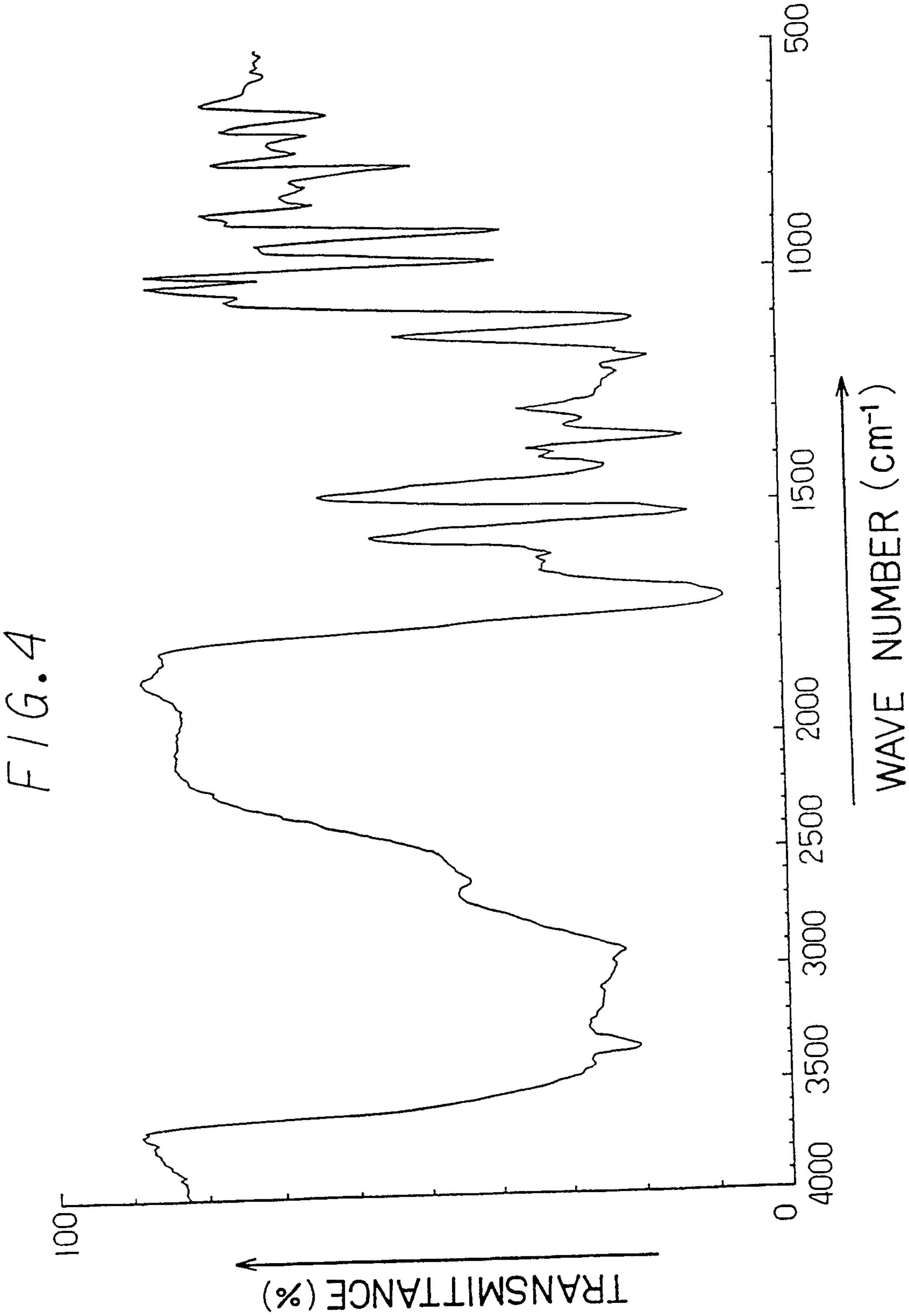
100
0
TRANSMITTANCE (%)
4000
3500
3000
2500
2000
1500
1000
500
WAVE NUMBER ($cm^{-1}$)

FIG.5

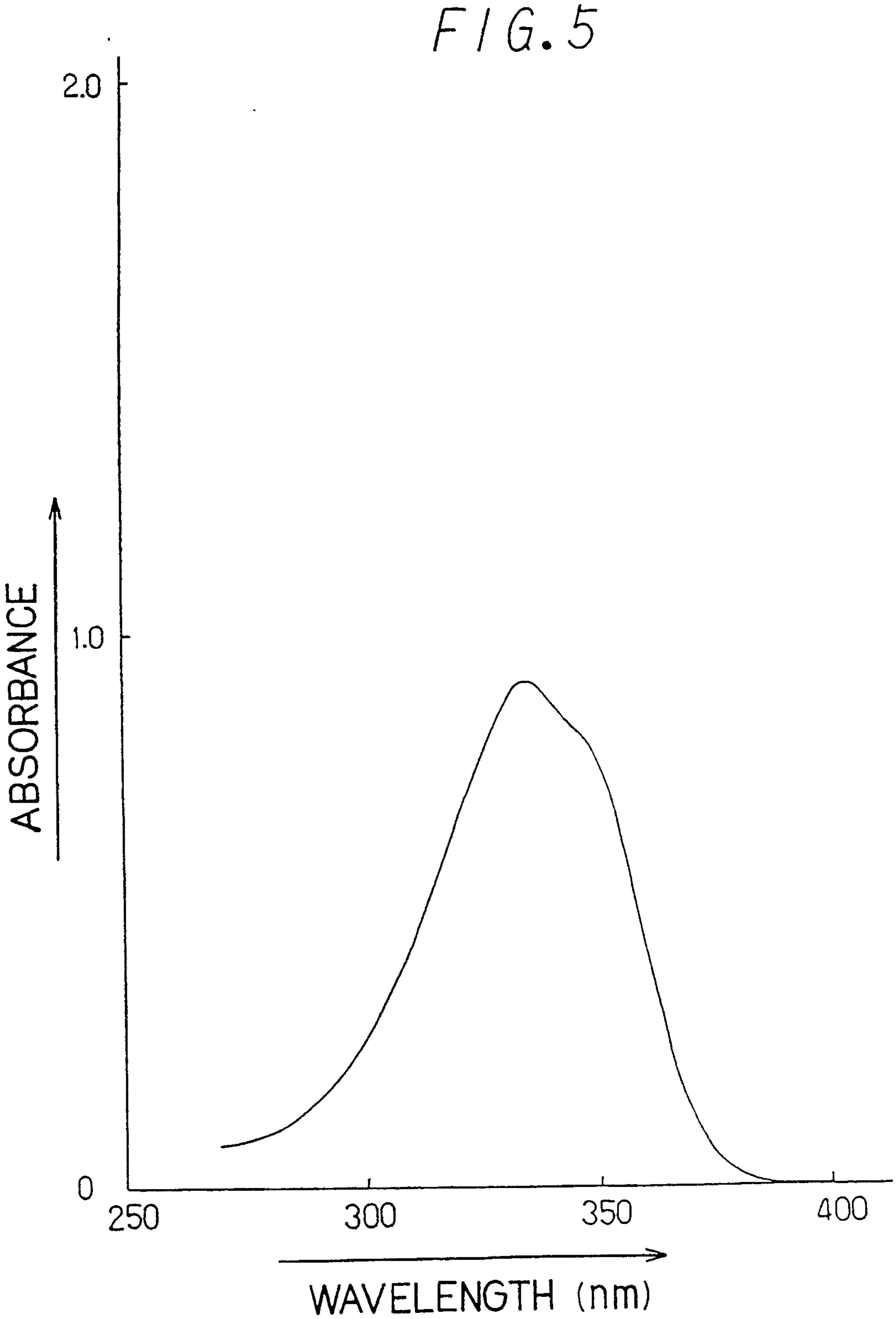

FIG. 6

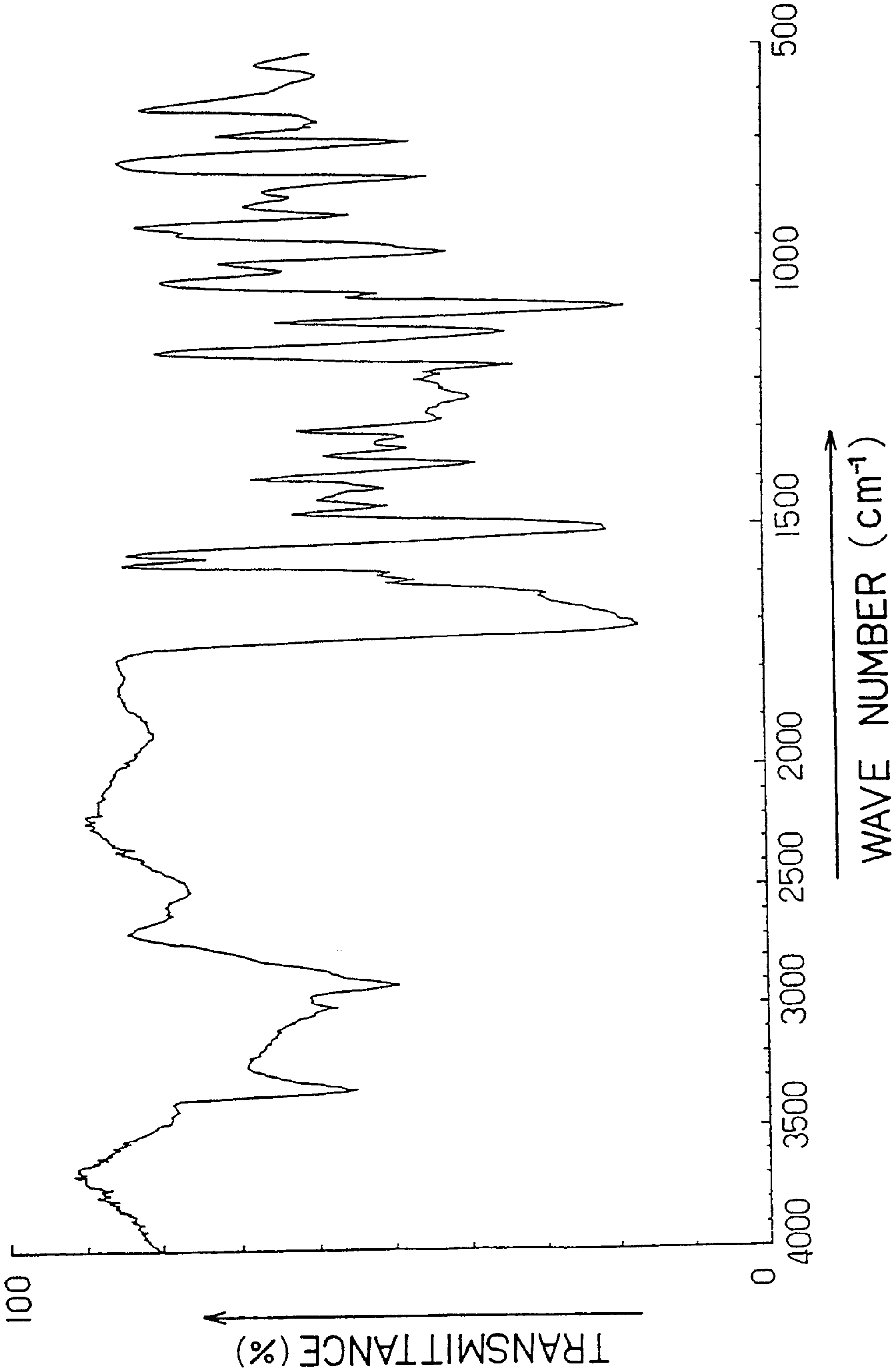

100
0
TRANSMITTANCE (%)
4000
3500
3000
2500
2000
1500
1000
500
WAVE NUMBER (cm-1)

FIG. 7

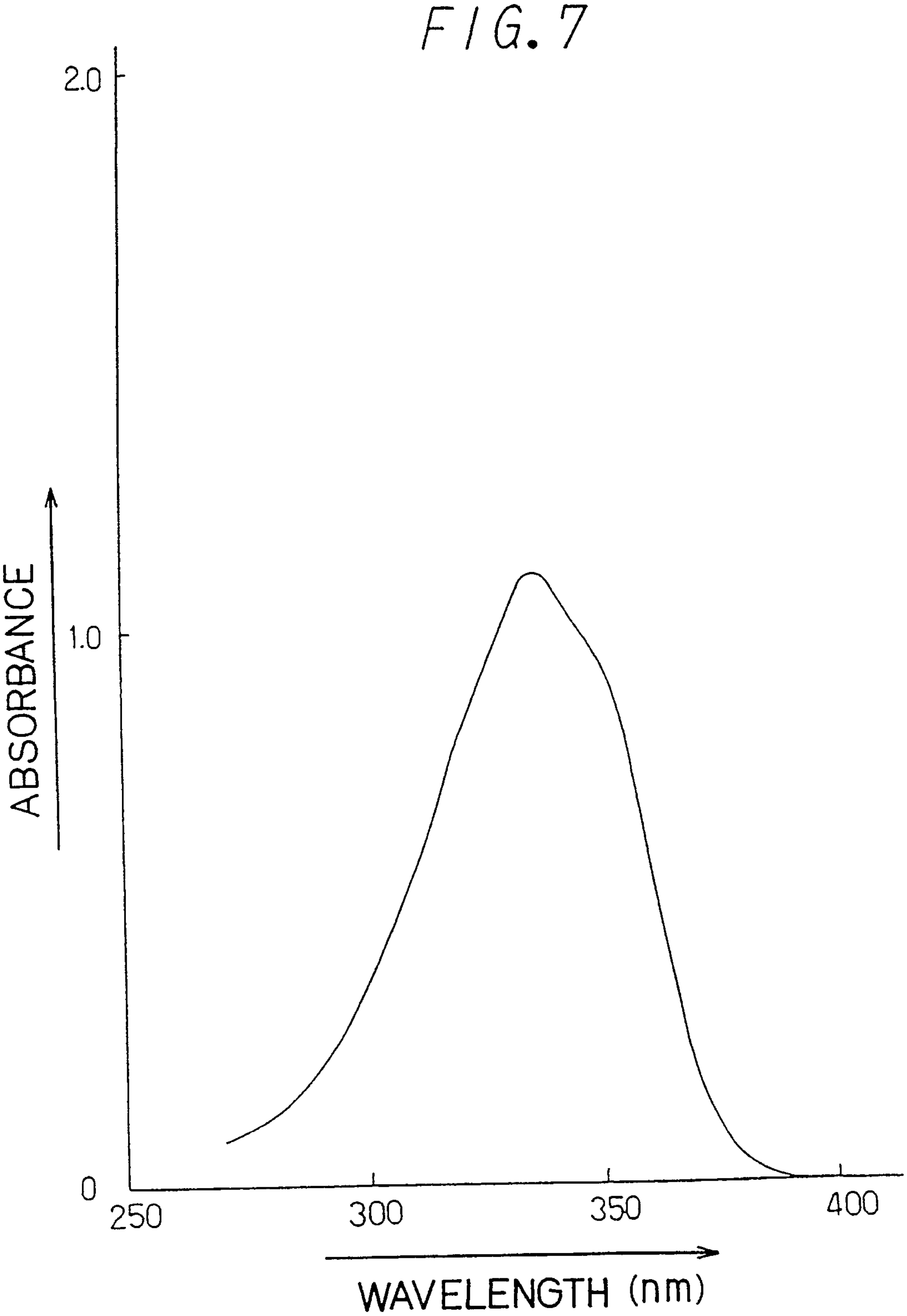

FIG. 8

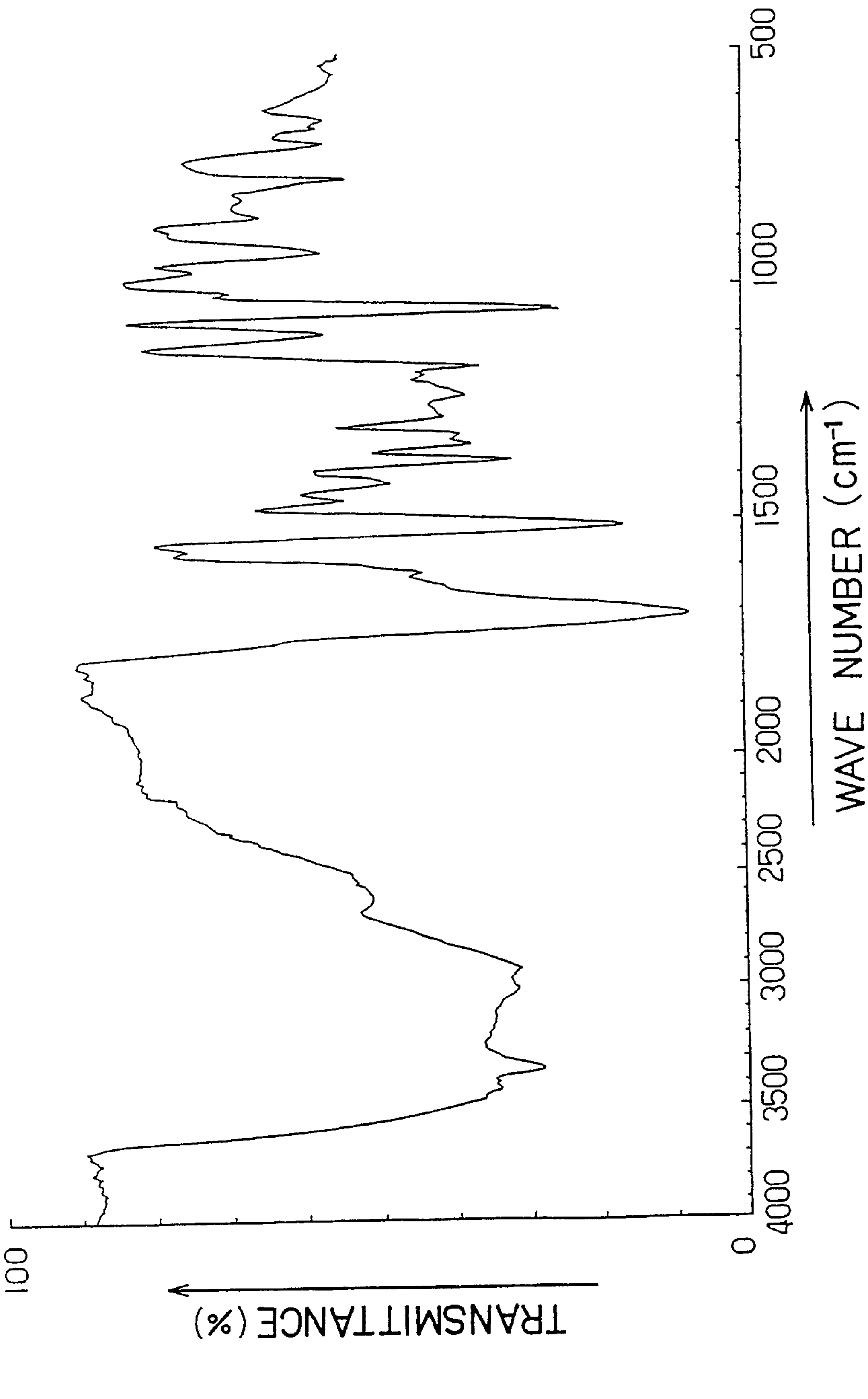
100
0
TRANSMITTANCE (%)
4000
3500
3000
2500
2000
1500
1000
500
WAVE NUMBER (cm⁻¹)

FIG. 9

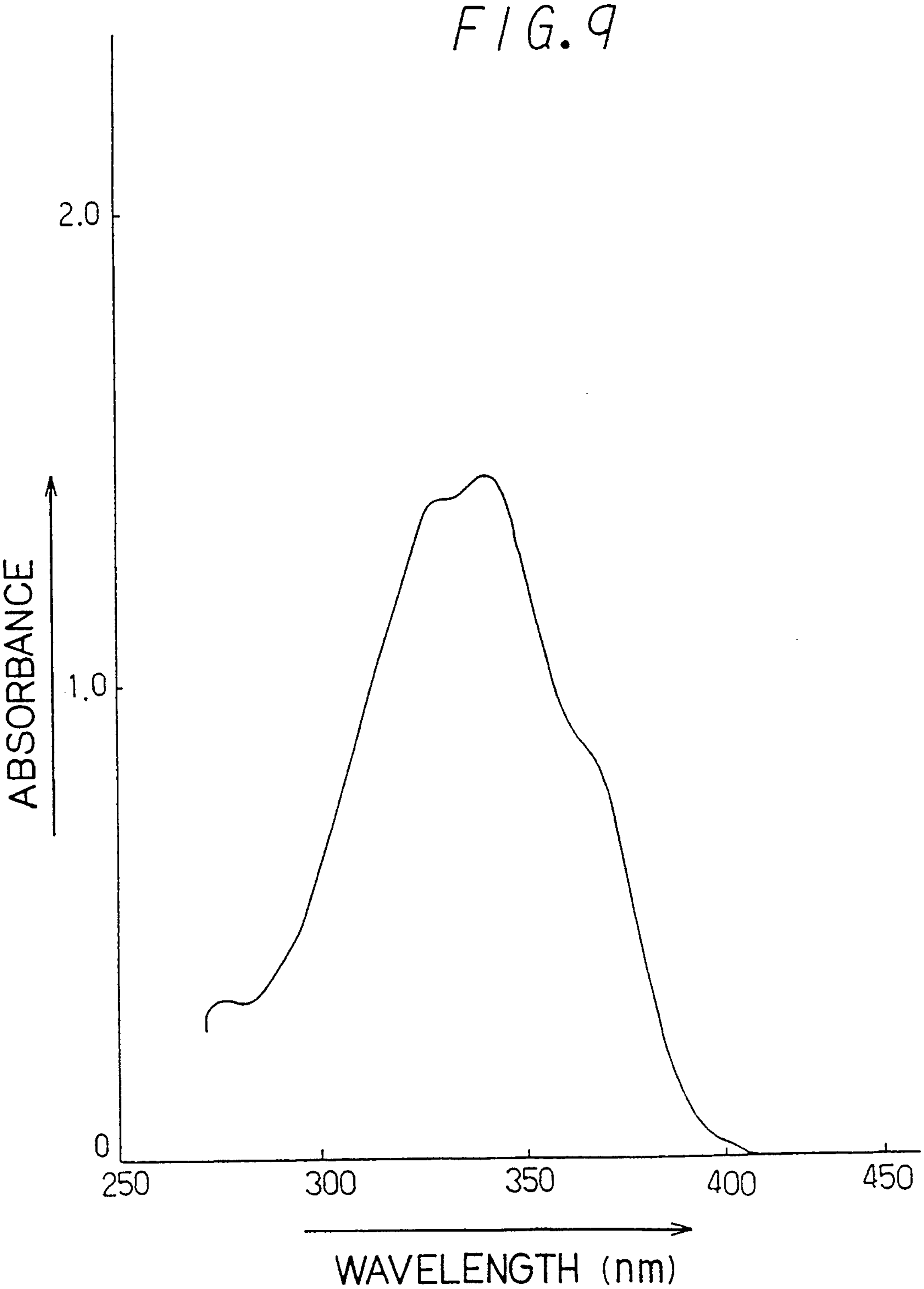

FIG. 10

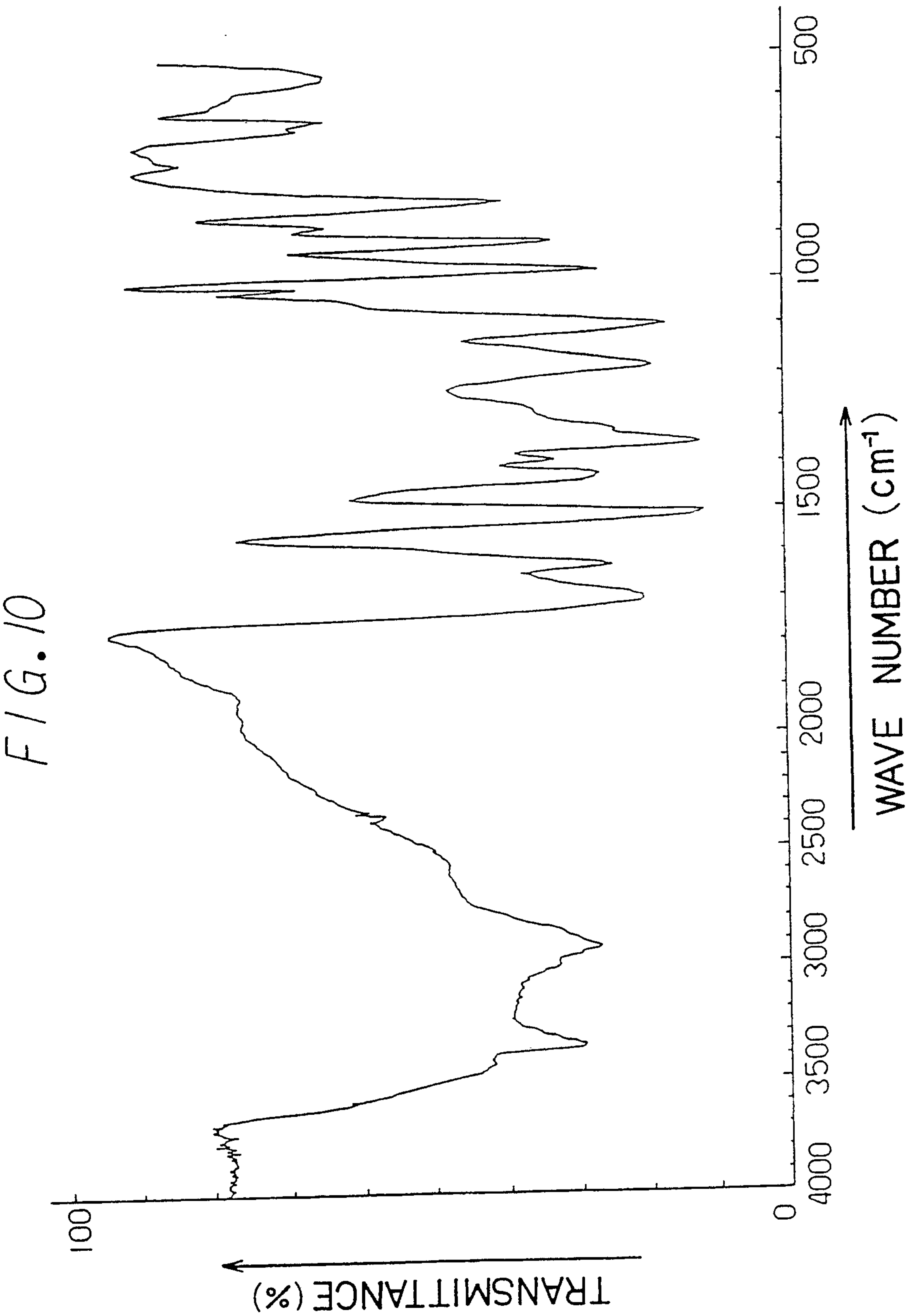
500
1000
1500
2000
2500
3000
3500
4000
0
100
WAVE NUMBER (cm-1)
TRANSMITTANCE (%)

FIG. 11

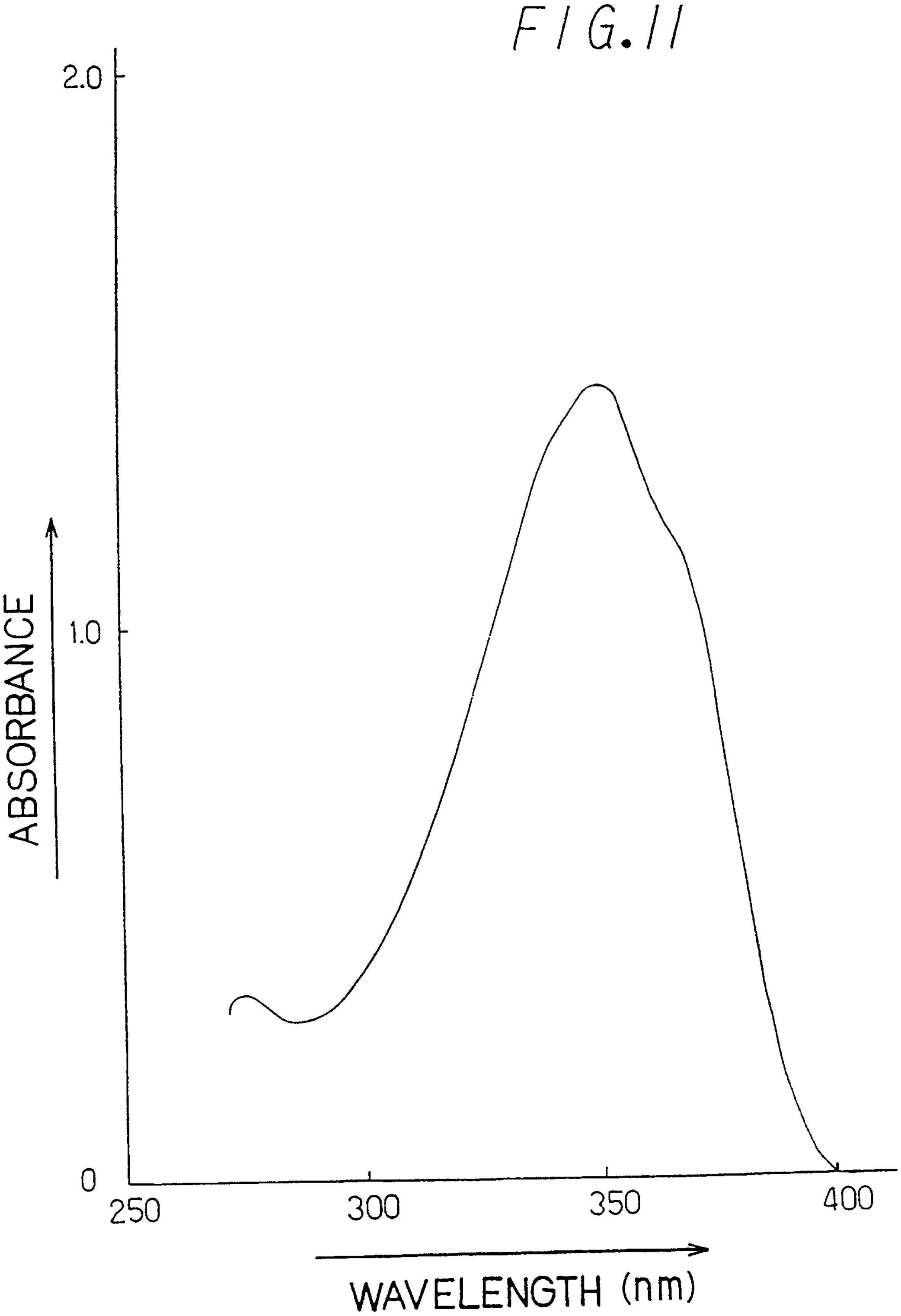

FIG. 12

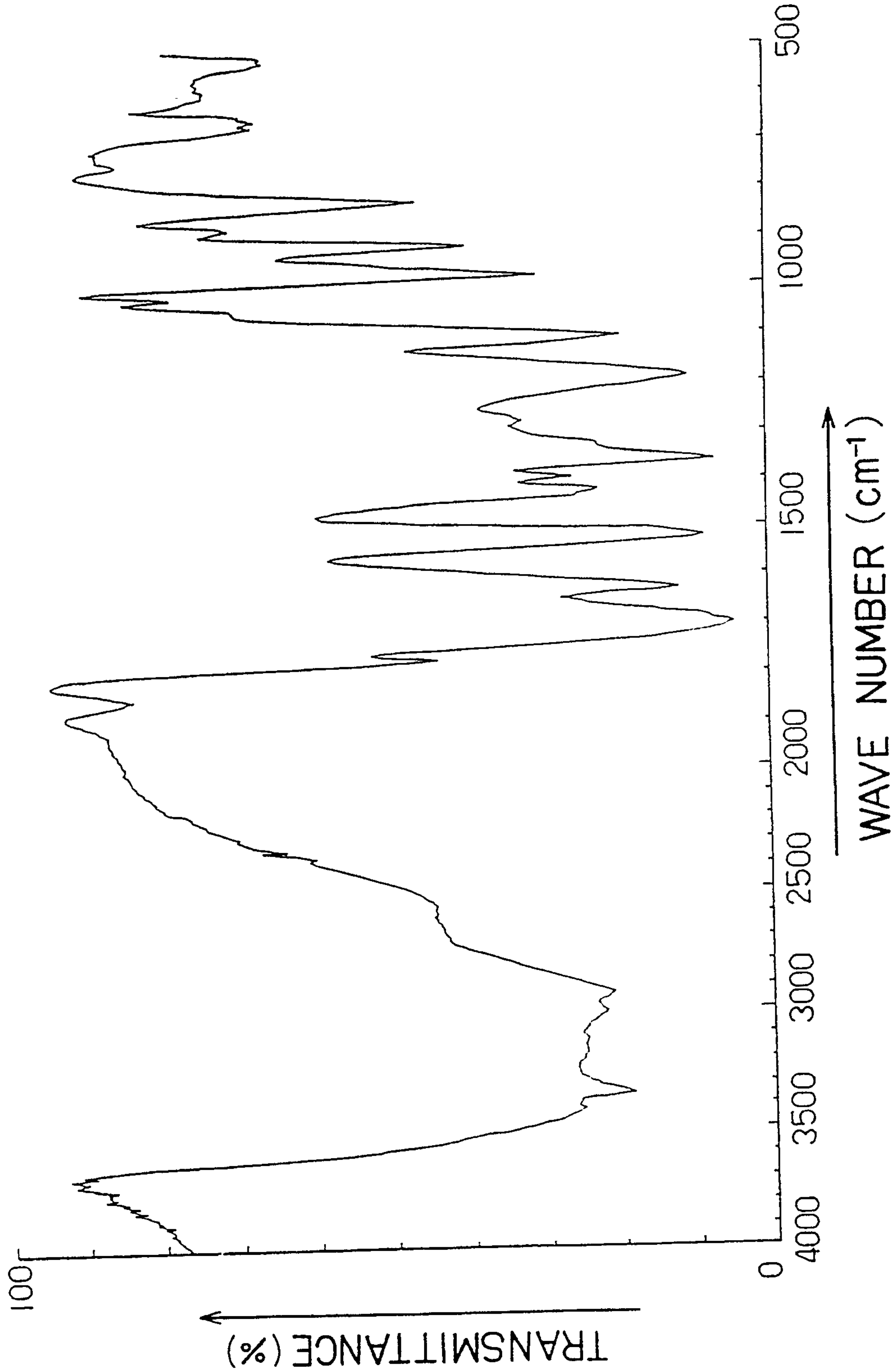
TRANSMITTANCE (%)
100
0
WAVE NUMBER (cm⁻¹)
4000
3500
3000
2500
2000
1500
1000
500

FIG. 13

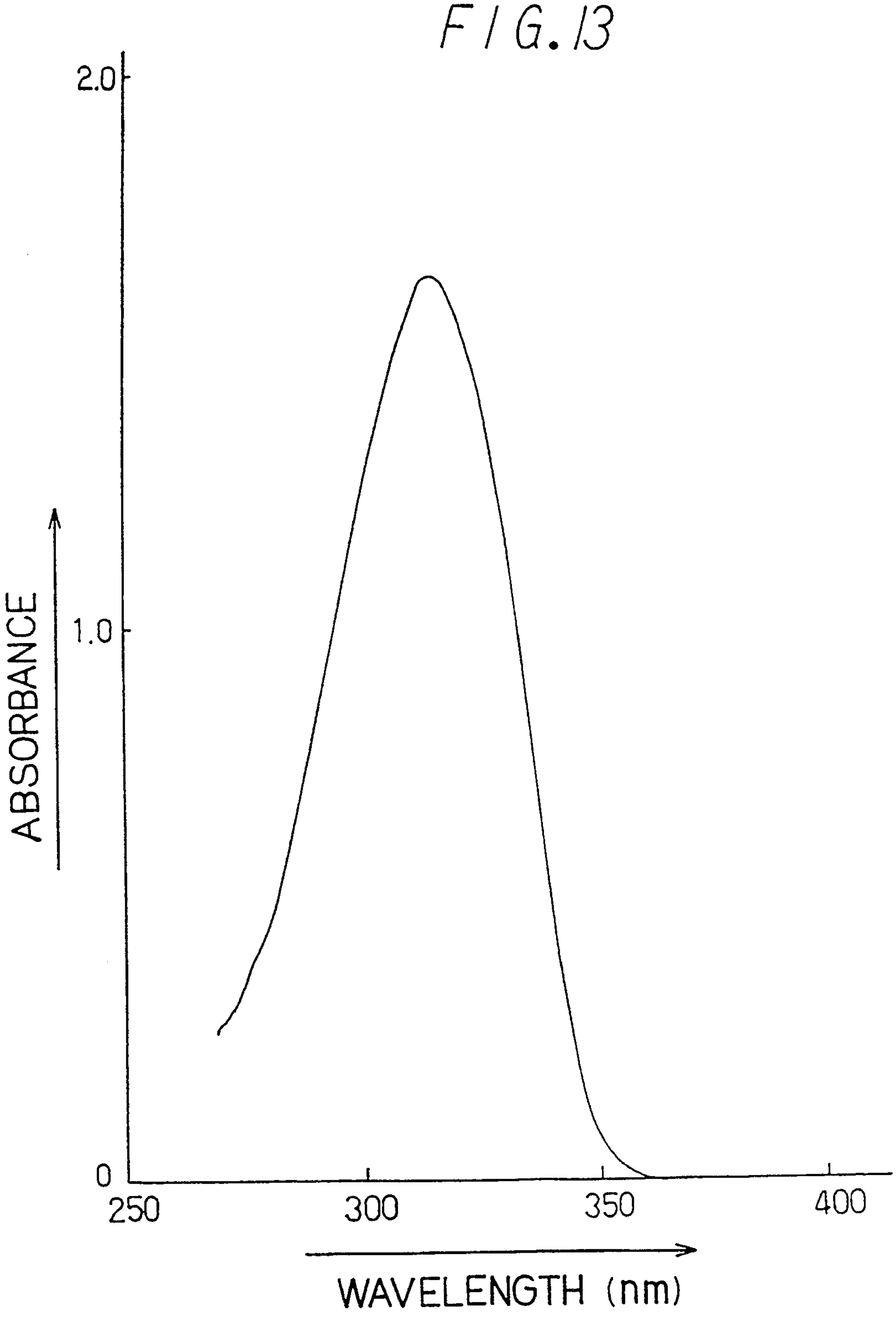

FIG. 14

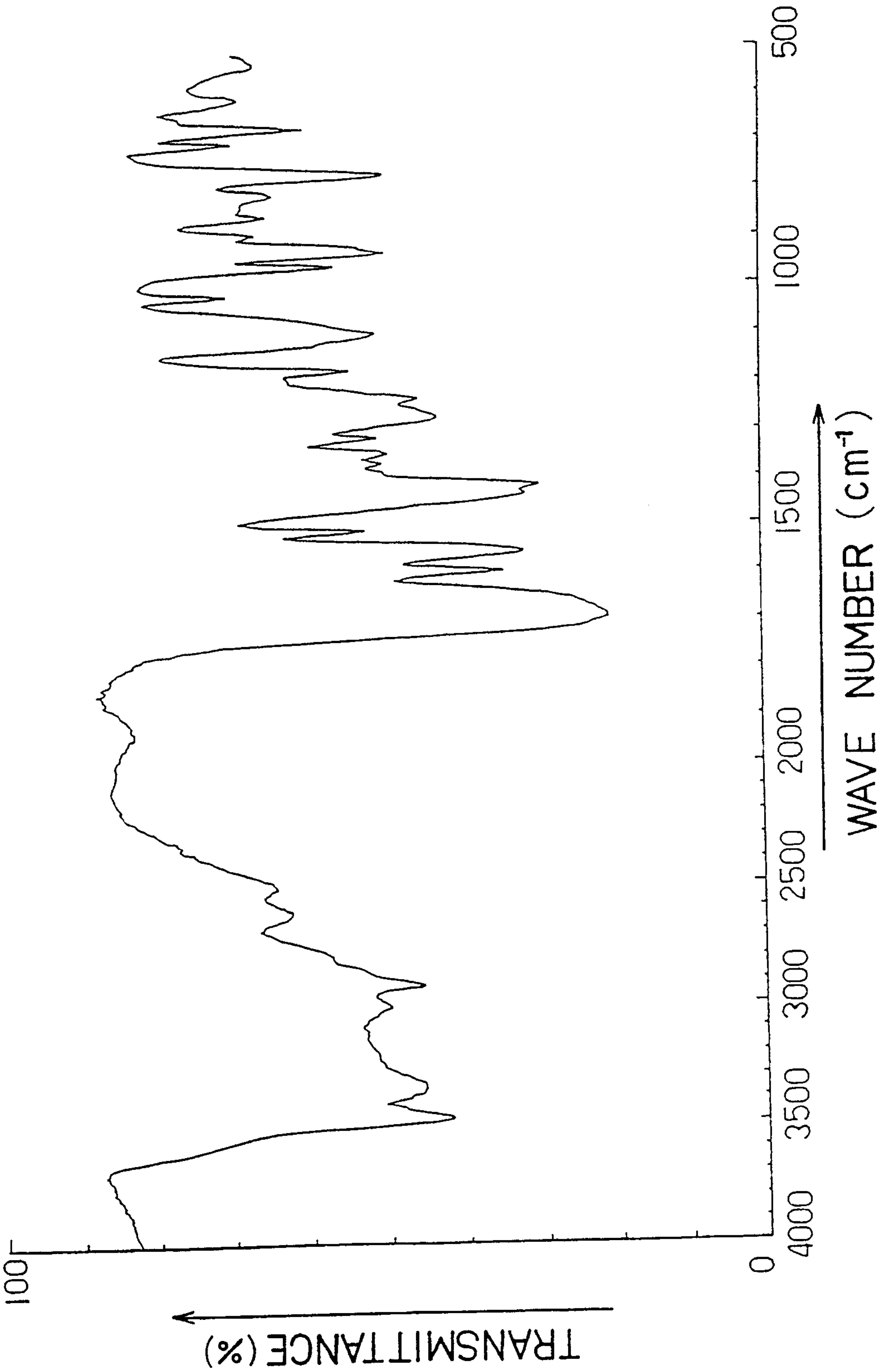
TRANSMITTANCE (%)
100
0
WAVE NUMBER (cm$^{-1}$)
4000
3500
3000
2500
2000
1500
1000
500

FIG.15

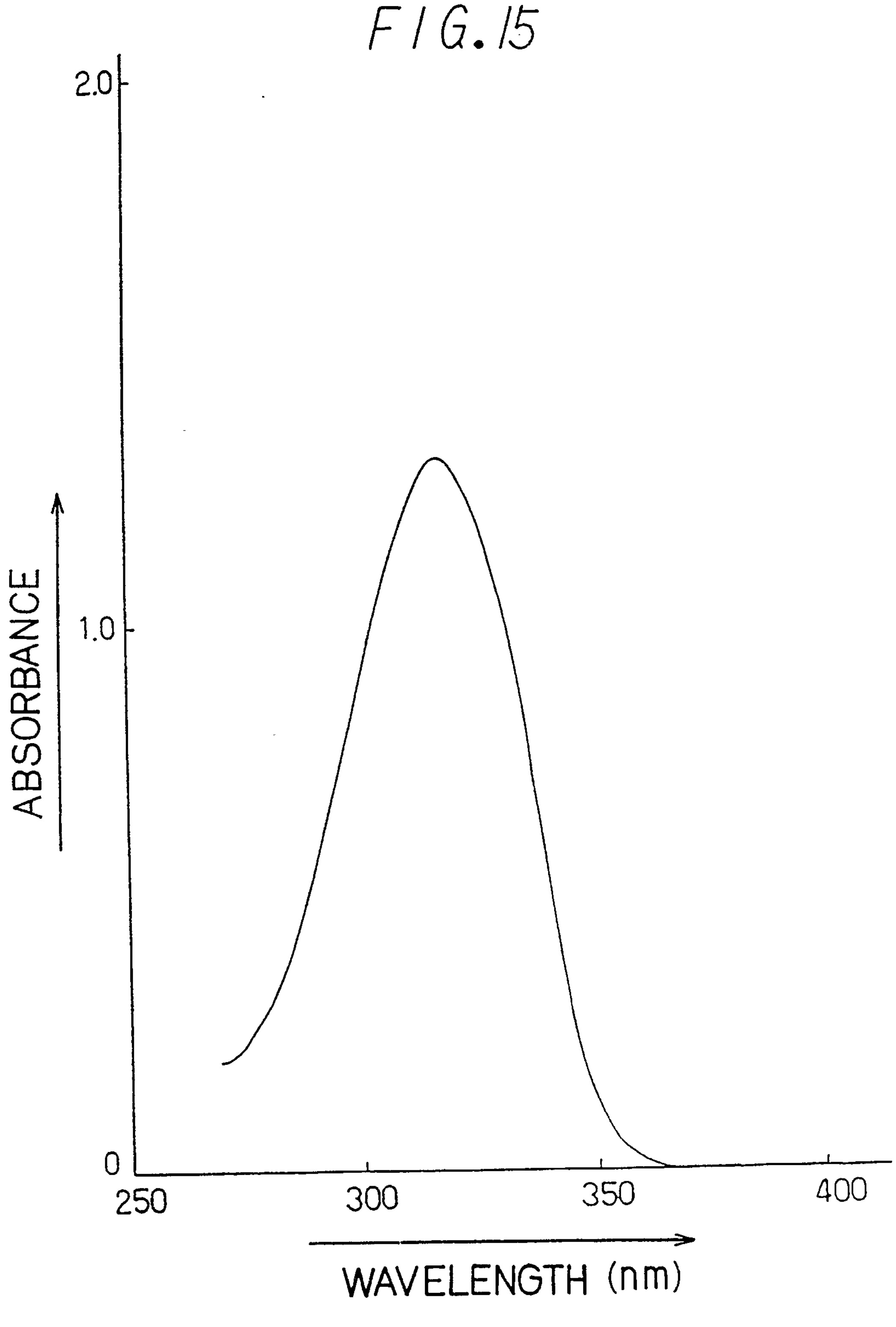

FIG. 16

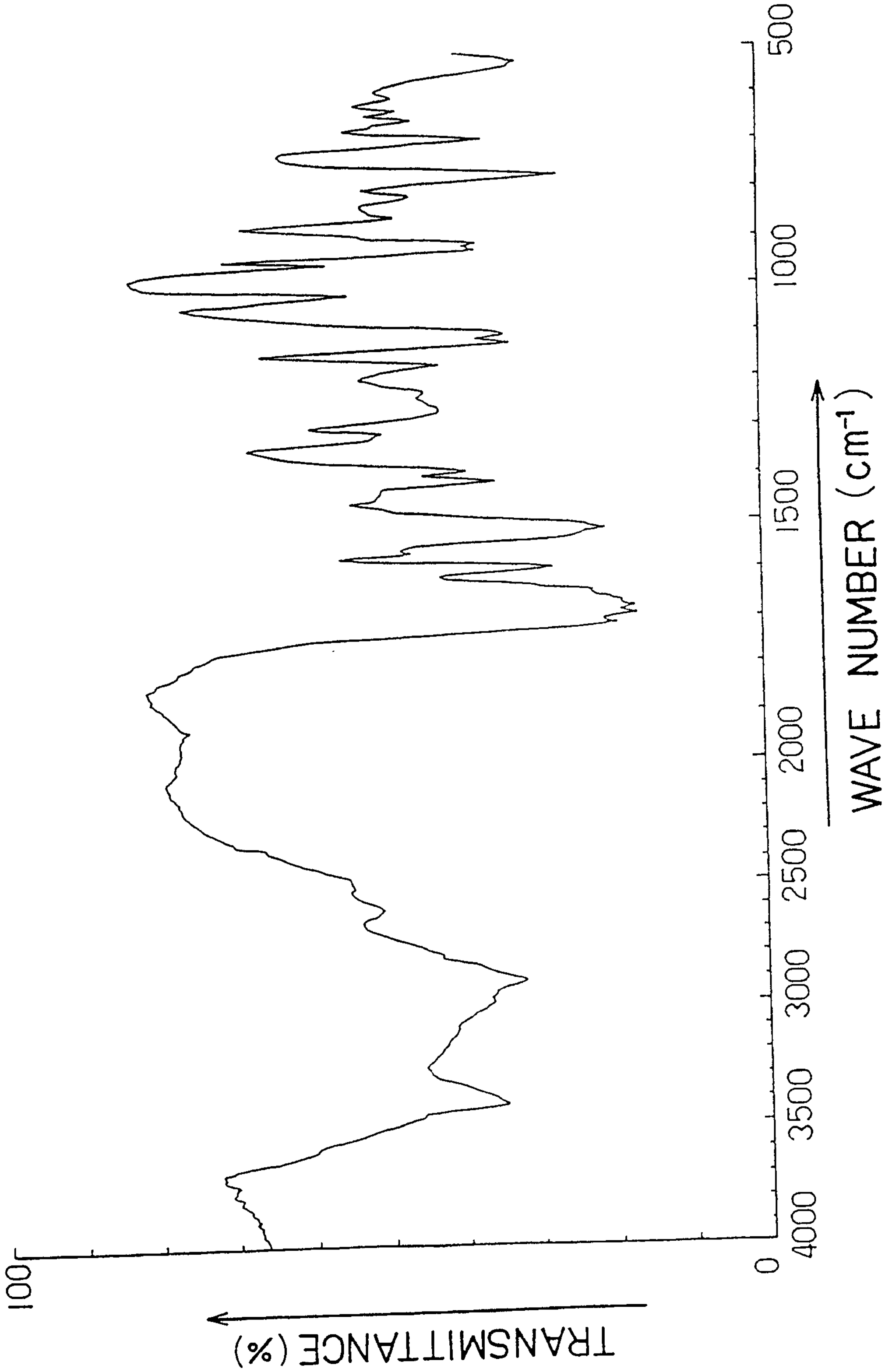
500
1000
1500
2000
2500
3000
3500
4000
0
100
WAVE NUMBER $(cm^{-1})$
TRANSMITTANCE (%)

FIG. 17

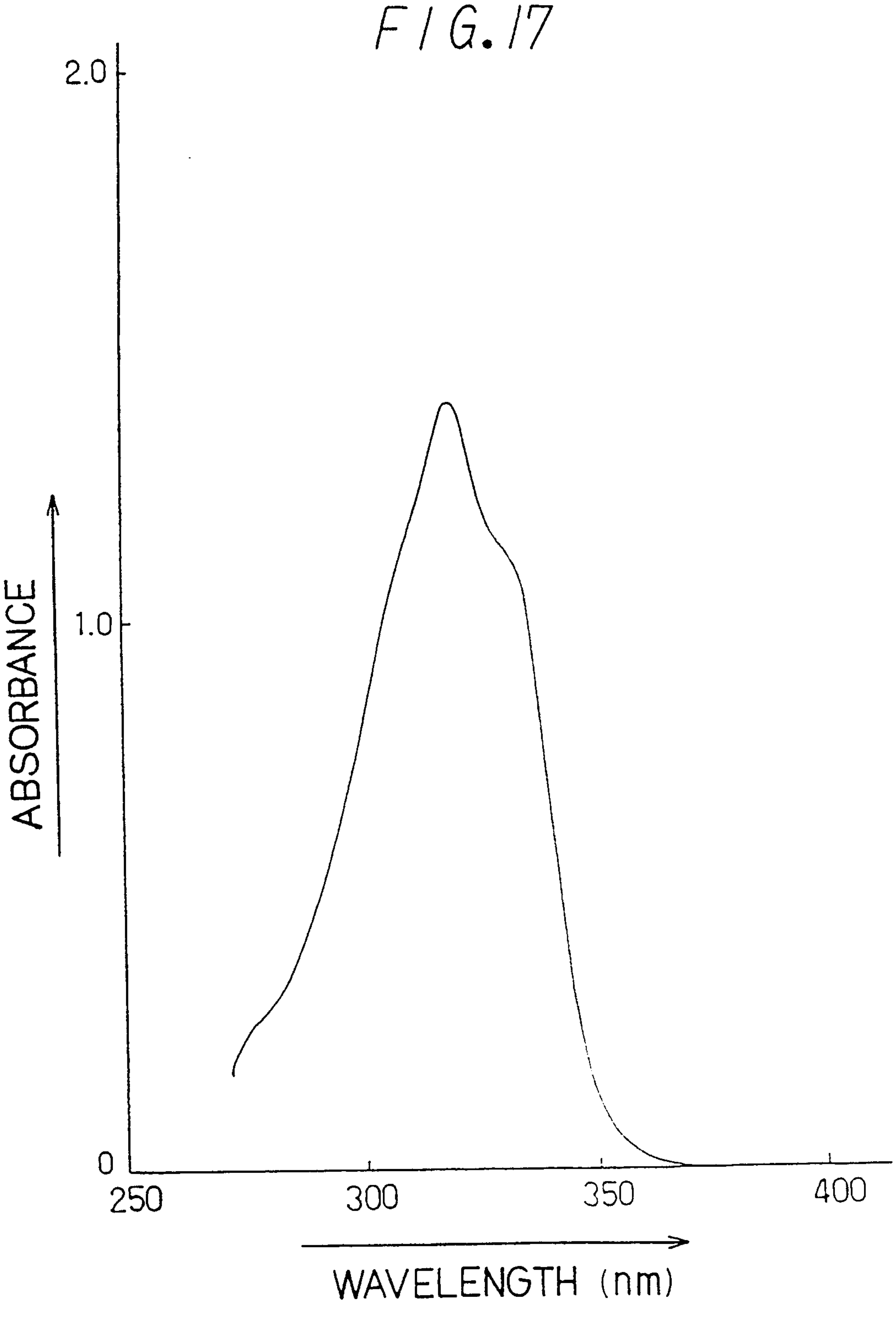

FIG. 18

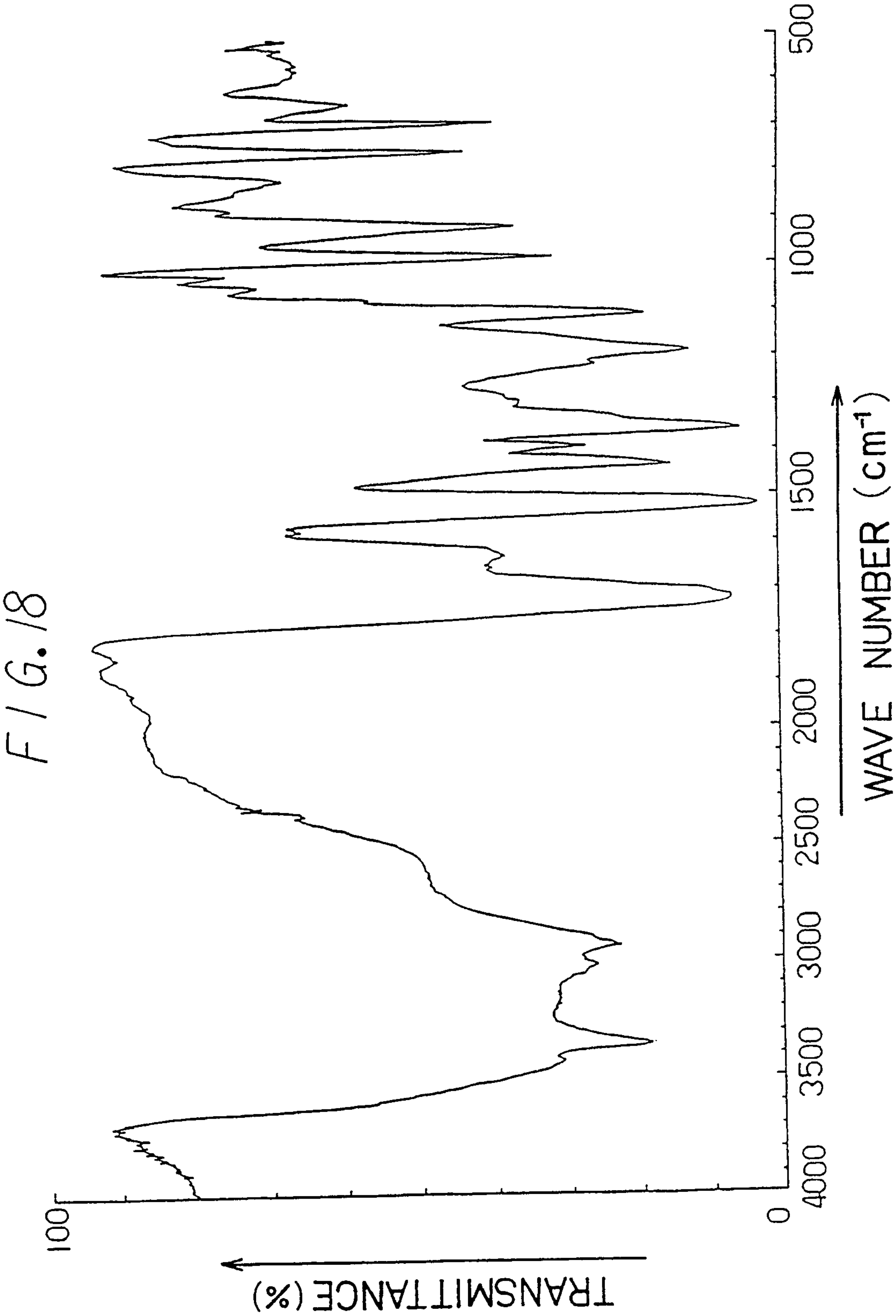
TRANSMITTANCE (%)
100
0
4000
3500
3000
2500
2000
1500
1000
500
WAVE NUMBER (cm⁻¹)

FIG. 19

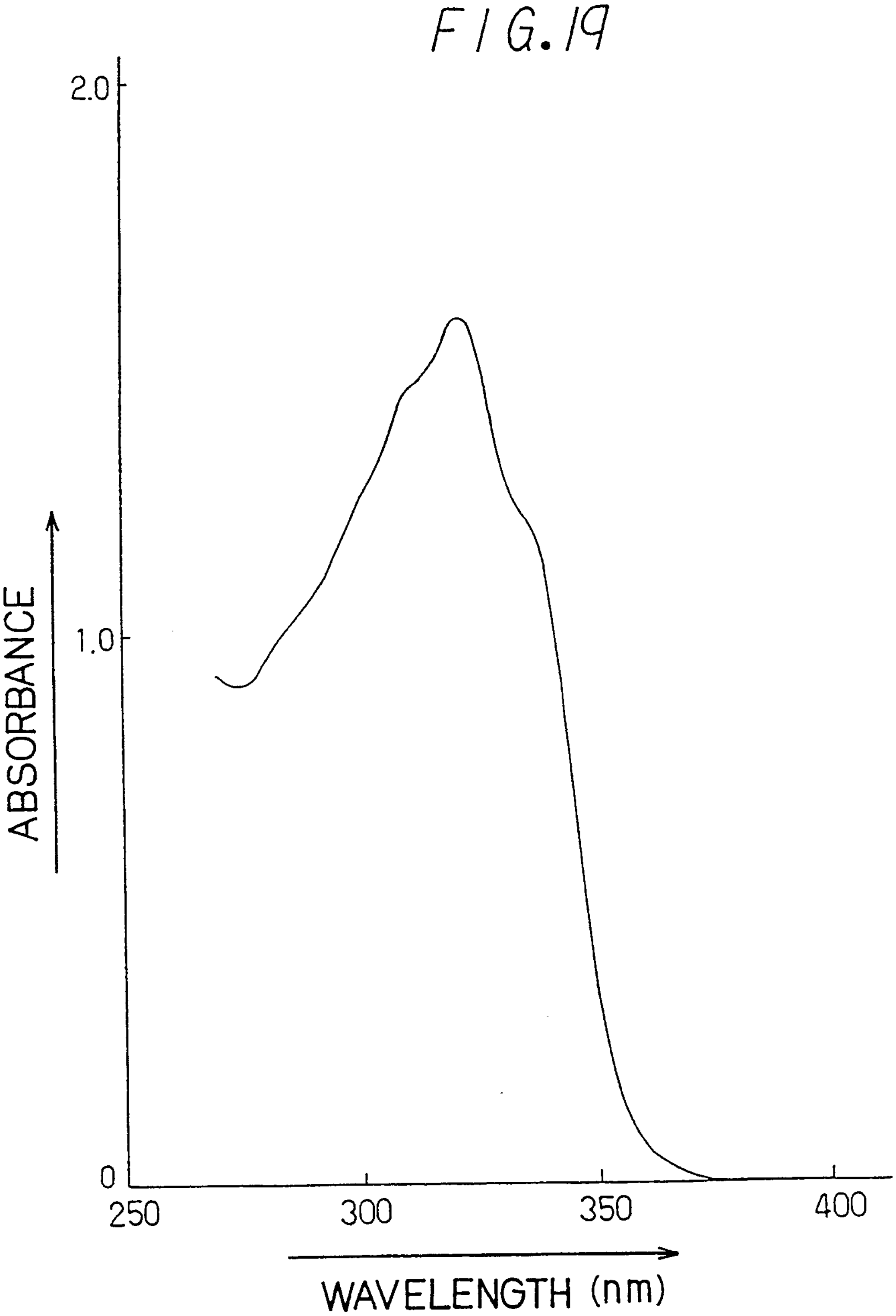

FIG. 20

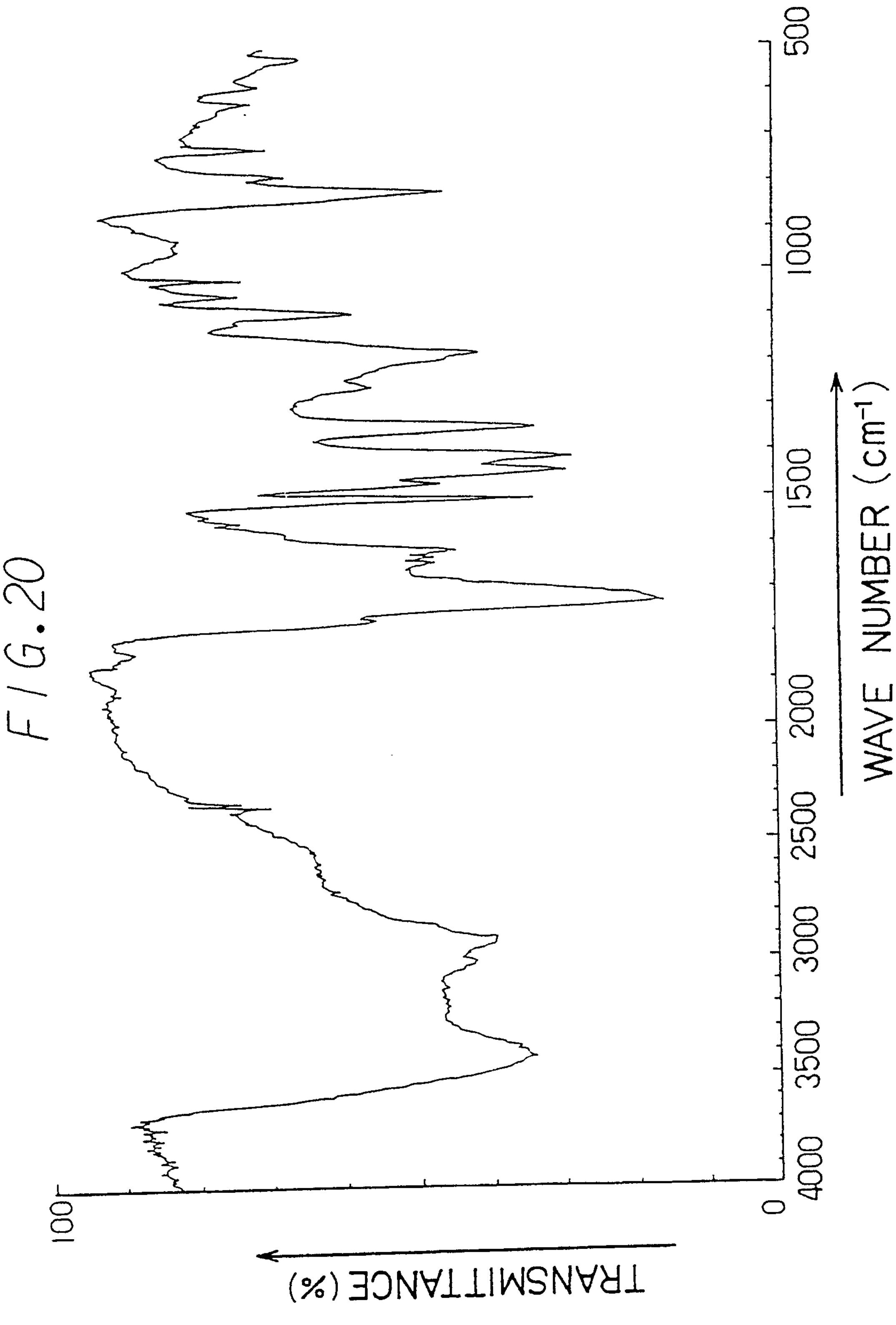
100
0
TRANSMITTANCE (%)
4000
3500
3000
2500
2000
1500
1000
500
WAVE NUMBER (cm$^{-1}$)

FIG. 21

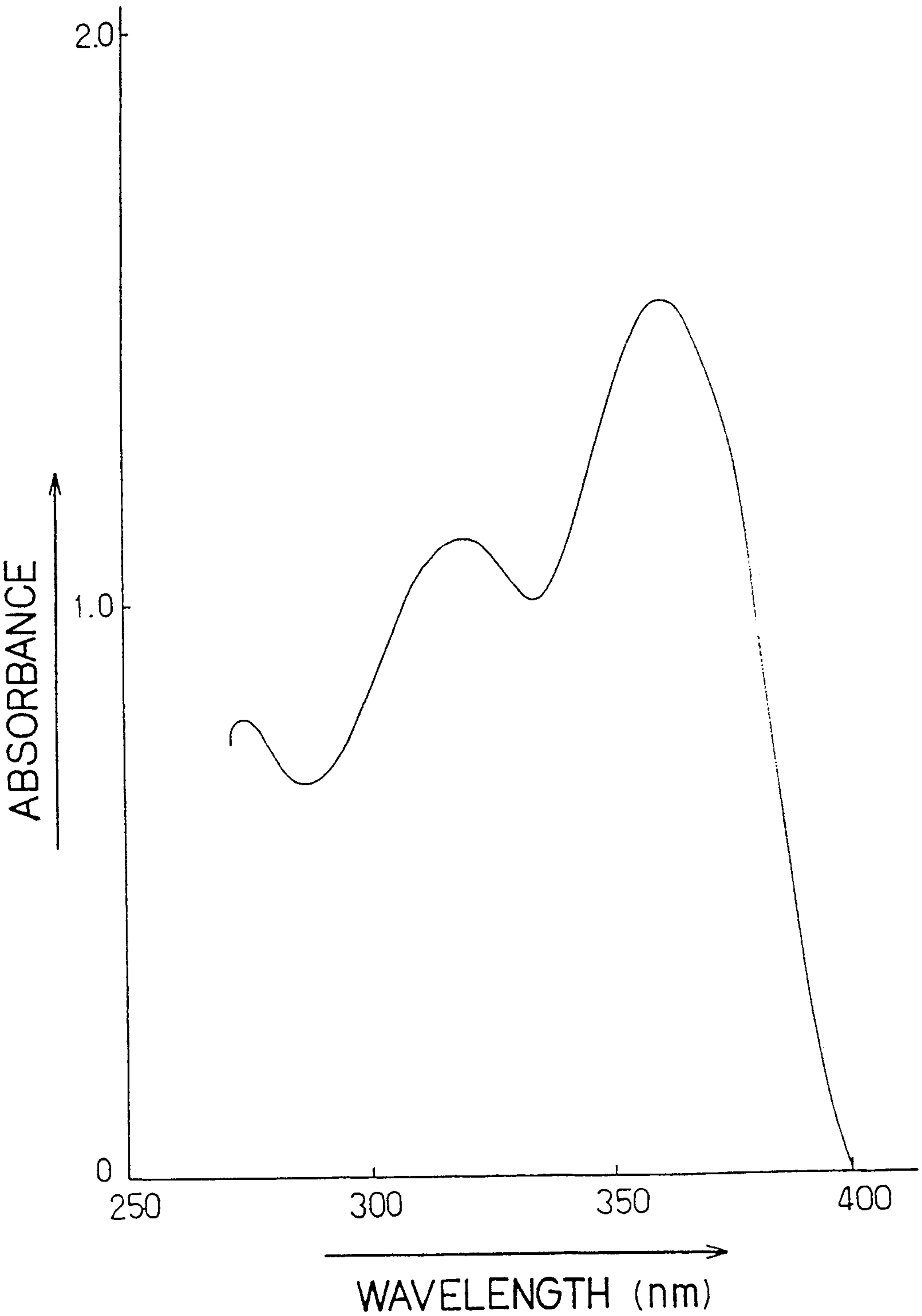

FIG. 22

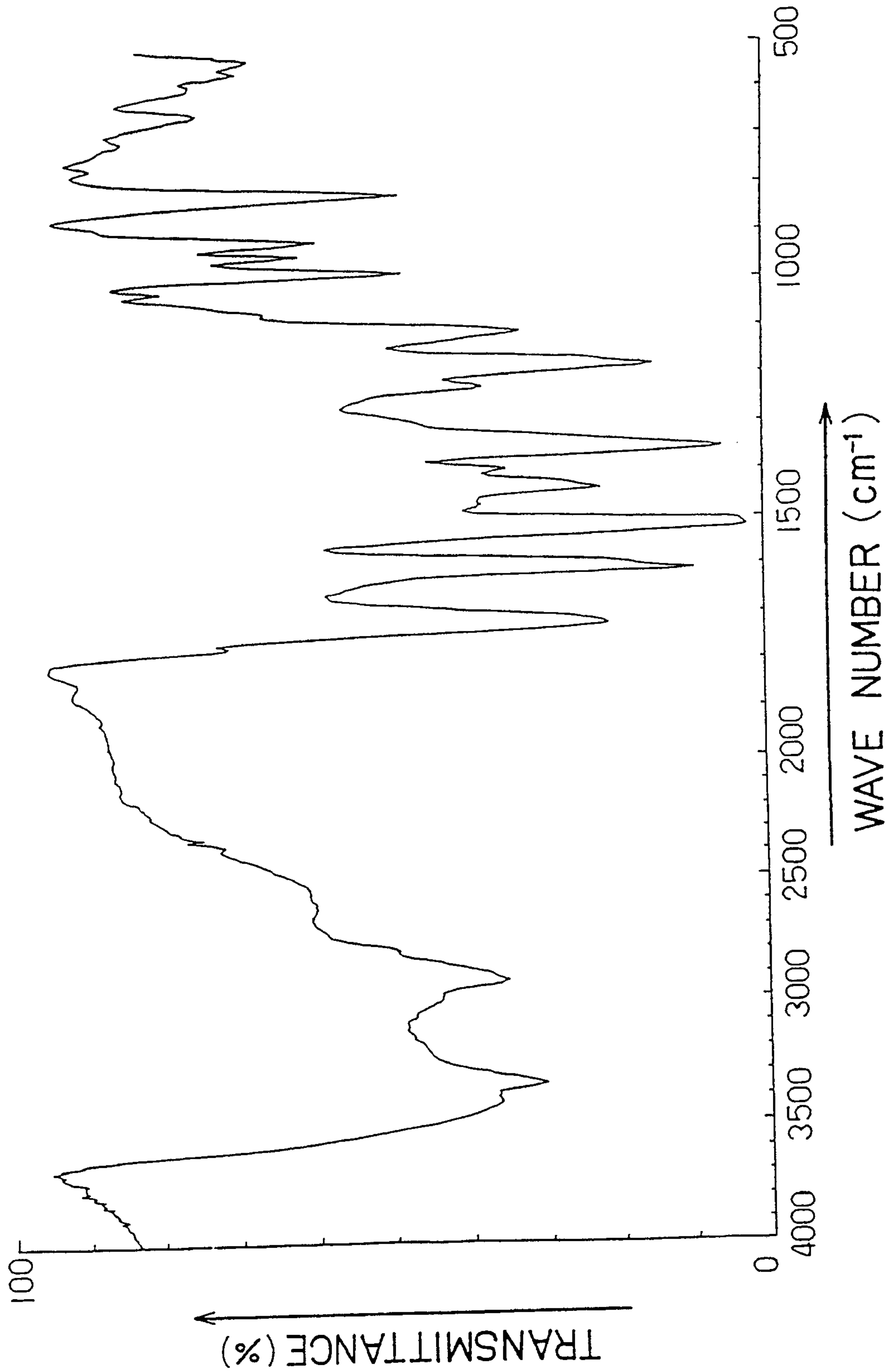
TRANSMITTANCE (%)
100
0
4000
3500
3000
2500
2000
1500
1000
500
WAVE NUMBER (cm⁻¹)

FIG. 23

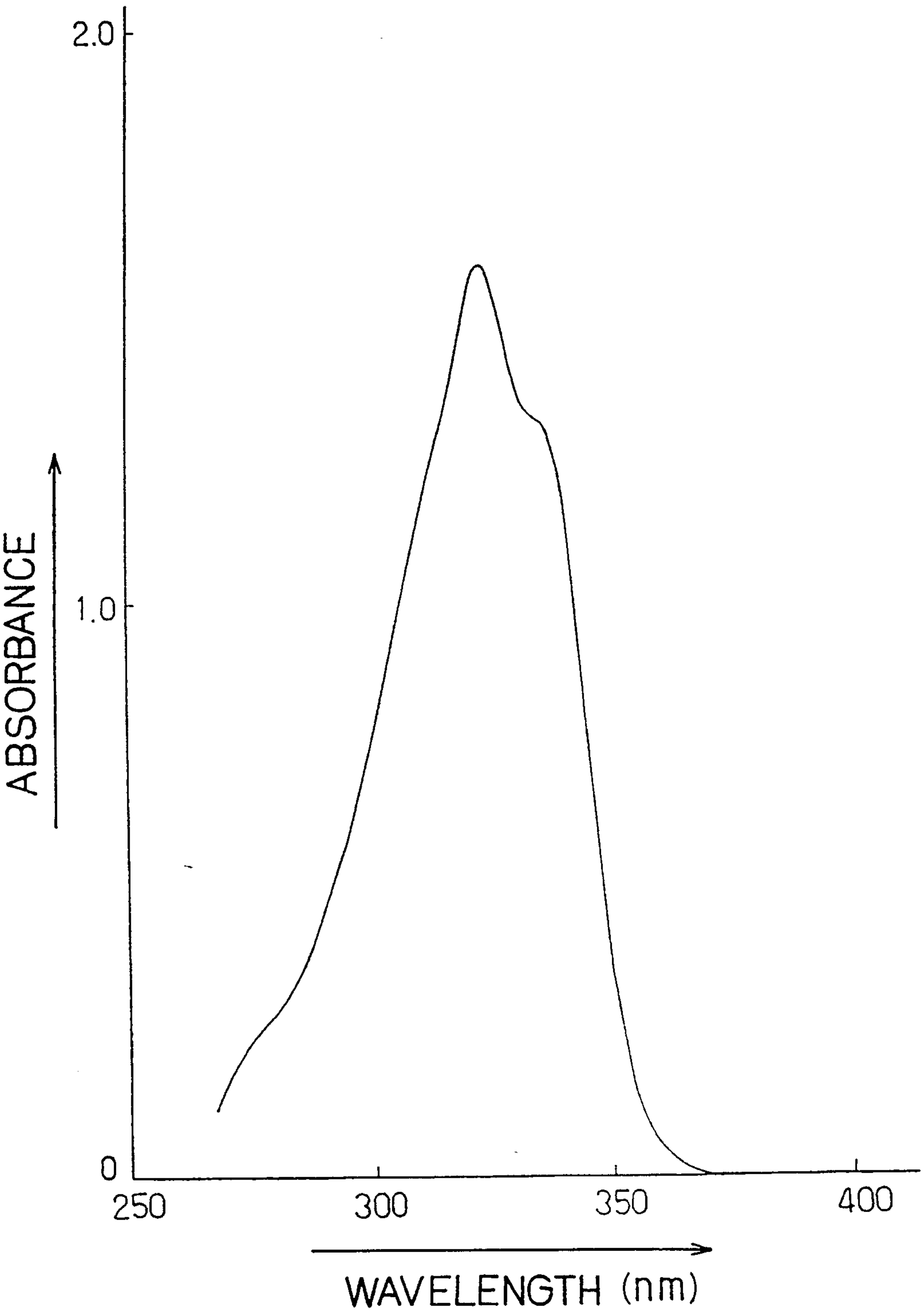

FIG. 24

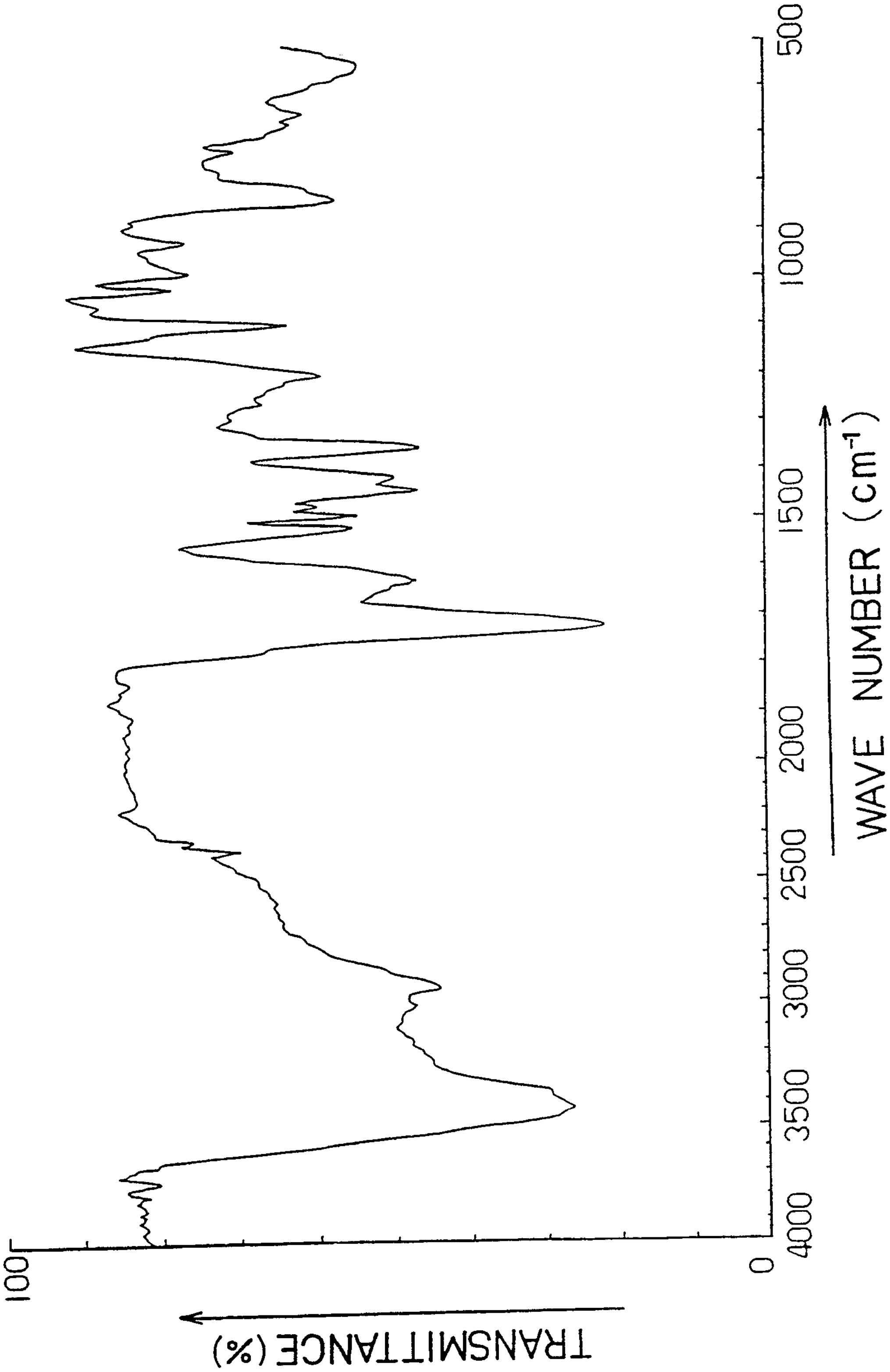
TRANSMITTANCE (%)
100
0
4000
3500
3000
2500
2000
1500
1000
500
WAVE NUMBER (cm⁻¹)

FIG. 25

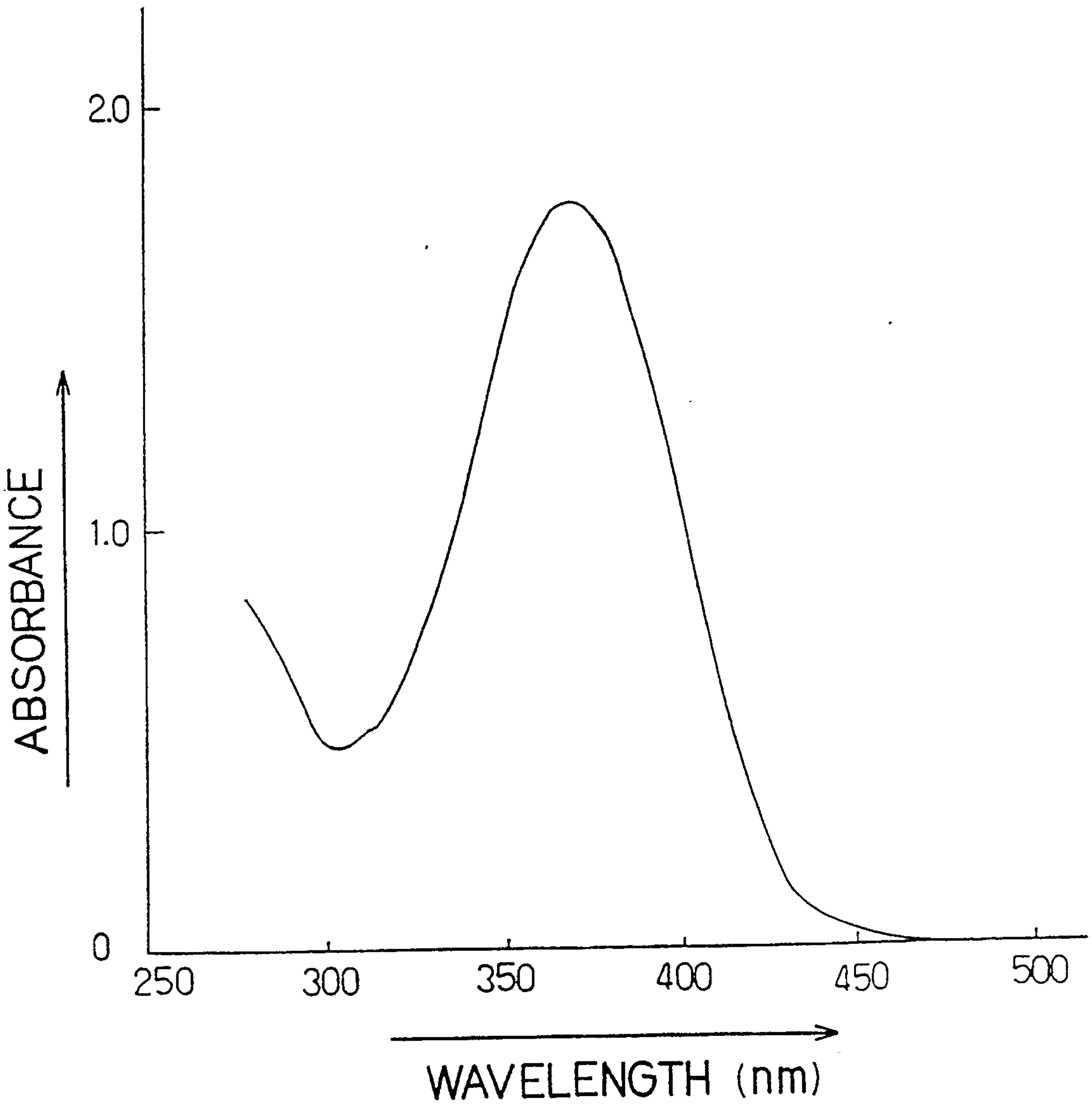

FIG. 26

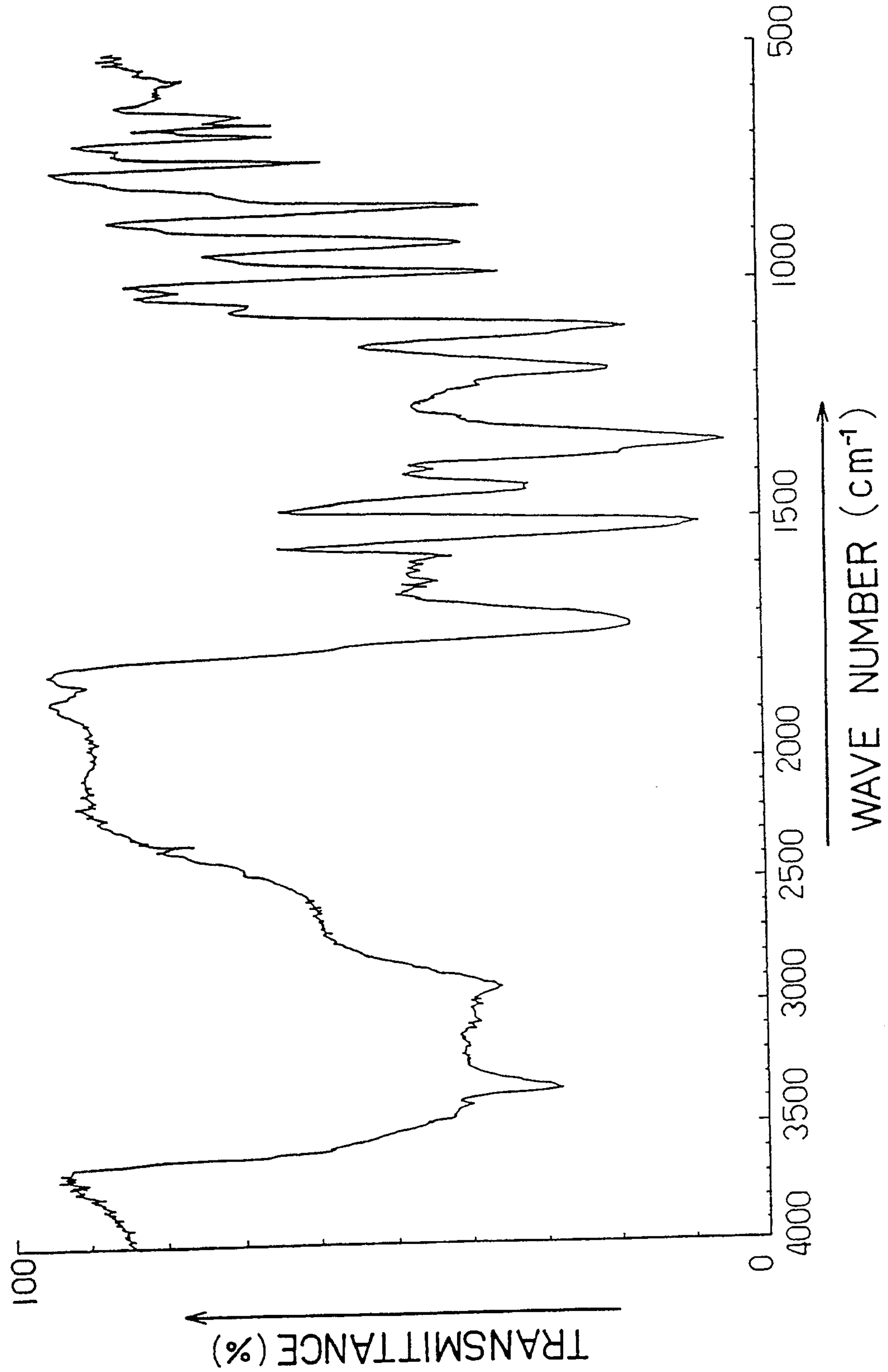
TRANSMITTANCE (%)
100
0
WAVE NUMBER ($cm^{-1}$)
4000
3500
3000
2500
2000
1500
1000
500

FIG. 27

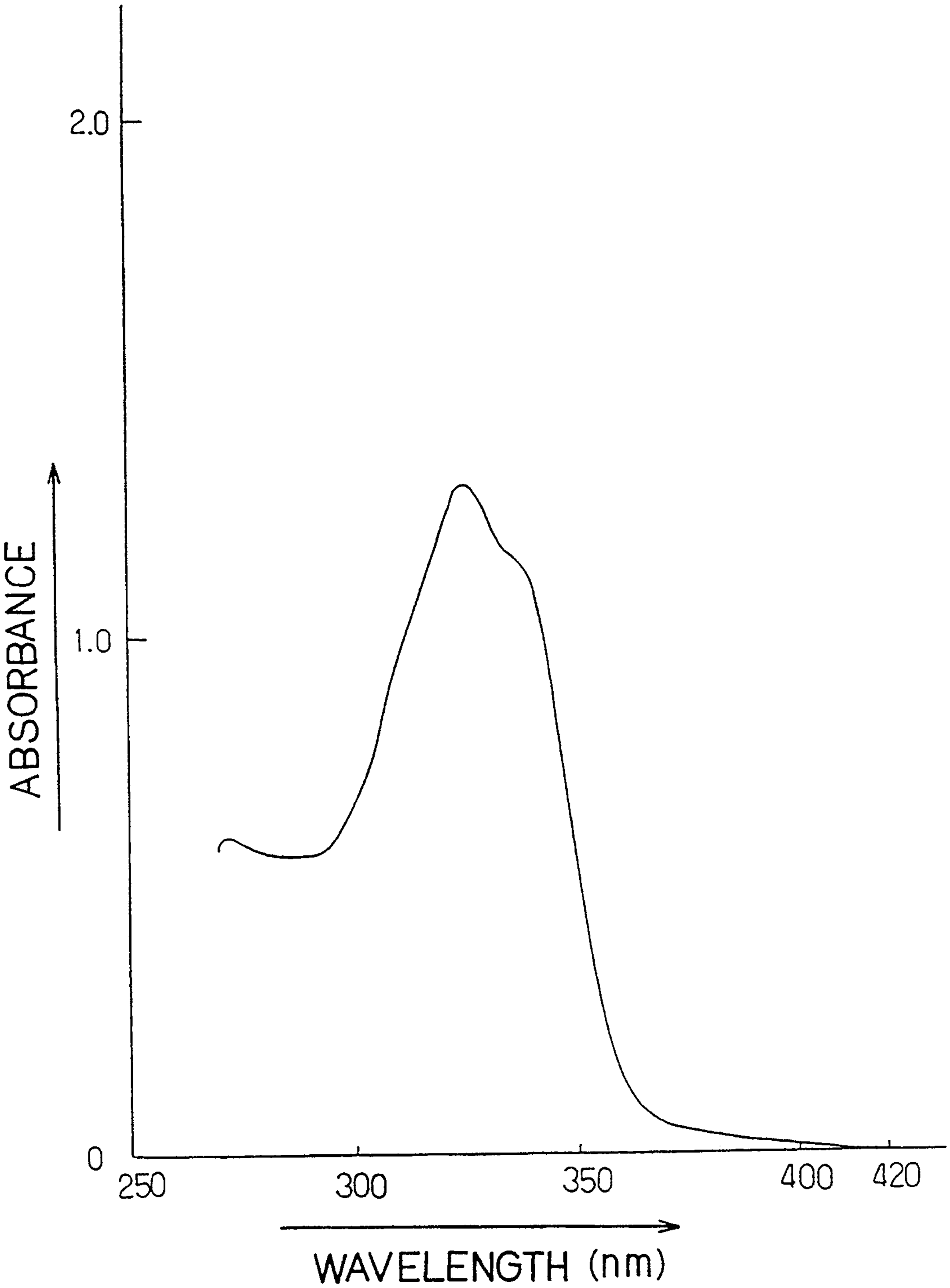

FIG. 28

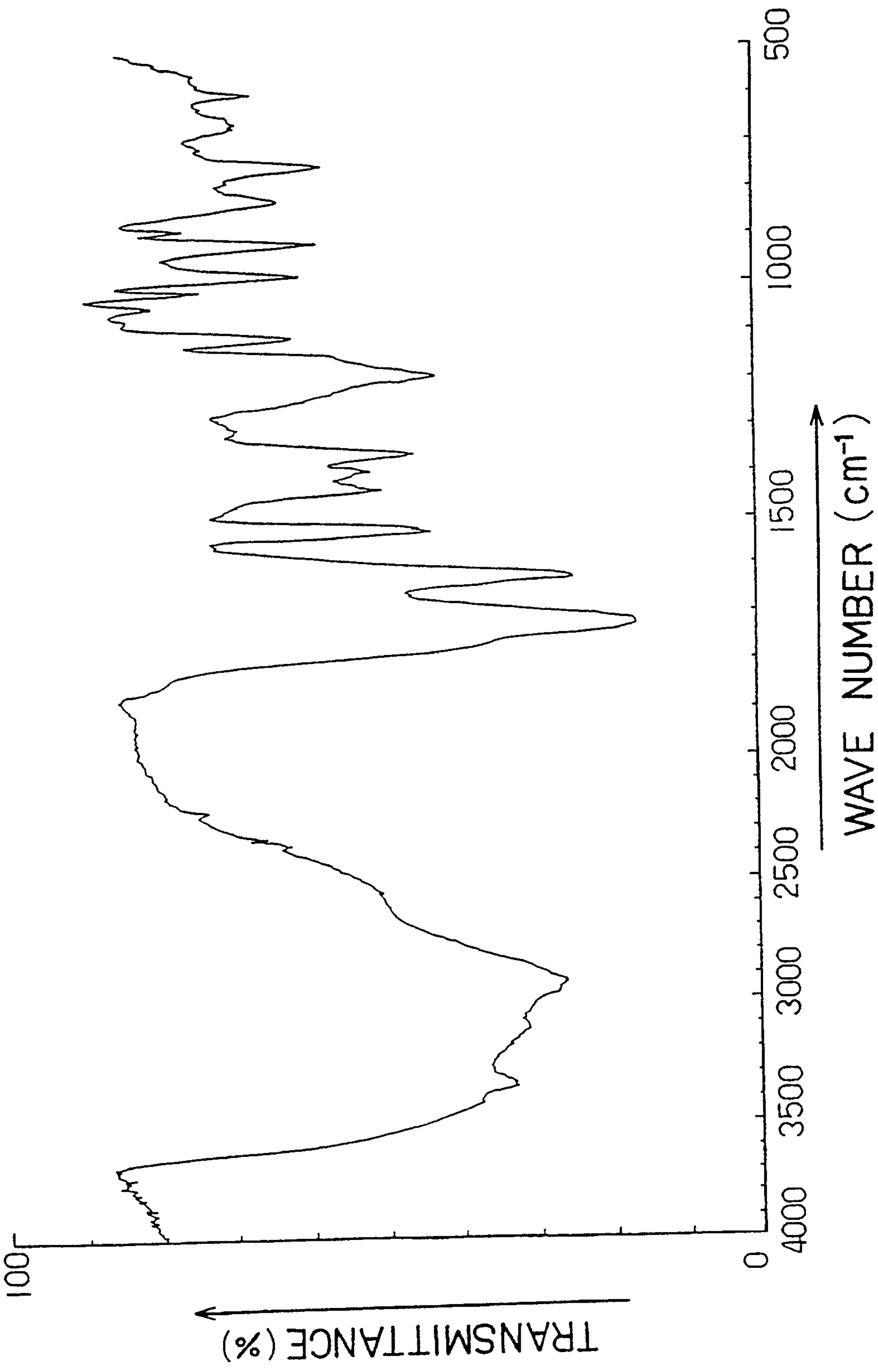

TRANSMITTANCE (%)
100
0
4000
3500
3000
2500
2000
1500
1000
500
WAVE NUMBER (cm$^{-1}$)

FIG. 29

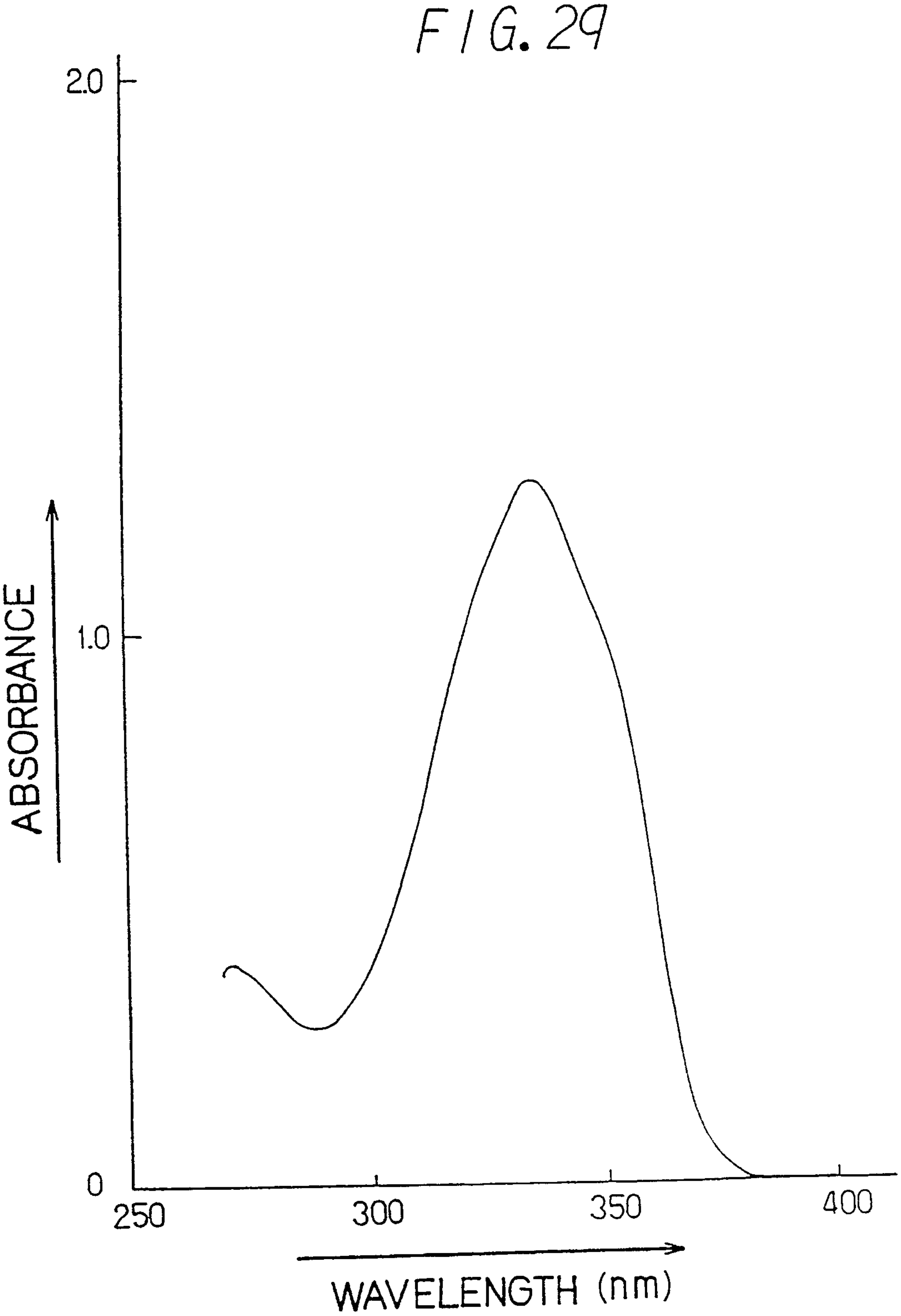

FIG. 30

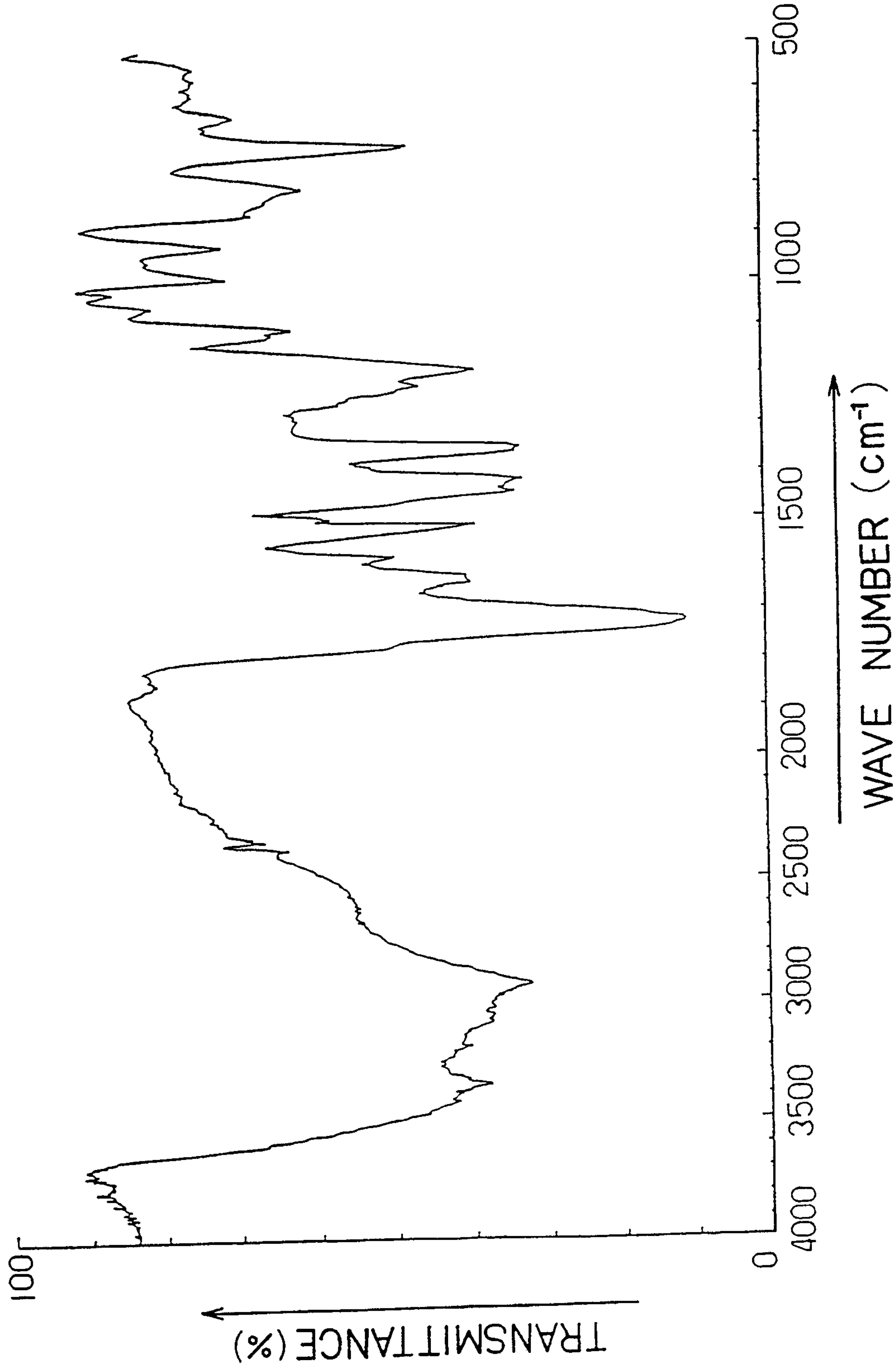
TRANSMITTANCE (%)
100
0
4000
3500
3000
2500
2000
1500
1000
500
WAVE NUMBER (cm⁻¹)

FIG. 31

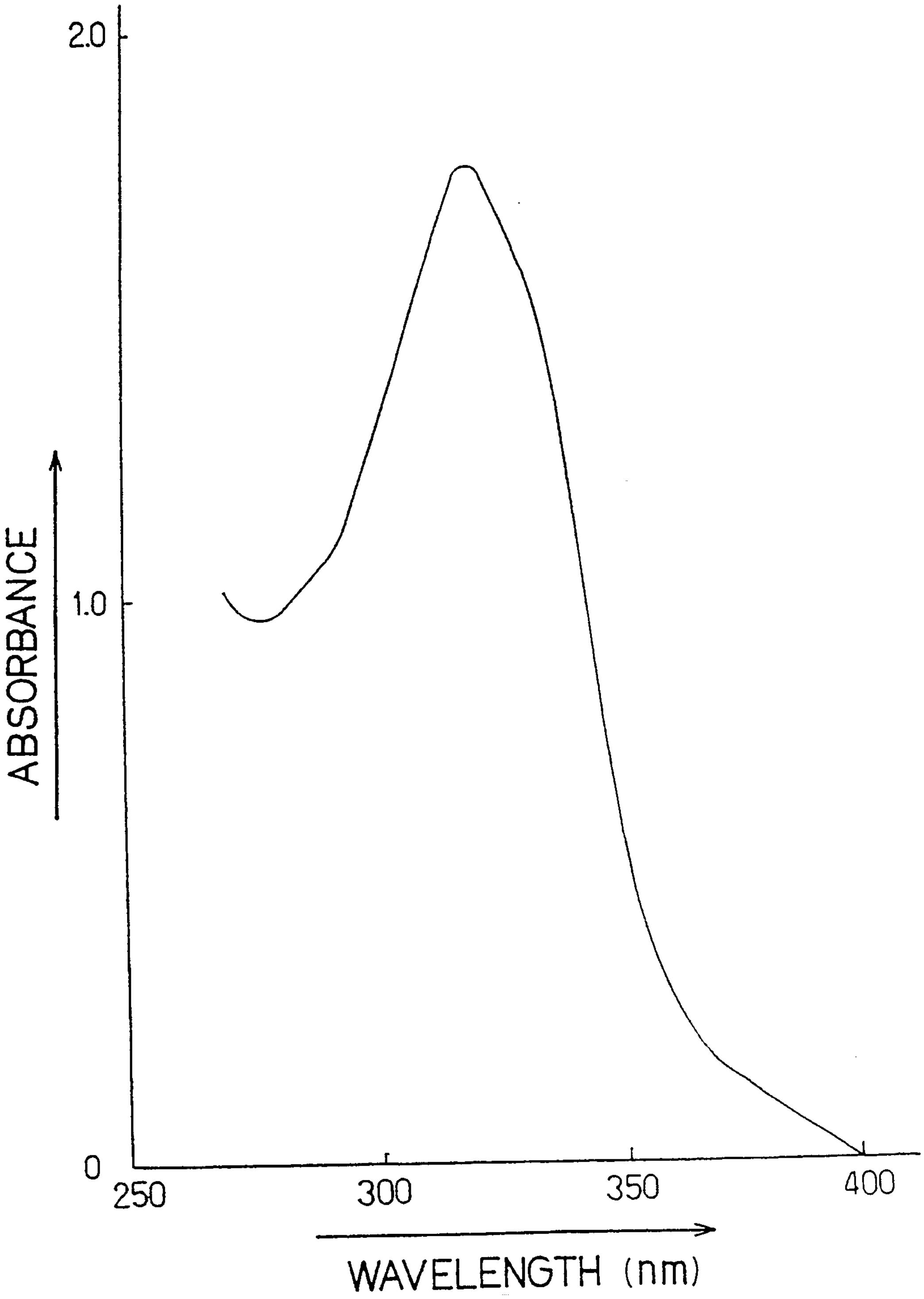

FIG. 32

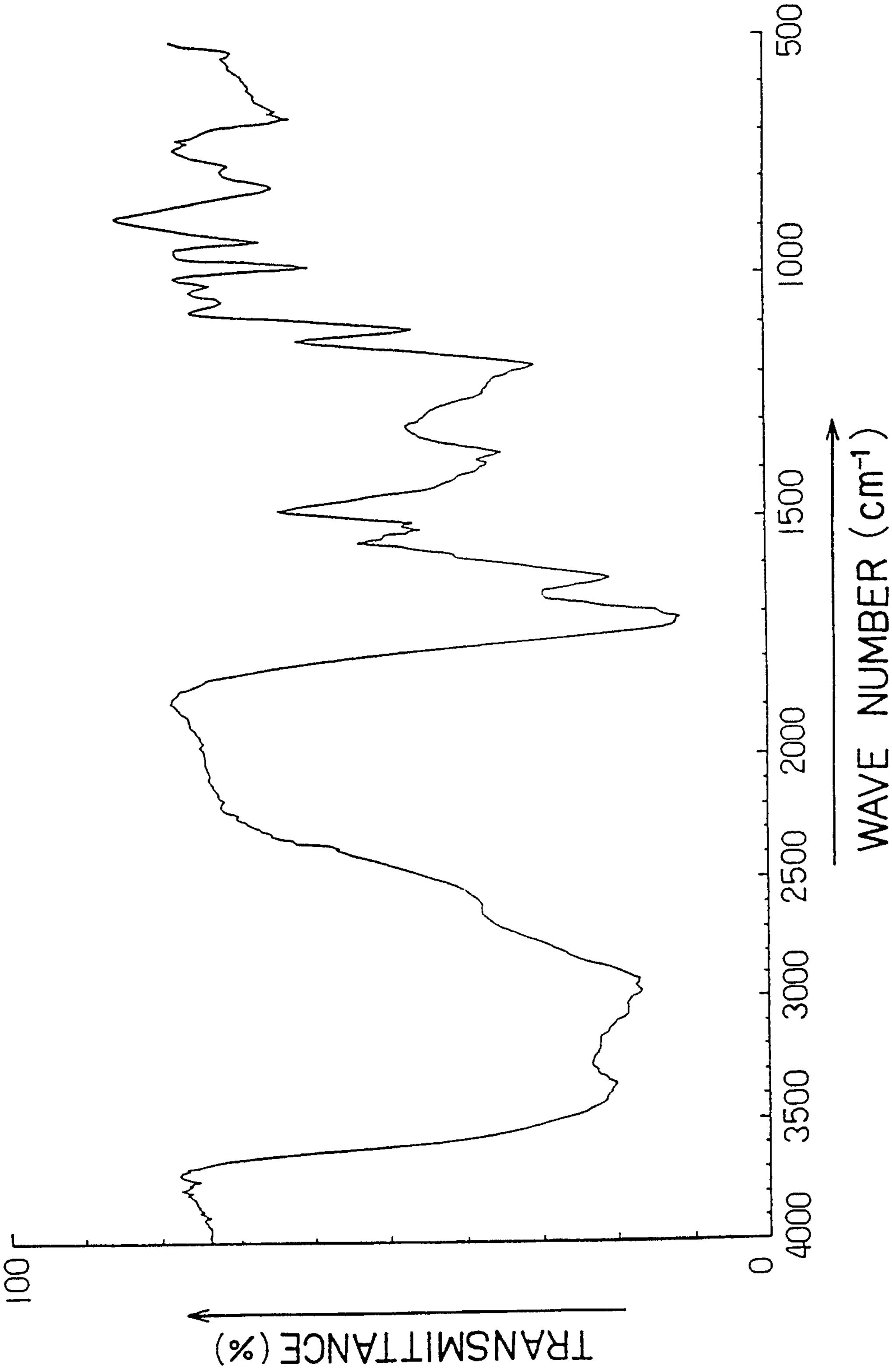
TRANSMITTANCE (%)
100
0
4000
3500
3000
2500
2000
1500
1000
500
WAVE NUMBER (cm⁻¹)

FIG.33

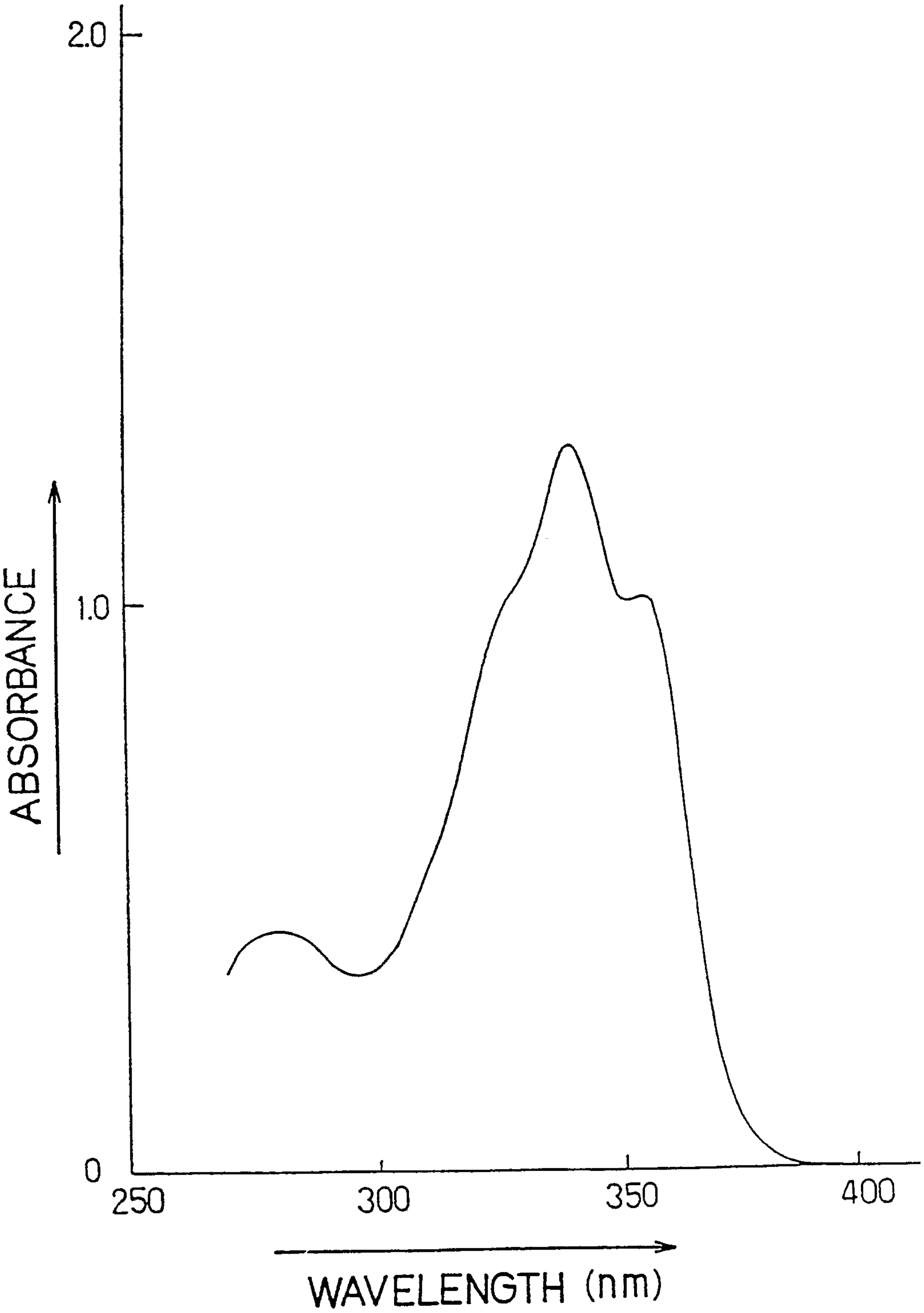

FIG. 34

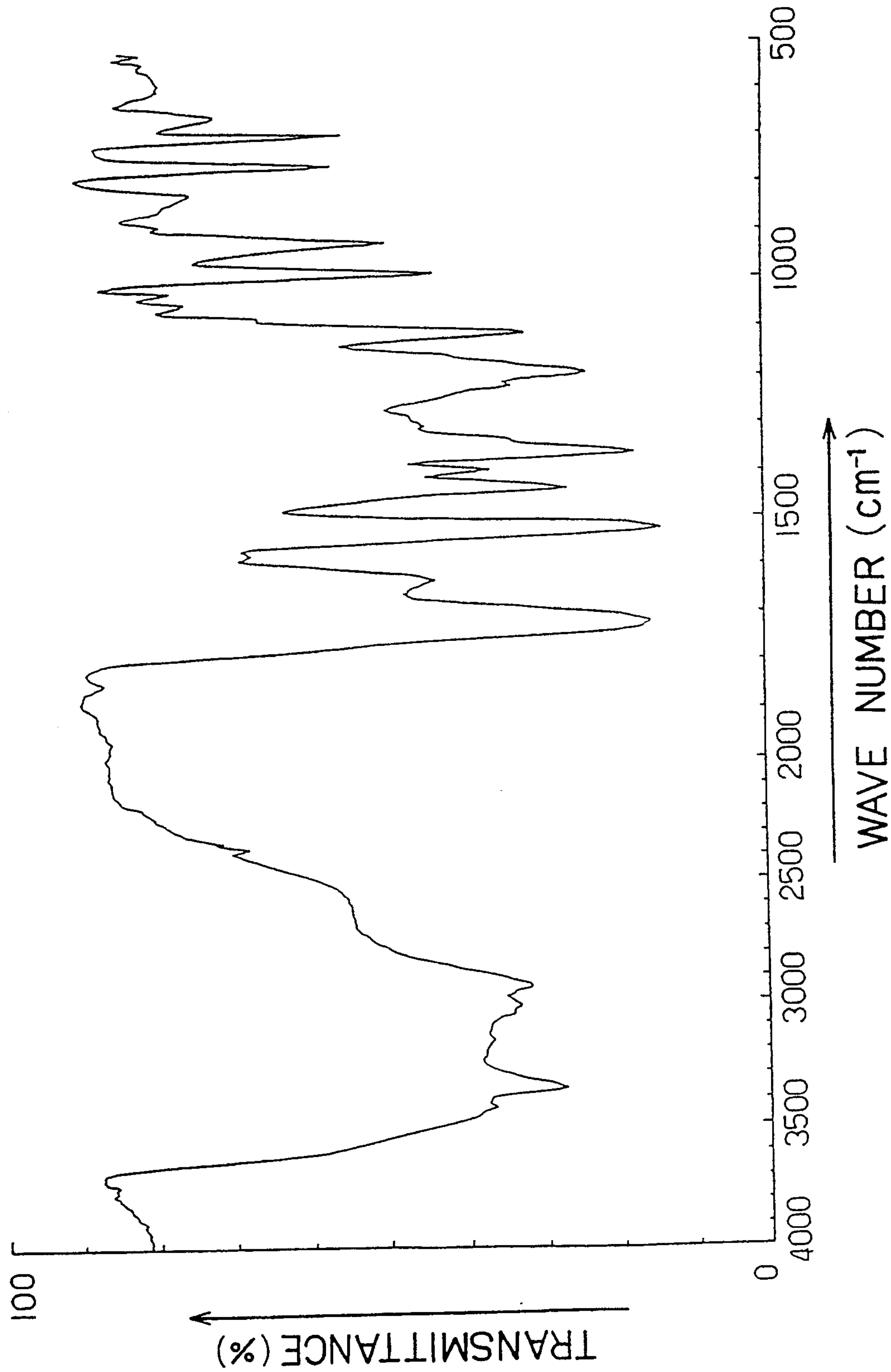
TRANSMITTANCE (%)
100
0
WAVE NUMBER (cm⁻¹)
4000
3500
3000
2500
2000
1500
1000
500

FIG. 35

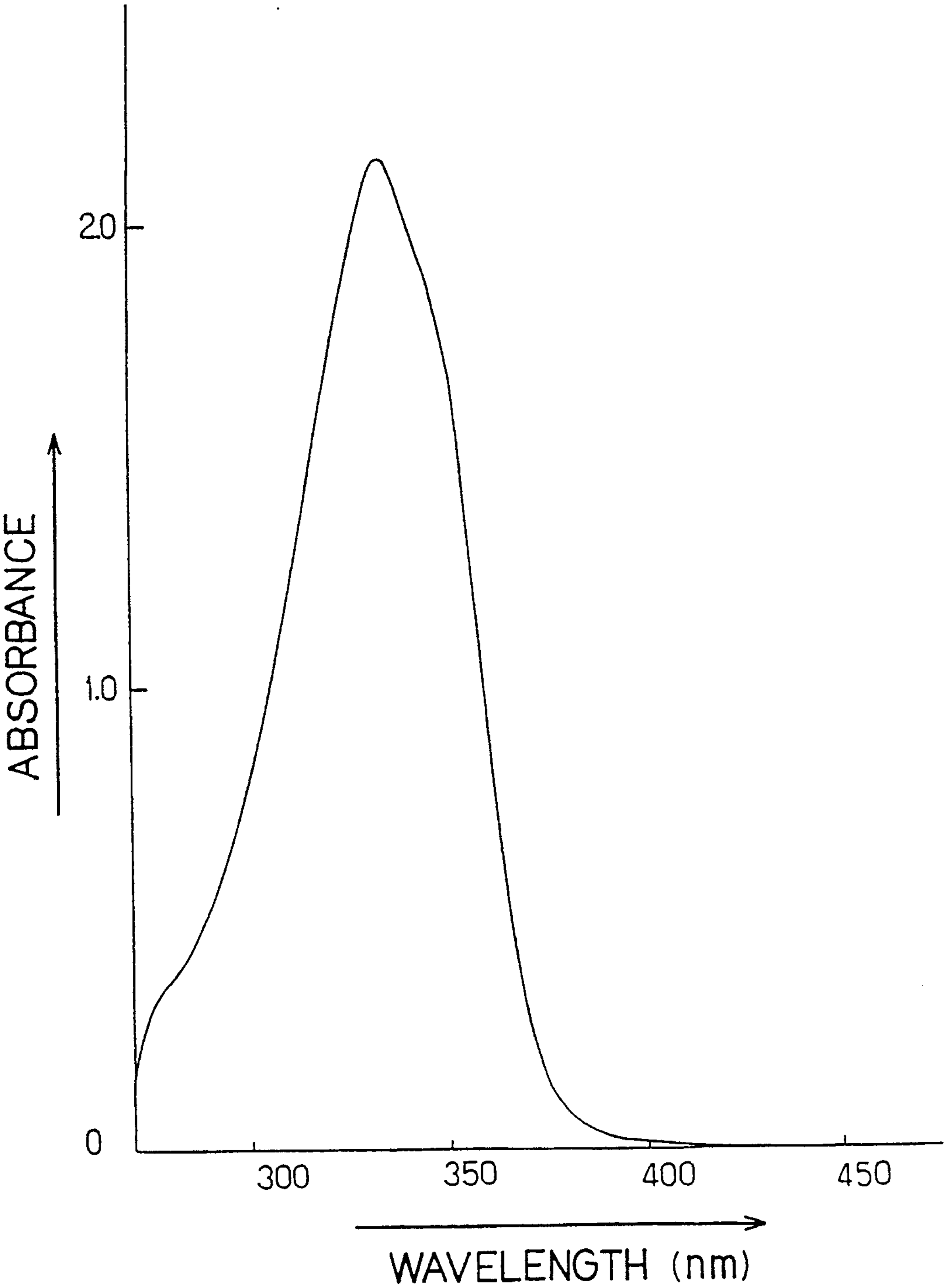

FIG. 36

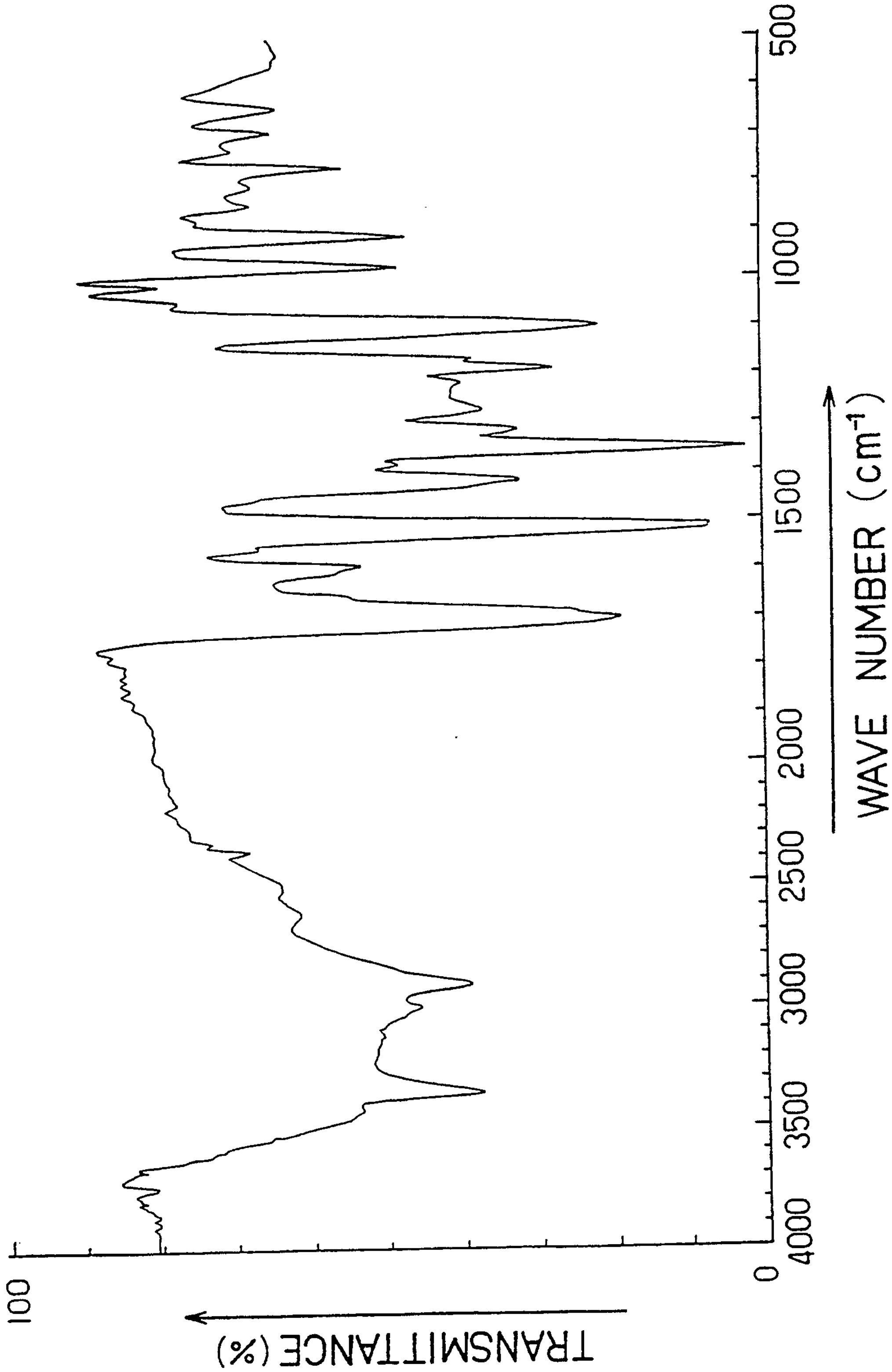
100
0
TRANSMITTANCE (%)
4000
3500
3000
2500
2000
1500
1000
500
WAVE NUMBER ($cm^{-1}$)

F I G. 37

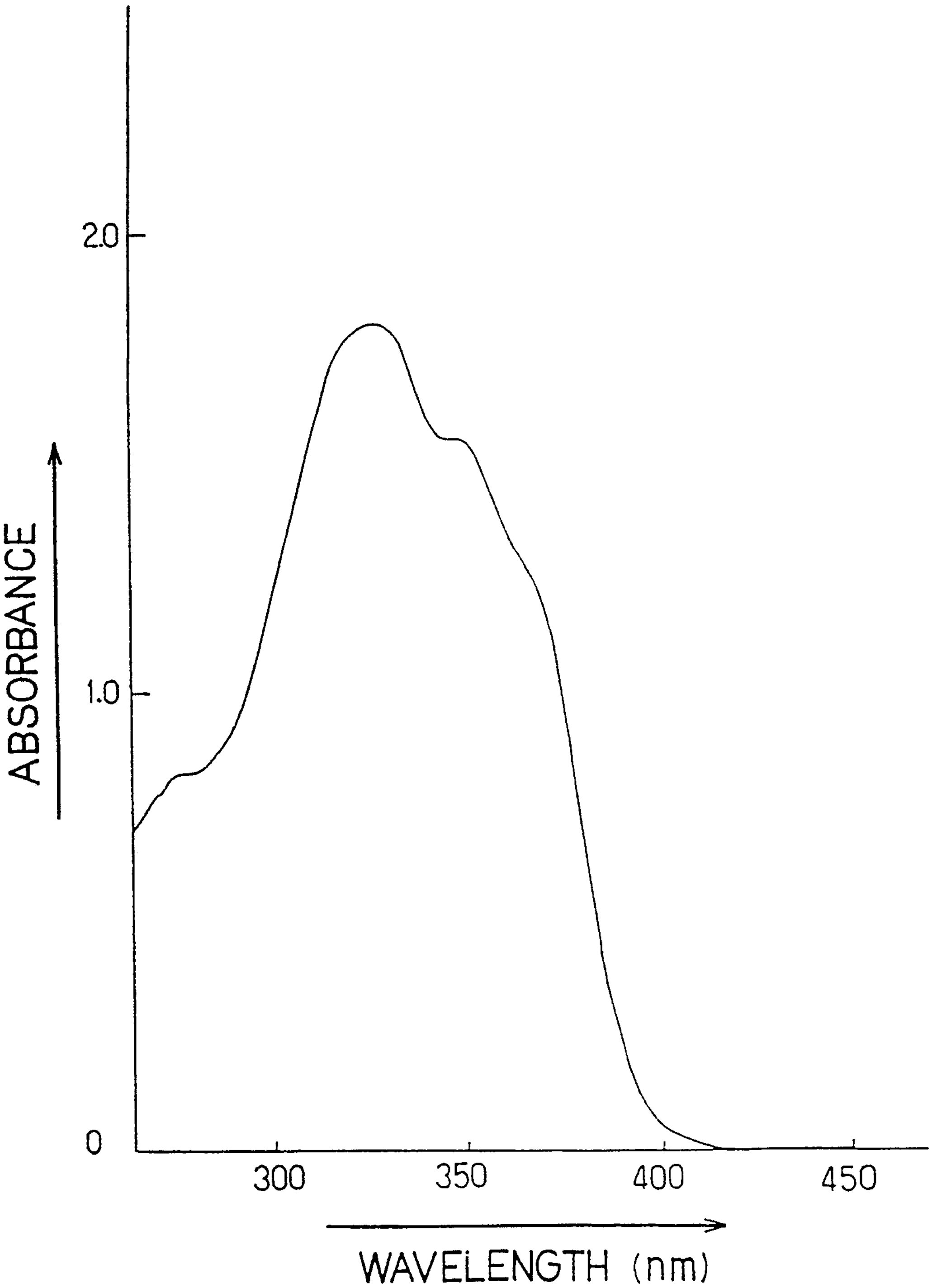

FIG. 38

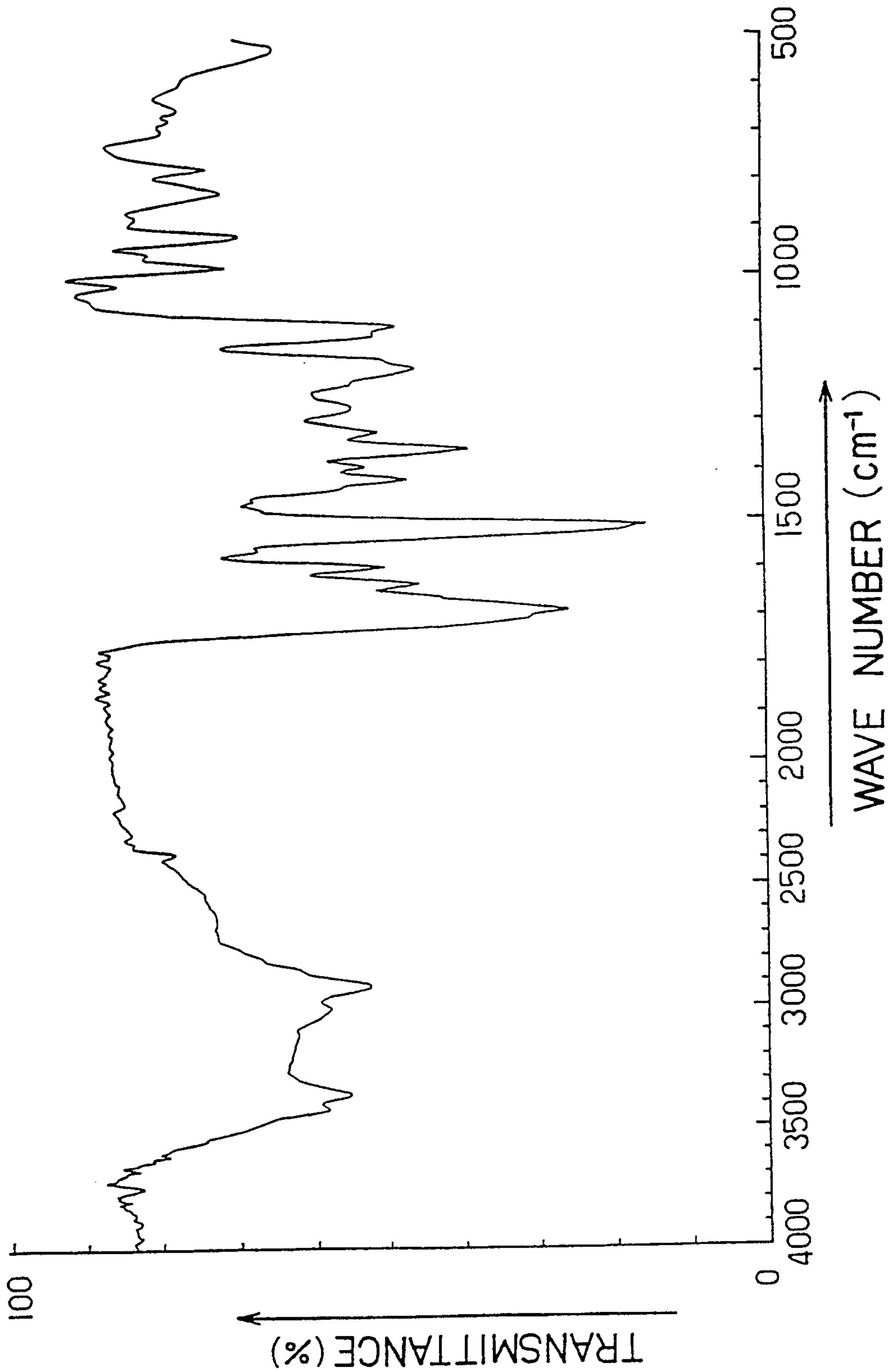
TRANSMITTANCE (%)
100
0
WAVE NUMBER (cm$^{-1}$)
4000
3500
3000
2500
2000
1500
1000
500

FIG. 39

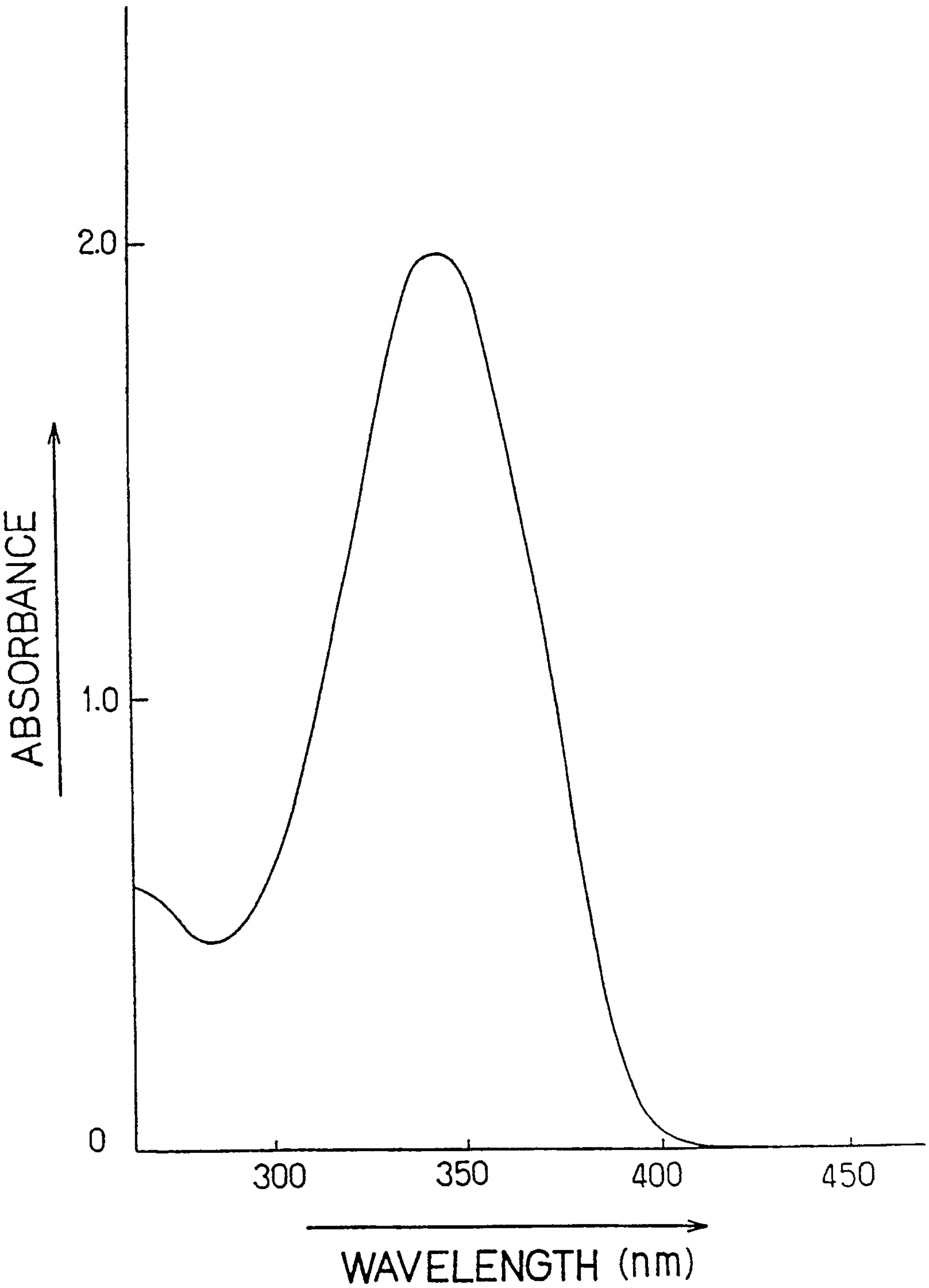

FIG. 40

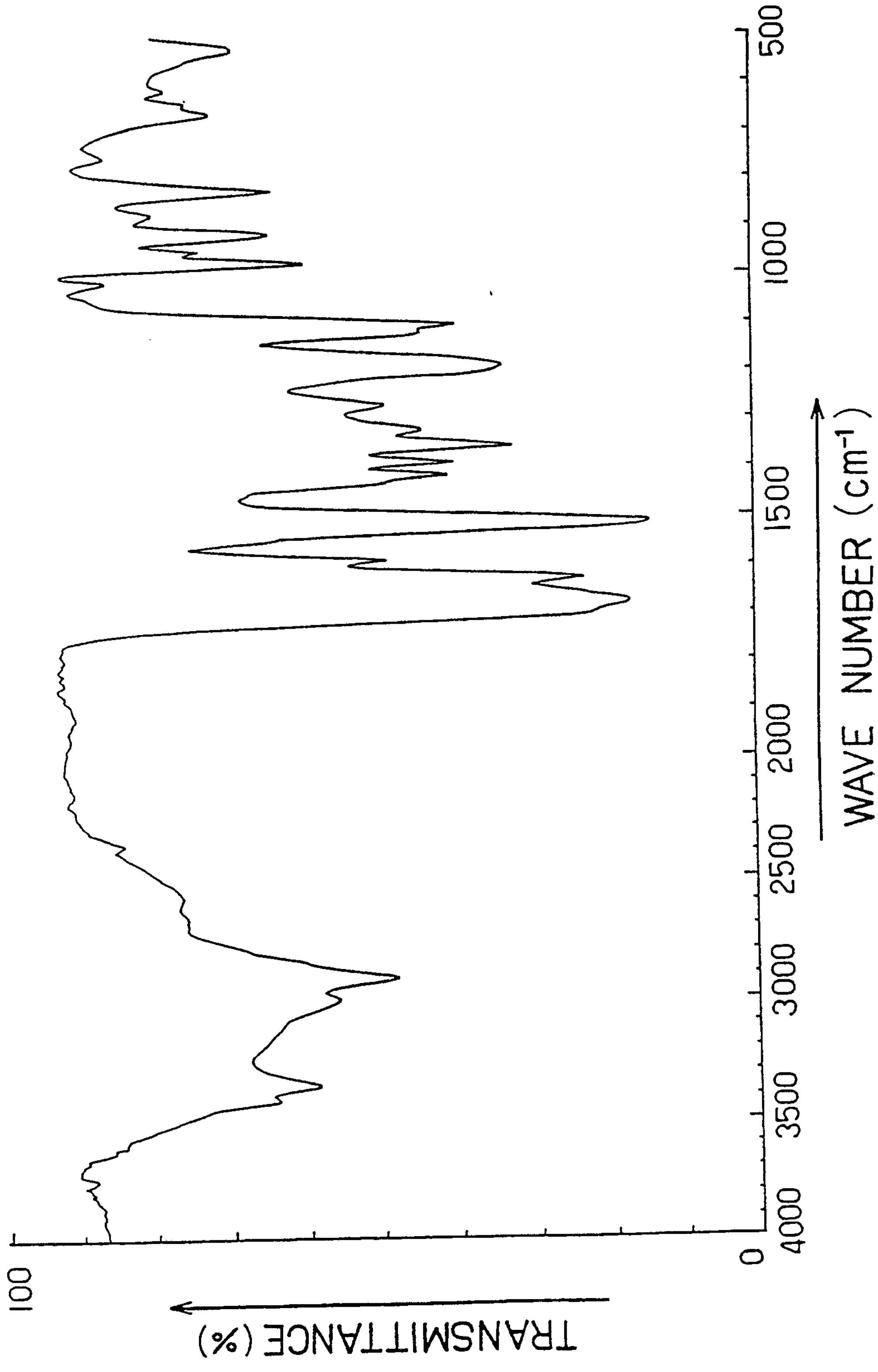
100
0
TRANSMITTANCE (%)
4000
3500
3000
2500
2000
1500
1000
500
WAVE NUMBER (cm⁻¹)

FIG. 41

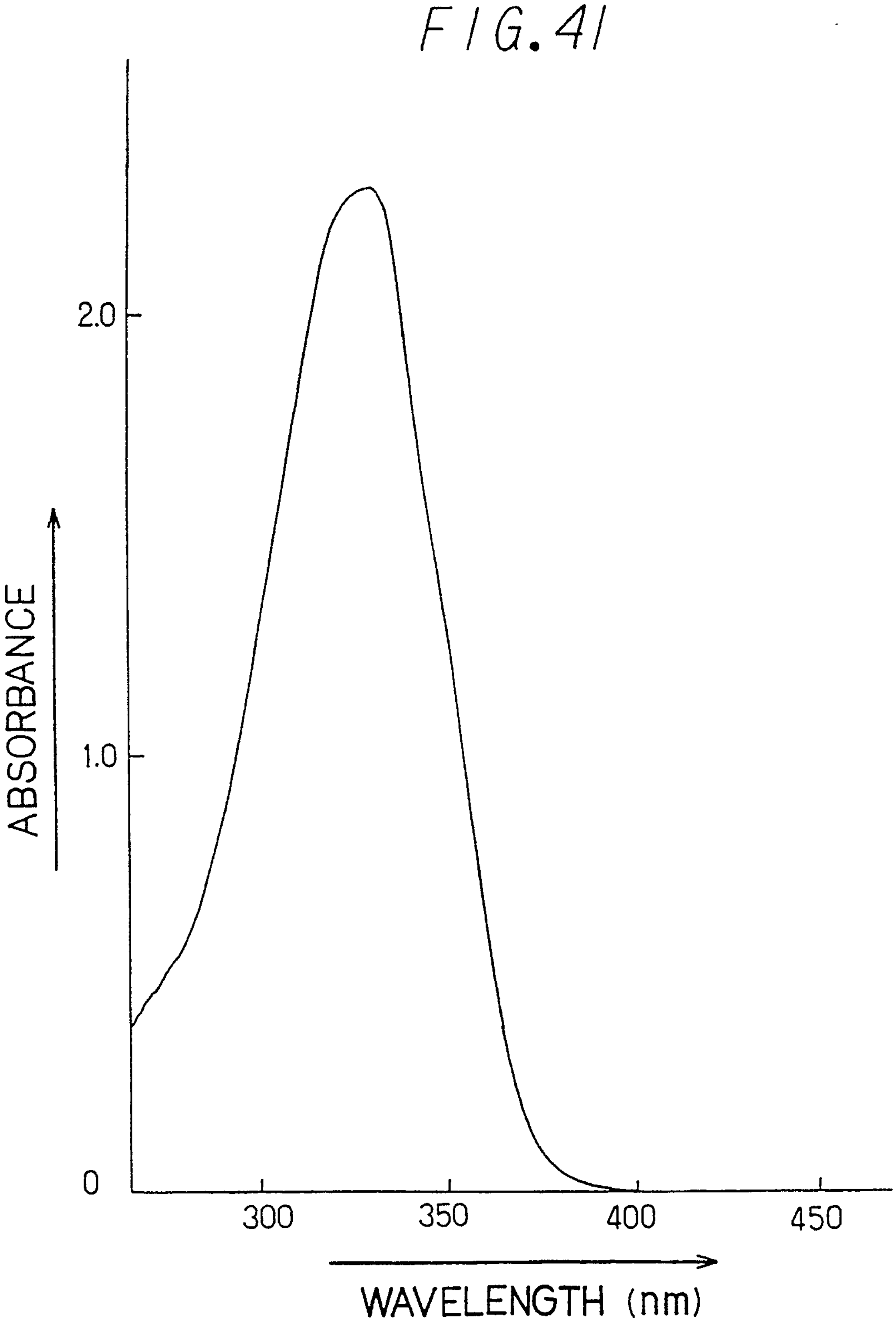

FIG. 42

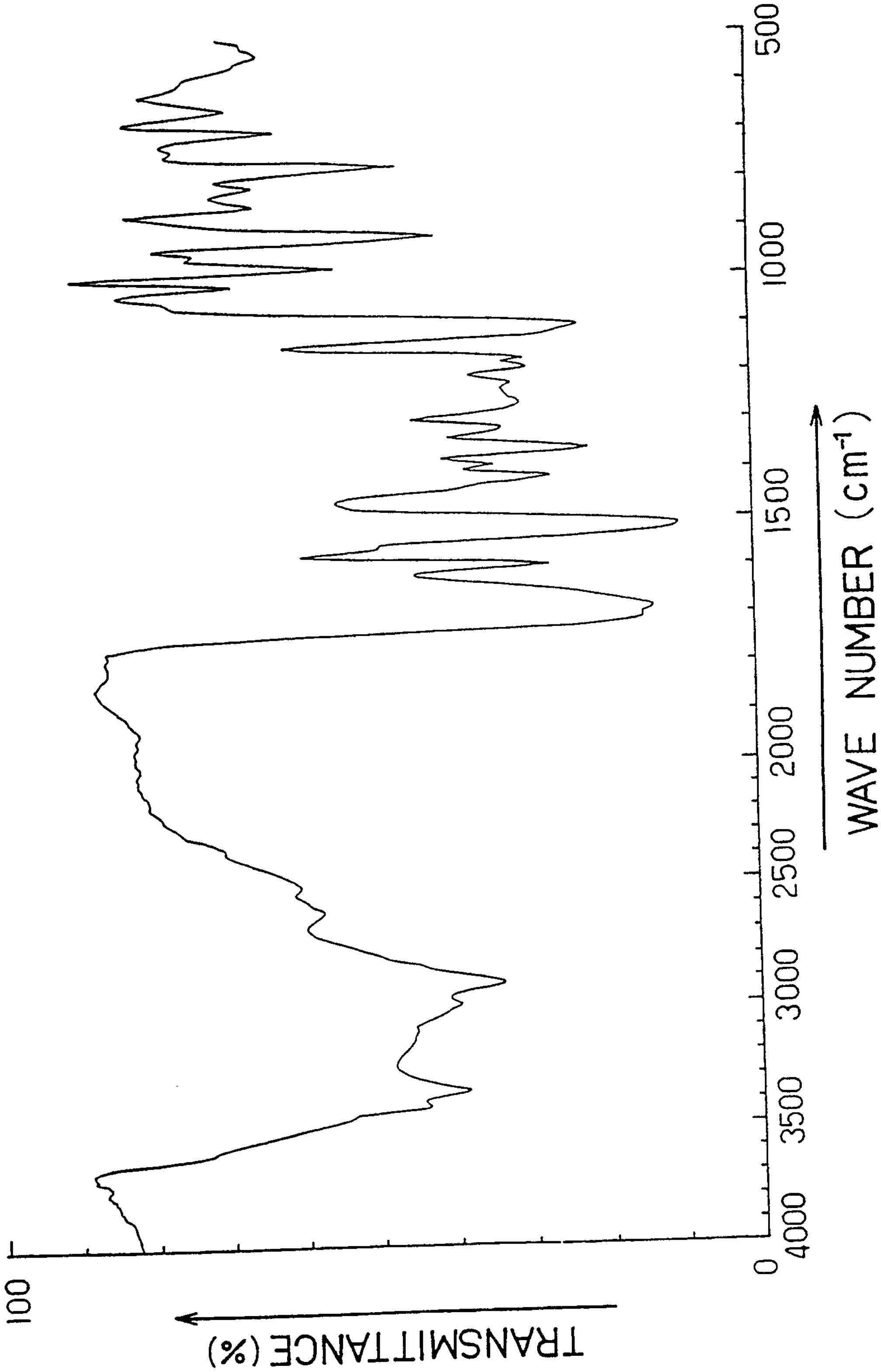
100
0
TRANSMITTANCE (%)
4000
3500
3000
2500
2000
1500
1000
500
WAVE NUMBER (cm-1)

FIG.43

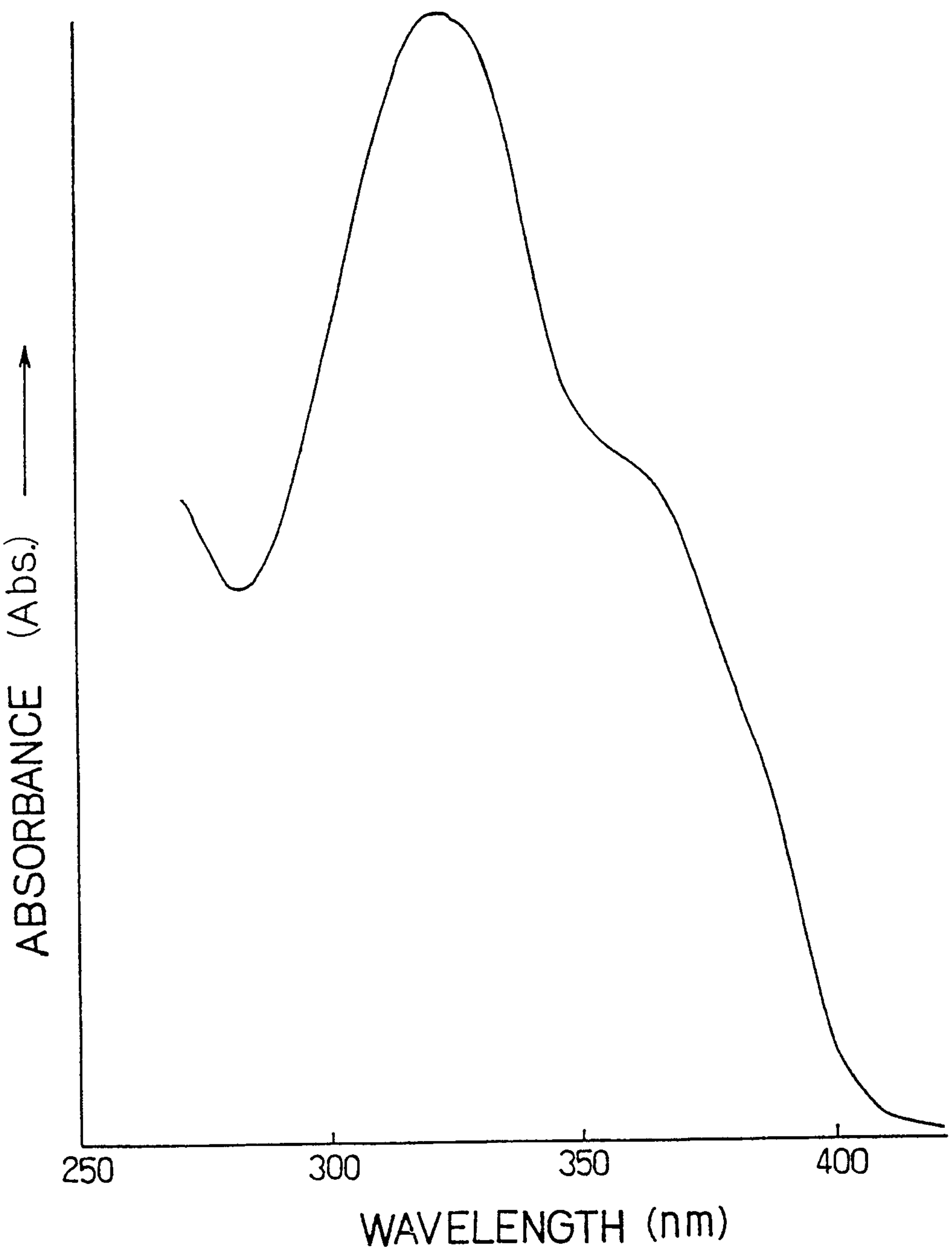

F I G. 44

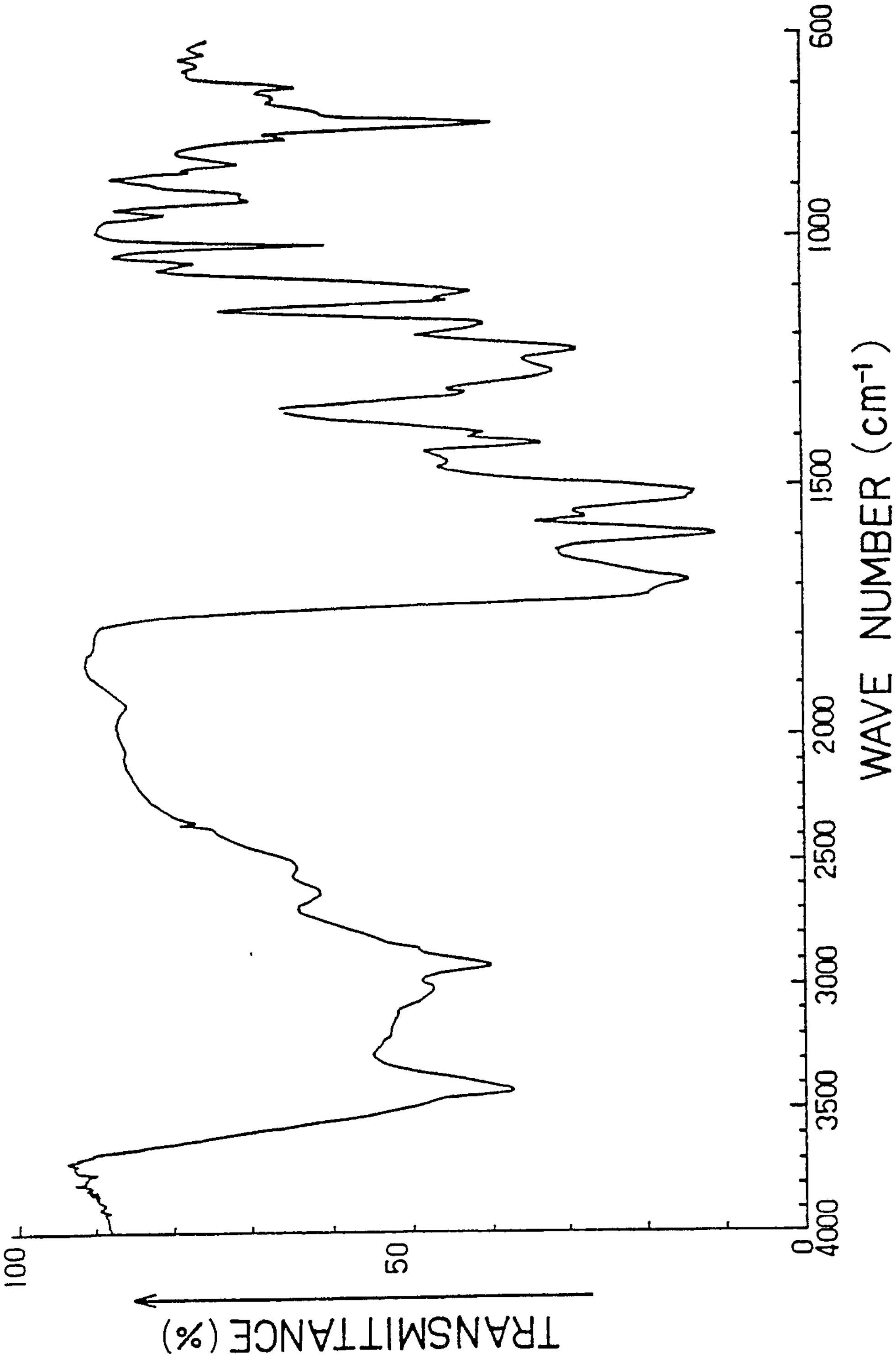
100
50
0
TRANSMITTANCE (%)
4000
3500
3000
2500
2000
1500
1000
600
WAVE NUMBER (cm⁻¹)

FIG. 45

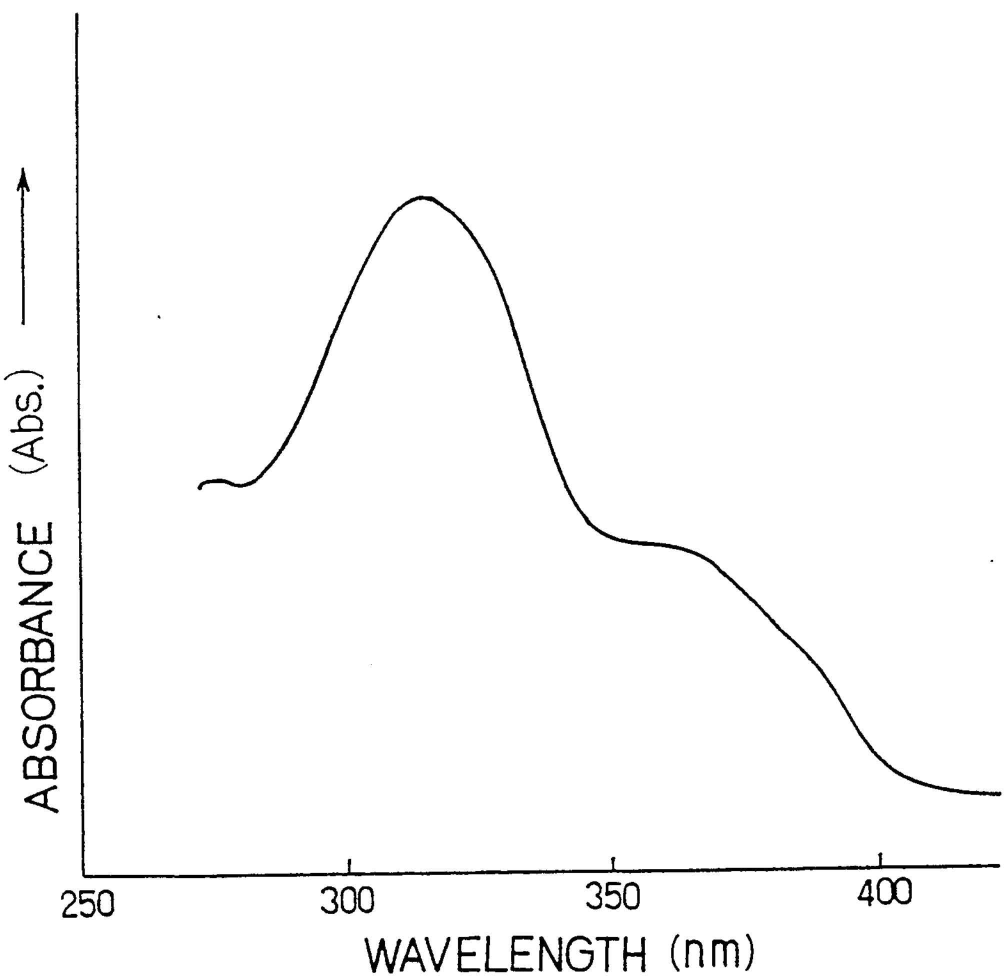

FIG. 46

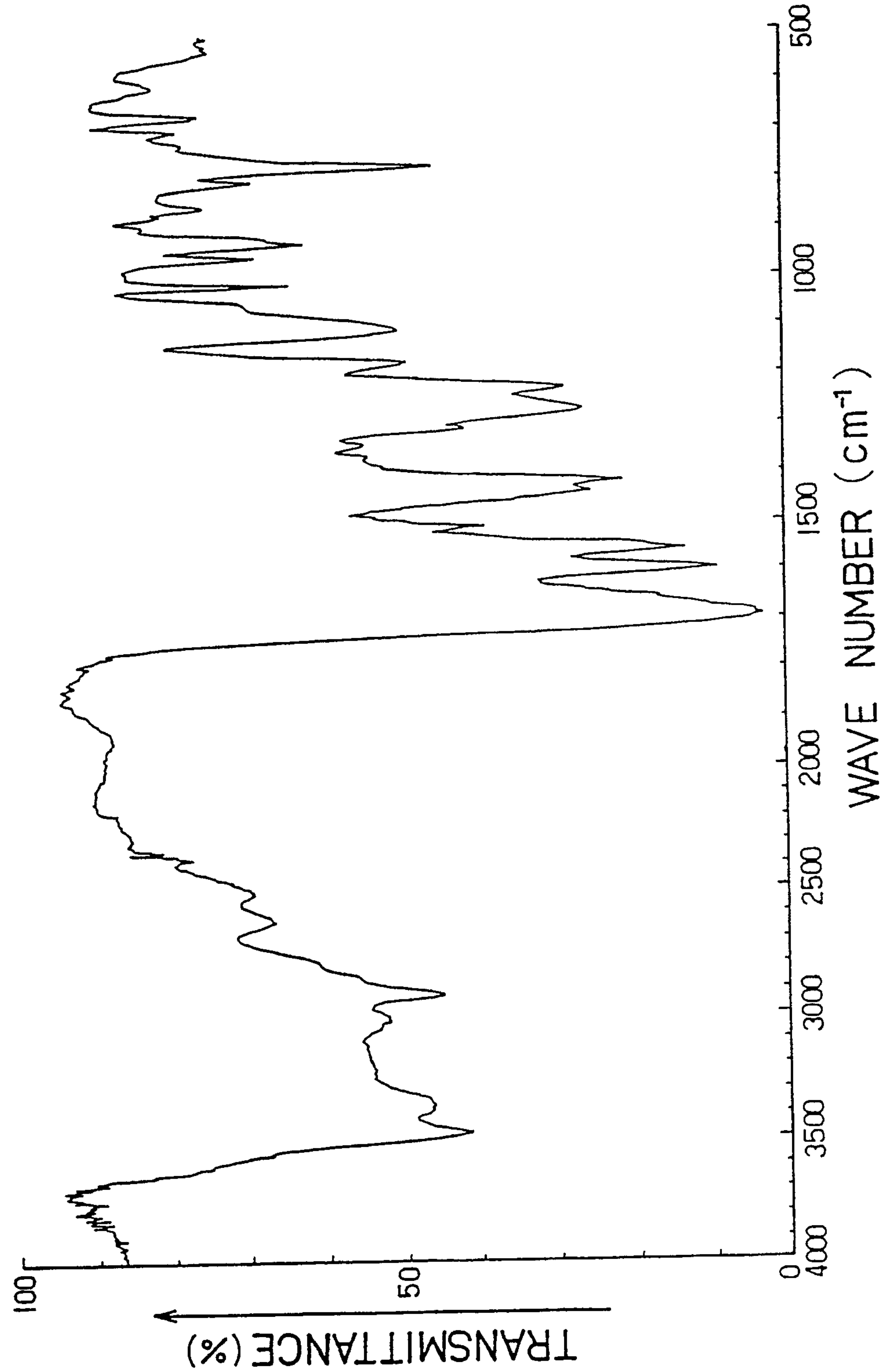
TRANSMITTANCE (%)
100
50
0
WAVE NUMBER (cm-1)
4000
3500
3000
2500
2000
1500
1000
500

# INTERNATIONAL SEARCH REPORT

International Application No PCT/JP90/00545

**I. CLASSIFICATION OF SUBJECT MATTER** (if several classification symbols apply, indicate all) [6]

According to International Patent Classification (IPC) or to both National Classification and IPC

Int. Cl[5] C07C281/14, 337/08, C07D207/337-34, 307/52-54, 333/22-24, A61K7/42, C09K3/00, C08F12/26

**II. FIELDS SEARCHED**

Minimum Documentation Searched [7]

| Classification System | Classification Symbols |
|---|---|
| IPC | C07C281/14, 337/08, C07D207/337-34, 307/52-54, 333/22-24, A61K7/42, C09K3/00, C08F12/26 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, B2, 63-64422 (Maruho K.K.), 12 December 1988 (12. 12. 88), Claim (Family: none) | 1 - 10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 16, 1990 (16. 07. 90) | July 30, 1990 (30. 07. 90) |
| **International Searching Authority** | **Signature of Authorized Officer** |
| Japanese Patent Office | |